(19) Europäisches Patentamt
European Patent Office
Office européen des brevets

(11) Veröffentlichungsnummer: **0 417 631 A2**

# (12) EUROPÄISCHE PATENTANMELDUNG

(21) Anmeldenummer: **90117078.7**

(22) Anmeldetag: **05.09.90**

(51) Int. Cl.5: **C07D 519/00**, C07D 487/10, A61K 31/55, //(C07D519/00, 487:00,471:00),(C07D519/00, 495:00,487:00),(C07D519/00, 471:00,471:00),(C07D519/00, 487:00,487:00),(C07D487/10, 209:00,209:00)

(30) Priorität: **11.09.89 DE 3930262**

(43) Veröffentlichungstag der Anmeldung: **20.03.91 Patentblatt 91/12**

(84) Benannte Vertragsstaaten: **AT BE CH DE DK ES FR GB GR IT LI LU NL SE**

(71) Anmelder: **Dr. Karl Thomae GmbH**
**Postfach 1755**
**W-7950 Biberach 1(DE)**

(72) Erfinder: **Engel, Wolfhard, Dr. Dipl.-Chem.**
**Mozartstrasse 13**
**W-7950 Biberach 1(DE)**
Erfinder: **Eberlein, Wolfgang, Dr. Dipl.-Chem.**
**Obere Au 6**
**W-7950 Biberach 1(DE)**
Erfinder: **Trummlitz, Günter, Dr. Dipl.-Chem.**
**Buchenweg 27**
**W-7951 Warthausen(DE)**
Erfinder: **Mihm, Gerhard, Dr. Dipl.-Chem.**
**Nickeleshalde 5/1**
**W-7950 Biberach 1(DE)**
Erfinder: **Doods, Henri, Dr.**
**Hornsteinweg 7**
**W-7951 Warthausen(DE)**
Erfinder: **Mayer, Norbert, Dr.**
**Friedrich-Ebert-Strasse 66**
**W-7950 Biberach 1(DE)**
Erfinder: **de Jonge, Adriaan, Dr.**
**De Boomgaard 19**
**NL-3971 LD Driebergen(NL)**

## (54) Kondensierte Diazepinone, Verfahren zu ihrer Herstellung und diese Verbindungen enthaltende Arzneimittel.

(57) Kondensierte Diazepinone der allgemeinen Formel I

EP 0 417 631 A2

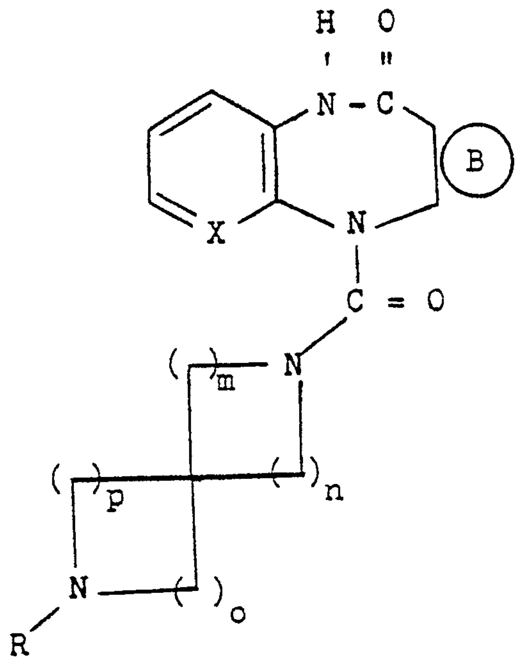

(I),

in der 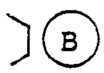 einen der zweiwertigen Reste

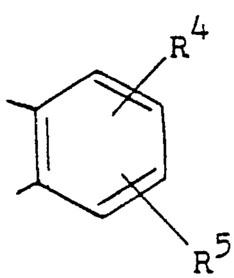 , 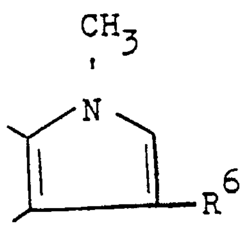 , 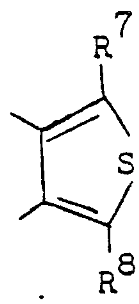 , 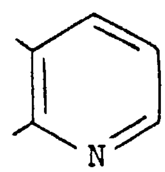

(S) (T) (U) (V)

darstellt, X eine =CH-Gruppe oder ein Stickstoffatom ist, R einen niederen Alkylrest, der gegebenenfalls noch substituiert sein kann durch ein gegebenenfalls Halogen, Methyl oder Methoxy tragendes Phenyl, $R^4$ und $R^5$ Wasserstoff, Halogen oder niederes Alkyl darstellen, $R^6$ Wasserstoff, Chlor oder Methyl ist, $R^7$ und $R^8$ niederes Alkyl, $R^8$ zusätzlich noch Halogen bedeuten und m, n, o und p die Zahlen 1 oder 2 sind, wobei die Summe aus m+n+o+p gleich oder kleiner als 6 sein muß, eignen sich zur Therapie cholinerg bedingter Spasmen und Motilitätsstörungen im Magen-Darm-Trakt, im Bereich der abführenden Gallengänge, zur symptomatischen Therapie der Cystitis und von Spasmen bei Urelithiasis, zur Therapie der relativen Inkontinenz, zur symptomatischen Therapie des Asthma bronchiale und der Bronchitis sowie zur Therapie ischämischer Herzerkrankungen. Die Verbindungen zeichnen sich durch eine hohe Selektivität aus.

# KONDENSIERTE DIAZEPINONE, VERFAHREN ZU IHRER HERSTELLUNG UND DIESE VERBINDUNGEN ENTHALTENDE ARZNEIMITTEL

Die Erfindung betrifft neue kondensierte Diazepinone, Verfahren zu ihrer Herstellung und diese Verbindungen enthaltende Arzneimittel.

Aus EP-A-0 039 519 und 0 057 428 sowie aus US-A 3.660.380; 3.691.159; 4.213.984; 4.213.985; 4.210.648, 4.410.527; 4.424.225; 4.424.222 und 4.424.226 sind bereits kondensierte Diazepinone mit ulcushemmenden und magensaftsekretionshemmenden Eigenschaften bekannt.

In EP-A-0 156 191 (US-Patent 4 550 107) ist für kondensierte Diazepinone beschrieben, daß durch Einführung neuartiger Aminoacylreste gegenüber den Verbindungen der obengenannten Publikationen völlig andersartige, wertvolle pharmakologische Eigenschaften induziert werden können. Gegenüber den oben genannten ulcushemmenden und den vorstehend erwähnten antibradycarden kondensierten Diazepinonen weisen die Verbindungen der vorliegenden Anmeldung - trotz naher struktureller Verwandschaft - überraschend eine weitere, von den obigen Effekten abweichende Wirkqualität auf. Die erfindungsgemäßen Verbindungen eignen sich zur Therapie cholinerg bedingter Spasmen und Motilitätsstörungen im Magen-Darm-Trakt und im Bereich der abführenden Gallengänge, zur symptomatischen Therapie der Cystitis und von Spasmen bei Urelithiasis durch Senkung des pathologisch erhöhten Tonus der Hohlorgane, zur Therapie der relativen Inkontinenz, die auf einem Mißverhältnis von Sphinkter- und Detrusor-Tonus beruht, zur symptomatischen Therapie des Asthma bronchiale und der Bronchitis durch Unterdrückung des muscarinisch vermittelten Anteils der Bronchokonstriktion, sowie zur Therapie ischämischer Herzerkrankungen durch Herzfrequenzsenkung bei gleichzeitiger Unterdrückung parasympathisch verursachter Coronarspasmen und Senkung des basalen Coronartonus. Die erfindungsgemäßen kondensierten Diazepinone zeigen die angegebenen Effekte mit hoher Selektivität und sind insbesondere im therapeutisch relevanten Dosisbereich frei von tachycarden Begleitwirkungen.

Die neuen kondensierten Diazepinone besitzen die allgemeine Formel I

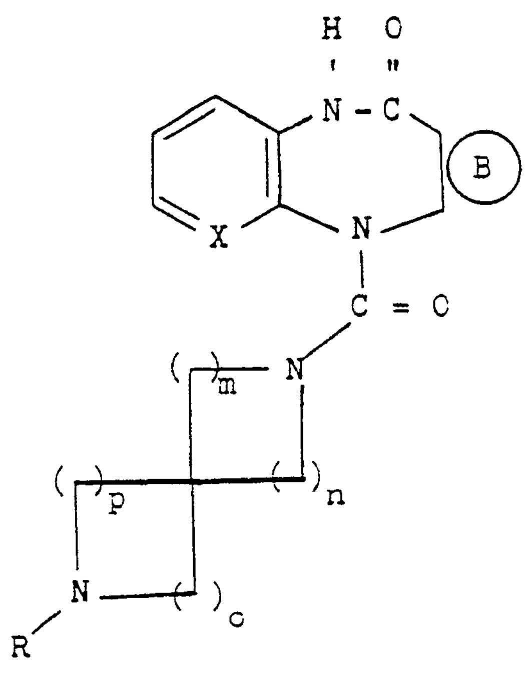

(I),

in der
]Ⓑ einen der zweiwertigen Reste

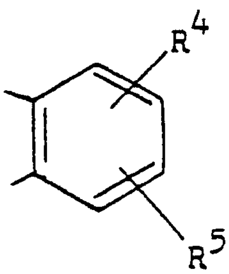 , 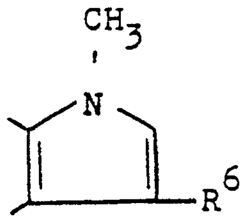 , 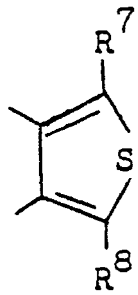 , 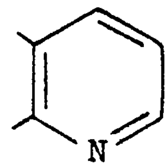

(S) (T) (U) (V)

darstellt und

X, R, $R^4$, $R^5$, $R^6$, $R^7$, $R^8$, m, n, o und p die folgenden Bedeutungen besitzen:

X ist eine =CH-Gruppe oder ein Stickstoffatom,

R ist ein geradkettiger oder verzweigter Alkylrest mit 1 bis 4 Kohlenstoffatomen, der gegebenenfalls noch durch ein durch Chlor, Brom, Fluor, Methyl oder Methoxy mono- oder disubstituiertes Phenyl substituiert sein kann,

$R^4$ und $R^5$, die gleich oder voneinander verschieden sein können, bedeuten ein Wasserstoff-, Fluor-, Chlor- oder Bromatom oder eine Alkylgruppe mit 1 bis 4 Kohlenstoffatomen,

$R^6$ ist ein Wasserstoff- oder Chloratom oder eine Methylgruppe,

$R^7$ und $R^8$, die gleich oder voneinander verschieden sein können, bedeuten Wasserstoffatome oder Alkylgruppen mit 1 bis 4 Kohlenstoffatomen, $R^8$ kann aber auch zusätzlich ein Halogenatom bedeuten,

m, n, o und p bedeuten jeweils die Zahlen 1 oder 2 mit der Einschränkung, daß die Summe aus m+n+o+p gleich oder kleiner als 6 sein muß.

Bevorzugte Verbindungen der obigen allgemeinen Formel I sind solche, in denen entweder

X ein Stickstoffatom und ]Ⓑ den Rest (S) oder

X die =CH-Gruppe und ]Ⓑden Rest (V) bedeuten,

R die Methylgruppe,

$R^4$ und $R^5$, die gleich oder verschieden sein können, jeweils ein Wasserstoff-, Fluor- oder Chloratom, die Methyl-oder Ethylgruppe darstellen und

m und p jeweils den Zahlenwert 1 und

n und o jeweils unabhängig voneinander die Werte 1 oder 2 annehmen.

Die Verbindungen der allgemeinen Formel I können nach Umsetzung mit anorganischen oder organischen Säuren auch in Form ihrer physiologisch verträglichen Salze vorliegen. Als Säuren haben sich beispielsweise Salzsäure, Bromwasserstoffsäure, Schwefelsäure, Methylschwefelsäure, Phosphorsäure, Weinsäure, Fumarsäure, Zitronensäure, Maleinsäure, Bernsteinsäure, Gluconsäure, Äpfelsäure, p-Toluolsulfonsäure, Methansulfonsäure oder Amidosulfonsäure als geeignet erwiesen.

Zur Erläuterung des Erfindungsgegenstandes seien als Beispiele folgende Verbindungen genannt:

(±)-5,11-Dihydro-11-[[7-methyl-2,7-diazaspiro[4,4]non-2-yl]carbonyl]-6H-pyrido[2,3-b][1,4]benzodiazepin-6-on

R-5,11-Dihydro-11-[[7-methyl-2,7-diazaspiro[4,4]non-2-yl]carbonyl]-6H-pyrido[2,3-b][1,4]benzodiazepin-6-on

S-5,11-Dihydro-11-[[7-methyl-2,7-diazaspiro[4,4]non-2-yl]carbonyl]-6H-pyrido[2,3-b][1,4]benzodiazepin-6-on

(±)-9-Chlor-5,11-dihydro-11-[[7-methyl-2,7-diazaspiro[4,4]non-2-yl]carbonyl]-6H-pyrido[2,3-b][1,4]-benzodiazepin-6-on

8-Chlor-5,11-dihydro-11-[[7-methyl-2,7-diazaspiro[4,4]non-2-yl]carbonyl]-6H-pyrido[2,3-b][1,4]benzodiazepin-6-on

8-Brom-5,11-dihydro-11-[[7-methyl-2,7-diazaspiro[4,4]non-2-yl]carbonyl]-6H-Pyrido[2,3-b][1,4]-benzodiazepin-6-on

5,11-Dihydro-8-ethyl-11-[[7-methyl-2,7-diazaspiro[4,4]non-2-yl]carbonyl]-6H-pyrido[2,3-b][1,4]benzodiazepin-6-on

5,11-Dihydro-9-methyl-11-[[7-methyl-2,7-diazaspiro[4,4]non-2-yl]carbonyl]-6H-pyrido[2,3-b][1,4]-

benzodiazepin-6-on
5,11-Dihydro-11-[[7-ethyl-2,7-diazaspiro[4,4]non-2-yl]carbonyl]-6H-pyrido[2,3-b][1,4]benzodiazepin-6-on
5,11-Dihydro-8-methyl-11-[[7-methyl-2,7-diazaspiro[4,4]non-2-yl]carbonyl]-6H-pyrido[2,3-b][1,4]-benzodiazepin-6-on
5,11-Dihydro-11-[[7-ethyl-2,7-diazaspiro[4,4]non-2-yl]carbonyl]-8-methyl-6H-Pyrido[2,3-b][1,4]benzodiazepin-6-on
5,11-Dihydro-11-[[7-ethyl-2,7-diazaspiro[4,4]ncn-2-yl]carbonyl]-9-methyl-6H-pyrido[2,3-b][1,4]benzodiazepin-6-on
9-Chlor-5,11-dihydro-11-[[7-ethyl-2,7-diazaspiro[4,4]non-2-yl]carbonyl]-6H-pyrido[2,3-b][1,4]benzodiazepin-6-on
5,11-Dihydro-11-[[6-methyl-2,6-diazaspiro[3,4]oct-2-yl]carbonyl]-6H-pyrido[2,3-b][1,4]benzodiazepin-6-on
5,11-Dihydro-8-ethyl-11-[[6-methyl-2,6-diazaspiro[3,4]oct-2-yl]carbonyl]-6H-pyrido[2,3-b][1,4]benzodiazepin-6-on
5,11-Dihydro-8-methyl-11-[[6-methyl-2,6-diazaspiro[3,4]oct-2-yl]carbonyl]-6H-pyrido[2,3-b][1,4]-benzodiazepin-6-on
5,11-Dihydro-9-methyl-11-[[6-methyl-2,6-diazaspiro[3,4]oct-2-yl]carbonyl]-6H-pyrido[2,3-b][1,4]-benzodiazepin-6-on
9-Chlor-5,11-dihydro-11-[[6-methyl-2,6-diazaspiro[3,4]oct-2-yl]carbonyl]-6H-pyrido[2,3-b][1,4]benzodiazepin-6-on
5,11-Dihydro-8,9-dimethyl-11-[[6-methyl-2,6-diazaspiro[3,4]oct-2-yl]carbonyl]-6H-pyrido[2,3-b][1,4]-benzodiazepin-6-on
5,11-Dihydro-11-[[2-methyl-2,6-diazaspiro[3,4]oct-6-yl]carbonyl]-6H-pyrido[2,3-b][1,4]benzodiazepin-6-on
5,11-Dihydro-11-[[6-(phenylmethyl)-2,6-diazaspiro[3,4]oct-2-yl]carbonyl]-6H-pyrido[2,3-b][1,4]benzodiazepin-6-on
5,11-Dihydro-8-methyl-11-[[2-methyl-2,6-diazaspiro[3,4]oct-6-yl]carbonyl]-6H-pyrido[2,3-b][1,4]-benzodiazepin-6-on
5,11-Dihydro-8-ethyl-11-[[2-methyl-2,6-diazaspiro[3,4]oct-6-yl]carbonyl]-6H-pyrido[2,3-b][1,4]benzodiazepin-6-on
5,11-Dihydro-9-methyl-11-[[2-methyl-2,6-diazaspiro[3,4]oct-6-yl]carbonyl]-6H-pyrido[2,3-b][1,4]-benzodiazepin-6-on
9-Chlor-11-[[2-methyl-2,6-diazaspiro[3,4]oct-6-yl]carbonyl]-6H-pyrido[2,3-b][1,4]benzodiazepin-6-on
8-Chlor-11-[[2-methyl-2,6-diazaspiro[3,4]oct-6-yl]carbonyl]-6H-pyrido[2,3-b][1,4]benzodiazepin-6-on
5,11-Dihydro-11-[[6-ethyl-2,6-diazaspiro[3,4]oct-2-yl]carbonyl]-8-methyl-6H-pyrido[2,3-b][1,4]benzodiazepin-6-on
5,11-Dihydro-11-[[6-ethyl-2,6-diazaspiro[3,4]oct-2-yl]carbonyl]-6H-pyrido[2,3-b][1,4]benzodiazepin-6-on
5,11-Dihydro-11-[[6-ethyl-2,6-diazaspiro[3,4]oct-2-yl]carbonyl]-9-methyl-6H-pyrido[2,3-b][1,4]benzodiazepin-6-on
9-Chlor-5,11-dihydro-11-[[6-ethyl-2,6-diazaspiro[3,4]oct-2-yl]carbonyl]-6H-pyrido[2,3-b][1,4]benzodiazepin-6-on
5,11-Dihydro-8-ethyl-11-[[6-ethyl-2,6-diazaspiro[3,4]oct-2-yl]carbonyl]-6H-pyrido[2,3-b][1,4]benzodiazepin-6-on
5,11-Dihydro-11-[[6-propyl-2,6-diazaspiro[3,4]oct-2-yl]carbonyl]-6H-pyrido[2,3-b][1,4]benzodiazepin-6-on
5,11-Dihydro-8-methyl-11-[[6-propyl-2,6-diazaspiro[3,4]oct-2-yl]carbonyl]-6H-pyrido[2,3-b][1,4]-benzodiazepin-6-on
8-Chlor-5,11-dihydro-11-[[6-propyl-2,6-diazaspiro[3,4]oct-2-yl]carbonyl]-6H-pyrido[2,3-b][1,4]benzodiazepin-6-on
5,11-Dihydro-9-methyl-11-[[6-propyl-2,6-diazaspiro[3,4]oct-2-yl]carbonyl]-6H-pyrido[2,3-b][1,4]-benzodiazepin-6-on
5,11-Dihydro-8-ethyl-11-[[6-propyl-2,6-diazaspiro[3,4]oct-2-yl]carbonyl]-6H-pyrido[2,3-b][1,4]benzodiazepin-6-on
5,11-Dihydro-11-[[2-ethyl-2,6-diazaspiro[3,4]oct-6-yl]carbonyl]-6H-pyrido[2,3-b][1,4]benzodiazepin-6-on
5,11-Dihydro-11-[[2-ethyl-2,6-diazaspiro[3,4]oct-6-yl]carbonyl]-8-methyl-6H-pyrido[2,3-b][1,4]benzodiazepin-6-on
5,11-Dihydro-11-[[2-ethyl-2,6-diazaspiro[3,4]oct-6-yl]carbonyl]-9-methyl-6H-pyrido[2,3-b][1,4]benzodiazepin-6-on
9-Chlor-5,11-dihydro-11-[[2-ethyl-2,6-diazaspiro[3,4]oct-6-yl]carbonyl]-6H-pyrido[2,3-b][1,4]benzodiazepin-6-on
5,11-Dihydro-11-[[6-(2-methylpropyl)-2,6-diazaspiro[3,4]oct-2-yl]carbonyl]-6H-pyrido[2,3-b][1,4]-

benzodiazepin-6-on
5,11-Dihydro-8-methyl-11-[[6-(2-methylpropyl)-2,6-diazaspiro[3,4]oct-2-yl]carbonyl]-6H-pyrido[2,3-b][1,4]-benzodiazepin-6-on
5,11-Dihydro-9-methyl-11-[[6-(2-methylpropyl)-2,6-diazaspiro[3,4]oct-2-yl]carbonyl]-6H-pyrido[2,3-b][1,4]-benzodiazepin-6-on
9-Chlor-5,11-dihydro-11-[[6-(2-methylpropyl)-2,6-diazaspiro[3,4]oct-2-yl]carbonyl]-6H-pyrido[2,3-b][1,4]-benzodiazepin-6-on
5,11-Dihydro-11-[[6-methyl-2,6-diazaspiro[3,3]hept-2-yl]carbonyl]-6H-pyrido[2,3-b][1,4]benzodiazepin-6-on
5,11-Dihydro-8-methyl-11-[{6-methyl-2,6-diazaspiro[3,3]hept-2-yl]carbonyl]-6H-pyrido[2,3-b][1,4]-benzodiazepin-6-on
5,11-Dihydro-9-methyl-11-[[6-methyl-2,6-diazaspiro[3,3]hept-2-yl]carbonyl]-6H-pyrido[2,3-b][1,4]-benzodiazepin-6-on
5,11-Dihydro-11-[[7-methyl-2,7-diazaspiro[3,5]non-2-yl]carbonyl]-6H-Pyrido[2,3-b][1,4]benzodiazepin-6-on
5,11-Dihydro-8-methyl-11-[[7-methyl-2,7-diazaspiro[3,5]non-2-yl]carbonyl]-6H-pyrido[2,3-b][1,4]-benzodiazepin-6-on
9-Chlor-5,11-dihydro-11-[[7-methyl-2,7-diazaspiro[3,5]non-2-yl]carbonyl]-6H-pyrido[2,3-b][1,4]benzodiazepin-6-on
5,11-Dihydro-11-[[2-methyl-2,7-diazaspiro-3,5]non-7-yl]carbonyl]-6H-pyrido[2,3-b][1,4]benzodiazepin-6-on
5,11-Dihydro-9-methyl-11-[[2-methyl-2,7-diazaspiro[3,5]non-7-yl]carbonyl]-6H-pyrido[2,3-b][1,4]-benzodiazepin-6-on
8-Chlor-5-11-dihydro-11-[[2-methyl-2,7-diazaspiro[3,5]non-7-yl]carbonyl]-6H-pyrido[2,3-b][1,4]benzodiazepin-6-on
B-Brom-5,11-dihydro-11-[[2-methyl-2,7-diazaspiro[3,5]non-7-yl]carbonyl]-6H-pyrido[2,3-b][1,4]-benzodiazepin-6-on
(±)-6,11-Dihydro-11-[[7-methyl-2,7-diazaspiro[4,4]non-2-yl]carbonyl]-5H-pyrido[2,3-b][1,5]benzodiazepin-5-on
R-6,11-Dihydro-11-[[7-methyl-2,7-diazaspiro[4,4]non-2-yl]carbonyl]-5H-pyrido[2,3-b][1,5]benzodiazepin-5-on
S-6,11-Dihydro-11-[[7-methyl-2,7-diazaspiro[4,4]non-2-yl]carbonyl]-5H-pyrido[2,3-b][1,5]benzodiazepin-5-on
(±)-6,11-Dihydro-11-[[7-ethyl-2,7-diazaspiro[4,4]non-2-yl]carbonyl]-5H-pyrido[2,3-b][1,5]benzodiazepin-5-on
6,11-Dihydro-11-[[2-methyl-2,6-diazaspiro[3,4]oct-6-yl]carbonyl]-5H-pyrido[2,3-b][1,5]benzodiazepin-5-on
6,11-Dihydro-11-[[6-ethyl-2,6-diazaspiro[3,4]oct-2-yl]carbonyl]-5H-pyrido[2,3-b][1,5]benzodiazepin-5-on
6,11-Dihydro-11-[[6-propyl-2,6-diazaspiro[3,4]oct-2-yl]carbonyl]-5H-pyrido[2,3-b][1,5]benzodiazepin-5-on
6,11-Dihydro-11-[[2-ethyl-2,6-diazaspiro[3,4]oct-6-yl]carbonyl]-5H-pyrido[2,3-b][1,5]benzodiazepin-5-on
6,11-Dihydro-11-[[6-(2-methylpropyl)-2,6-diazaspiro[3,4]oct-2-yl]-carbonyl]-5H-pyrido[2,3-b][1,5]-benzodiazepin-5-on
6,11-Dihydro-11-[[6-methyl-2,6-diazaspiro[3,3]hept-2-yl]carbonyl]-5H-pyrido[2,3-b][1,5]benzodiazepin-5-on
6,11-Dihydro-11-[[7-methyl-2,7-diazaspiro[3,5]non-2-yl]carbonyl]-5H-pyrido[2,3-b][1,5]benzodiazepin-5-on
6,11-Dihydro-11-[[2-methyl-2,7-diazaspiro[3,5]non-7-yl]carbonyl]-5H-pyrido[2,3-b][1,5]benzodiazepin-5-on
(±)-5,10-Dihydro-5-[[7-methyl-2,7-diazaspiro[4,4]non-2-yl]carbonyl]-11H-dibenzo[b,e][1,4]diazepin-11-on
5,10-Dihydro-5-[[6-methyl-2,6-diazaspiro[3,4]oct-2-yl]carbonyl]-11H-dibenzo[b,e][1,4]diazepin-11-on
(±)-4,9-Dihydro-3-methyl-4-[[7-methyl-2,7-diazaspiro[4,4]non-2-yl]carbonyl]-10H-thieno[3,4-b][1,5]-benzodiazepin-10-on
(±)-4,9-Dihydro-4-[[7-ethyl-2,7-diazaspiro[4,4]non-2-yl]carbonyl]-3-methyl-10H-thieno[3,4-b][1,5]-benzodiazepin-10-on
4,9-Dihydro-3-methyl-4-[[6-methyl-2,6-diazaspiro[3,4]oct-2-yl]carbonyl]-10H-thieno[3,4-b][1,5]benzodiazepin-10-on
4,9-Dihydro-3-methyl-4-[[2-methyl-2,6-diazaspiro[3,4]oct-6-yl]carbonyl]-10H-thieno[3,4-b][1,5]benzodiazepin-10-on
4,9-Dihydro-4-[[6-ethyl-2,6-diazaspiro[3,4]oct-2-yl]carbonyl]-3-methyl-10H-thieno[3,4-b][1,5]benzodiazepin-10-on
4,9-Dihydro-3-methyl-4-[[6-propyl-2,6-diazaspiro[3,4]oct-2-yl]carbonyl]-10H-thieno[3,4-b][1,5]benzodiazepin-10-on
4,9-Dihydro-4-[[2-ethyl-2,6-diazaspiro[3,4]oct-6-yl]carbonyl]-3-methyl-10H-thieno[3,4-b][1,5]benzodiazepin-10-on
4,9-Dihydro-3-methyl-4-[[6-(2-methylpropyl)-2,6-diazaspiro[3,4]oct-2-yl]carbonyl]-10H-thieno[3,4-b][1,5]-benzodiazepin-10-on
4,9-Dihydro-3-methyl-4-[[6-methyl-2,6-diazaspiro[3,3]hept-2-yl]carbonyl]-10H-thieno[3,4-b][1,5]-benzodiazepin-10-on
4,9-Dihydro-4-[[6-methyl-2,6-diazaspiro[3,3]hept-2-yl]carbonyl]-10H-thieno[3,4-b][1,5]benzodiazepin-10-on

4,9-Dihydro-3-methyl-4-[[7-methyl-2,7-diazaspiro[3,5]non-2-yl]carbonyl]-10H-thieno[3,4-b][1,5]-benzodiazepin-10-on

4,9-Dihydro-3-methyl-4-[[2-methyl-2,7-diazaspiro[3,5]non-7-yl]carbonyl]-10H-thieno[3,4-b][1,5]-benzodiazepin-10-on

(±)-3-Chlor-1-methyl-4-[[7-methyl-2,7-diazaspiro[4,4]non-2-yl]carbonyl]-1,4,9,10-tetrahydropyrrolo[3,2-b][1,5]-benzodiazepin-10-on

(±)-1-Methyl-4-[[7-methyl-2,7-diazaspiro[4,4]non-2-yl]carbonyl]-1,4,9,10-tetrahydropyrrolo[3,2-b][1,5]-benzodiazepin-10-on

3-Chlor-1-methyl-4-[[6-Propyl-2,6-diazaspiro[3,4]oct-2-yl]-carbonyl]-1,4,9,10-tetrahydropyrrolo[3,2-b][1,5]-benzodiazepin-10-on

3-Chlor-1-methyl-4-[[6-(2-methylpropyl)-2,6-diazaspiro[3,4]-oct-2-yl]Carbonyl]-1,4,9,10-tetrahydropyrrolo[3,2-b][1,5]-benzodiazepin-10-on

3-Chlor-1-methyl-4-[[6-methyl-2,6-diazaspiro[3,4]oct-2-yl]carbonyl]-1,4,9,10-tetrahydropyrrolo[3,2-b][1,5]-benzodiazepin-10-on

3-Chlor-1-methyl-4-[[6-methyl-2,6-diazaspiro[3,3]hept-2-yl]carbonyl]-1,4,9,10-tetrahydropyrrolo[3,2-b][1,5]-benzodiazepin-10-on

Erfindungsgemäß erhält man die neuen basisch substituierten kondensierten Diazepinone der allgemeinen Formel I nach folgenden Verfahren:

a) Basisch substituierte kondensierte Diazepinone der allgemeinen Formel Ia

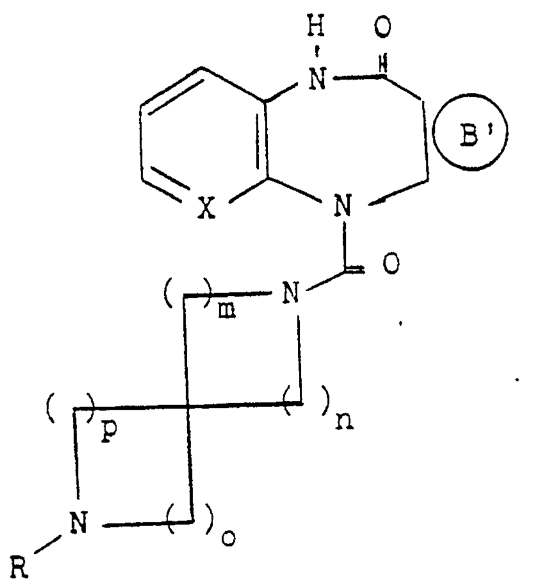

(Ia),

in der
X, R, m, n, o und p die oben angegebenen Bedeutungen haben und ] Ⓑ einen der zweiwertigen Reste (S), (U), (V) oder (T′)

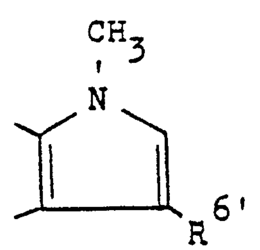

(T'),

darstellt, worin $R^4$, $R^5$, $R^7$ und $R^8$ wie oben definiert sind und $R^{6'}$ ein Chloratom oder eine Methylgruppe bedeutet,
erhält man durch Umsetzung von Kohlensäurederivaten der allgemeinen Formel II

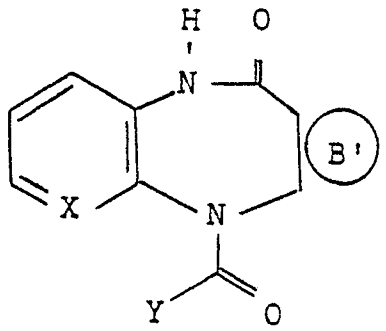

(II),

in der

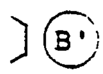

und X die angegebenen Bedeutungen haben und Y ein Halogenatom, bevorzugt das Brom- oder Chloratom, oder den Rest $OR^{11}$ bedeutet, wobei $R^{11}$ einen gegebenenfalls halogensubstituierten Alkylrest mit 1 bis 5 Kohlenstoffatomen, einen gegebenenfalls durch Halogenatome oder Nitrogruppen substituierten Phenylrest oder einen Aralkylrest mit 7 bis 15 Kohlenstoffatomen darstellt, mit Verbindungen der allgemeinen Formel III,

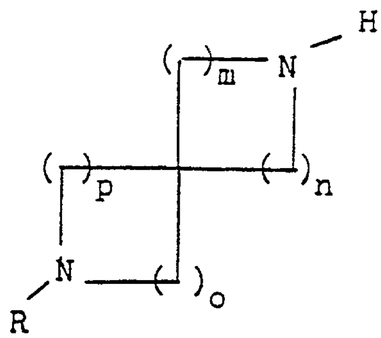

(III),

in der
R, m, n, o, p wie vorstehend definiert sind.

Die Umsetzung wird ohne oder bevorzugt in Gegenwart von Lösungsmitteln, wie z. B. von Wasser, Toluol oder von Alkoholen, wie z. B. Methanol, Ethanol oder Isopropanol, ganz bevorzugt jedoch in Gegenwart aprotischer polarer Lösungsmittel, z. B. von Tetrahydrofuran, 1,4-Dioxan, Acetonitril, N,N-Dimethylformamid, Dimethylsulfoxid, Hexamethylphosphorsäuretriamid, oder von Gemischen davon und bei Temperaturen zwischen -10 °C und dem Siedepunkt des Reaktionsgemisches, bevorzugt zwischen 40 und 100 °C durchgeführt. Bewährt hat sich die Verwendung zusätzlicher anorganischer oder organischer Basen, z. B. von Alkali- oder Erdalkalihydroxiden, -alkoholaten oder -carbonaten, etwa von Natriumhydroxid, Natriummethanolat, Kalium-tert.butanolat, Natriumcarbonat, Kaliumcarbonat; von tertiären Aminen, z. B. von Triethylamin, Ethyldiisopropylamin, N,N-Dimethylanilin, Pyridin oder von 4-(Dimethylamino)pyridin; sowie die Umsetzung in Gegenwart eines Überschusses einer Verbindung der allgemeinen Formel III.

Verwendet man die Spirodiamine der allgemeinen Formel III und die Kohlensäurederivate der allgemeinen Formel II in äquimolaren Mengenverhältnissen, so erhält man - sofern Y ein Halogenatom bedeutet - direkt die halogenwasserstoffsauren Salze der gesuchten Verbindungen der allgemeinen Formel Ia.

Die Umsetzung kann jedoch auch mit einer Metallverbindung der allgemeinen Formel IIIa,

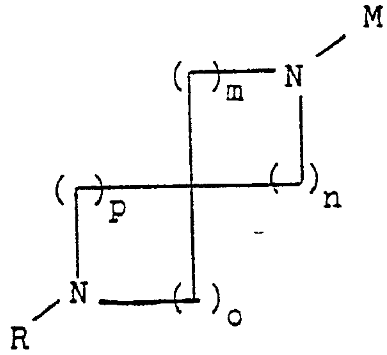

(IIIa),

in der
M ein Alkalimetallatom oder 1 Äquivalent eines Erdalkalimetallatoms bedeutet, durchgeführt werden. Dabei lassen sich Metallverbindungen der allgemeinen Formel IIIa aus III durch Umsetzung mit Alkali- oder Erdalkalimetallen, etwa mit Natrium, Kalium oder Barium, oder mit Alkali- oder Erdalkalihydriden, etwa mit Natrium-, Kalium- oder Calciumhydrid, oder durch Reaktion mit Alkali- oder Erdalkaliorganylen, z. B. mit n-Butyllithium oder Phenyllithium, in situ leicht herstellen.

b.) Basisch substituierte kondensierte Diazepinone der allgemeinen Formel Ia lassen sich auch durch Umsetzung von Tricyclen der allgemeinen Formel IV,

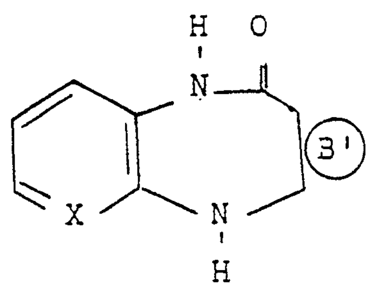

(IV),

in der X und

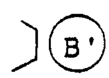

wie oben definiert sind, mit einem Chlorkohlensäurederivat der allgemeinen Formel V

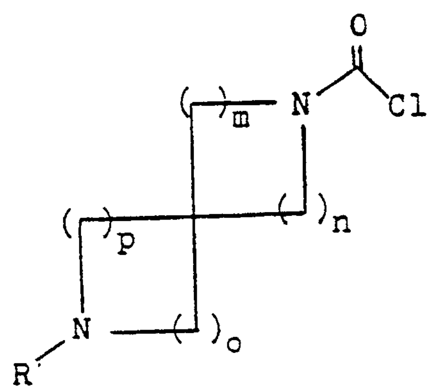

(V),

worin R, m, n, o, p die oben erwähnten Bedeutungen haben, erhalten.

Die Umsetzung wird vorzugsweise in inerten organischen Lösungsmitteln, beispielsweise in aromatischen Kohlenwasserstoffen wie Toluol, Xylol, in Äthern wie Diisopropyläther, Tetrahydrofuran oder Dioxan, in Ketonen wie 3-Pentanon, in chlorierten aliphatischen Kohlenwasserstoffen, wie 1,2-Dichlorethan oder in anderen Lösungsmitteln, wie Acetonitril oder Dimethylformamid oder in Gemischen davon, gegebenenfalls in Gegenwart tertiärer organischer Basen, wie Pyridin, und bei Temperaturen bis zum Siedepunkt des Reaktionsgemisches, bevorzugt bei Temperaturen zwischen +30 und +100 °C, durchgeführt.

c) Die unter die allgemeine Formel I fallenden neuen Pyrrolokondensierten Diazepinone der allgemeinen Formel Ib,

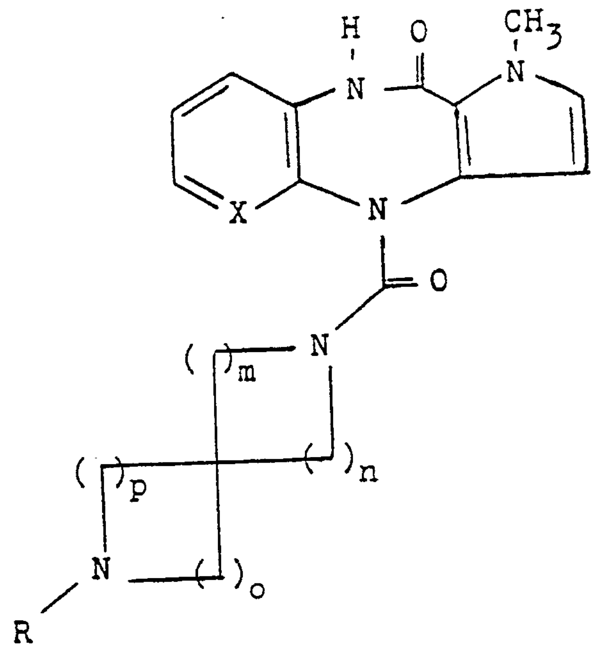

(Ib'),

worin

X, R, m, n, o und p die eingangs erwähnten Bedeutungen haben, können auch durch Hydrogenolyse aus solchen Verbindungen der allgemeinen Formel Ia hergestellt werden, in denen $R^{6'}$ ein Chloratom bedeutet.

Die Hydrogenolyse wird in Gegenwart von Katalysatoren von Metallen aus der VIII. Nebengruppe des Periodensystems der Elemente, beispielsweise von Palladium auf Tierkohle, Palladium auf Bariumsulfat, Raney-Nickel oder Raney-Cobalt, und bei Wasserstoffdrucken von 1 bis 300 bar und Temperaturen von 0 °C bis 130 °C in Gegenwart von Lösungsmitteln, beispielsweise Alkoholen, wie Methanol, Ethanol; Ethern wie Dioxan, Tetrahydrofuran, Carbonsäuren, beispielsweise Essigsäure oder tertiären Aminen, beispielsweise Triethylamin, durchgeführt. Arbeitet man dabei in Abwesenheit zusätzlicher Chlorwasserstoff-Akzeptoren, beispielsweise von Natriumcarbonat, Kaliumhydrogencarbonat, Triethylamin oder Natriumacetat, so entstehen direkt die Hydrochloride der gesuchten Verbindungen, die nach Entfernung des Katalysators durch Eindampfen der Reaktionslösung isoliert werden können. Ersetzt man in der vorstehenden Hydrogenolyse-Reaktion den Wasserstoff durch Ameisensäure, so gelingt die Umsetzung prinzipiell schon bei drucklosem Arbeiten. Bei dieser Variante haben sich besonders die Umsetzung mit Ameisensäure in Gegenwart von Dimethylformamid als Lösungsmittel und von Palladium auf Kohle als Katalysator bei Temperaturen zwischen 70 und 110 °C, sowie die Reduktion mit Triethylammoniumformiat in Gegenwart überschüssigen Triethylamins und von Palladium auf Tierkohle oder von Palladiumacetat und Triarylphosphinen, wie Triphenylphosphin, Tris-(o-tolyl)phosphin, Tris-(2,5-diisopropylphenyl)phosphin, bei Temperaturen zwischen 40 und 110 °C, bewährt.

So erhaltene Basen der allgemeinen Formel I können anschließend in ihre Säureadditionssalze oder erhaltene Säureadditionssalze in die freien Basen oder andere pharmakologisch verträgliche Säureadditionssalze übergeführt werden.

Nehmen in den erfindungsgemäßen aminocarbonylierten kondensierten Diazepinonen der allgemeinen Formel I m und o jeweils den Wert 1, n und p jeweils den Wert 2 an, so sind diese Verbindungen chiral, da sie ein asymmetrisches Kohlenstoffatom in der Seitenkette tragen. Diese Verbindungen können deshalb jeweils als enantiomere (+)- und (-)-Formen auftreten. Die Erfindung umfaßt die einzelnen Isomeren ebenso wie ihre Racemate.

Die Spaltung evtl. Razemate der Verbindungen der allgemeinen Formel I kann nach bekannten

Verfahren durchgeführt werden, beispielsweise unter Verwendung einer optisch aktiven Säure, wie (+)- oder (-)-Weinsäure, oder eines Derivats davon, wie (+)- oder (-)-Diacetylweinsäure, (+)- oder (-)-Monomethyltartrat oder (+)-Camphersulfonsäure.

Nach einem üblichen Verfahren zur Isomerentrennung wird das Racemat einer Verbindung der allgemeinen Formel I mit einer der vorstehend angegebenen optisch aktiven Säuren in äquimolarer Menge in einem Lösungsmittel umgesetzt und die erhaltenen kristallinen diastereomeren Salze werden unter Ausnutzung ihrer verschiedenen Löslichkeit getrennt. Diese Umsetzung kann in jeder Art von Lösungsmittel durchgeführt werden, solange dieses einen ausreichenden Unterschied in der Löslichkeit der Salze aufweist. Vorzugsweise werden Methanol, Ethanol oder deren Gemische, beispielsweise im Volumenverhältnis 50:50, verwendet. Sodann wird jedes der diastereomeren Salze in Wasser gelöst, mit einer Base, wie Natriumhydroxid oder Kaliumhydroxid, neutralisiert und dadurch die entsprechende freie Verbindung in der (+)- oder (-)-Form erhalten.

Jeweils nur ein Enantiomeres wird auch dadurch erhalten, daß man die oben beschriebenen Synthesen mit nur einem Enantiomeren der allgemeinen Formel III bzw. V durchführt.

Die Herstellung der als Zwischenprodukte benötigten Kohlensäurederivate der allgemeinen Formel II wird in der DE-37 26 908-A1 ausführlich beschrieben.

Zwischenverbindungen der allgemeinen Formel III, die im allgemeinen neu und noch nicht vorbeschrieben sind, sind beispielsweise nach folgenden Methoden zugänglich:

a.) Die unter die allgemeine Formel III fallenden 2-substituierten 2,7-Diazaspiro[4,4]nonane erhält man entsprechend oder in völliger Analogie zu den Angaben in Warner-Lambert Comp., AU 83 18698 (vgl. Derwent 107 300).

b.) Die unter die allgemeine Formel III fallenden 6-substituierten 2,6-Diazaspiro[3,4]octane lassen sich beispielsweise ausgehend von 1,1,2-Ethantricarbonsäureestern bereiten, die bei der Umsetzung mit geeigneten 1,3,5-trisubstituierten Hexahydro-1,3,5-triazinen in Gegenwart katalytischer Mengen von Trifluoressigsäure in guter Ausbeute 1-substituierte 2-Pyrrolidinon-4,4-dicarbonsäureester ergeben. Reduktion mit Lithiumaluminiumhydrid führt zu 1-substituierten 3,3-Bis-(hydroxymethyl)-pyrrolidinen, die sich mit konzentrierter wässeriger Bromwasserstoffsäure im Bombenrohr glatt zu den entsprechenden Bis-(brommethyl)pyrrolidin-hydrobromiden umsetzen lassen. Diese reagieren mit p-Toluolsulfonsäureamid in Gegenwart von konzentrierter Kalilauge und unter Verwendung von Dioxan oder Dimethylformamid als Lösungsmitteln in hoher Ausbeute zu 6-substituierten 2-(4-Methylbenzensulfonyl)-2,6-diazaspiro[3,4]-octanen, die sich beispielsweise mit Natrium-bis-(2-methoxyethoxy)-aluminiumhydrid (RED-Al$^R$), bevorzugt in Toluol-Lösung, zu den gesuchten 6-substituierten 2,6-Diazaspiro[3,4]octanen detosylieren lassen. Alternativ kann man für die Umsetzung mit den 1,3,5-trisubstituierten Hexahydro-s-triazinen statt der 1,1,2-Ethantricarbonsäureester 2-Cyanbernsteinsäureester verwenden. Dabei resultieren 1-substituierte 4-Cyan-4-carbalkoxy-2-pyrrolidinone, die bei der Reduktion mit Lithiumaluminiumhydrid 1-substituierte 3-(Aminomethyl)-3-(hydroxymethyl)-pyrrolidine ergeben. Umsetzung mit konzentrierter wässeriger Bromwasserstoffsäure liefert in guter Ausbeute 1-substituierte 3-(Aminomethyl)-3-(brommethyl)-pyrrolidine, die bei der Behandlung mit konzentrierter Natron- oder Kalilauge in Gegenwart von mit Wasser mischbaren Lösemitteln, z. B. von Dioxan, Ethanol, Methanol, Dimethylformamid, glatt zu den gesuchten 6-substituierten 2,6-Diazaspiro[3,4]octanen cyclisieren.

c.) Die unter die allgemeine Formel III fallenden 2-substituierten 2,6-Diazaspiro[3,4]octane sind - nach einer der unter b) angegebenen Varianten - aus leicht erhältlichem 6-(Phenylmethyl)-2,6-diazaspiro[3,4]-octan dadurch zugäng lich, daß man in üblicher Weise in 2-Stellung alkyliert, beispielsweise durch Umsetzung mit einem geeigneten Acylierungsmittel mit nachfolgender Reduktion mit Lithiumaluminiumhydrid, und anschließend den Benzylrest hydrogenolytisch entfernt.

d.) Die unter die allgemeine Formel III fallenden 2-substituierten 2,6-Diazaspiro[3,3]heptane sind aus dem vorbekannten unsubstituierten 2,6-Diazaspiro[3,3]heptan (A. Litherland und F.G. Mann, J. chem. Soc. [London] 1938, 1588; F. Govaert und M. Beyaert. Bull. Soc. chim. Belg. 55, 106, 112 [1946]; F.G. Mann und A. Litherland, Nature 141, 789-790 [1938]) in üblicher Weise herstellbar, beispielsweise durch Monoacylierung und anschließende vorsichtige Reduktion mit Lithiumaluminiumhydrid in Diethylether.

e.) Die unter die allgemeine Formel III fallenden 7-substituierten 2,7-Diazaspiro[3,5]nonane lassen sich aus den in Analogie zu Angaben von L. Ciszewski, Pol. J. Chem. 62, 451-455 [1988] zugänglichen 1-substituierten 4-Cyan-4-piperidincarbonsäureestern herstellen. Beispielsweise werden die 1-substituierten 4-Cyan-4-piperidincarbonsäureester in Eisessig und in Gegenwart von Platin(IV)-oxid und konzentrierter Schwefelsäure zu den entsprechenden ß-Alaninestern katalytisch hydriert. Diese können dann mit Grignard-Reagenzien, beispielsweise mit Ethylmagnesiumbromid, in Ether und bei 0 bis 5 °C zu 7-substituierten 2,7-Diazaspiro[3,5]nonan-1-onen cyclisiert werden, die bei der Reduktion mit Lithiumaluminiumhydrid in Ether in die gesuchten 7-substituierten 2,7-Diazaspiro[3,5]nonane übergehen. Alternativ

erhält man die 7-substituierten 2,7-Diazaspiro[3,5]nonan-1-one auch dadurch, daß man die erwähnten ß-Alaninester zu den entsprechenden ß-Alaninen verseift und dann in prinzipiell bekannter Weise den Azetidinon-Ring schließt, z. B. durch Einwirkung von Triphenylphosphin und Tetrachlormethan bzw. N-Bromuccinimid, von Ethyldichlorphosphat, Phenyldichlorphosphat oder Phenyl phosphonsäuredichlorid, jeweils in Gegenwart von Triethylamin und unter Verwendung von Acetonitril als Lösungsmittel.

f.) Aus nach e) synthetisiertem 7-(Phenylmethyl)-2,7-diazaspiro[3,5]nonan lassen sich mittels der unter c) angegebenen Reaktionsabfolge schließlich auch die unter die allgemeine Formel III fallenden 2-substituierten 2,7-Diazaspiro[3,5]-nonane ohne nennenswerte Probleme bereiten.

Die Tricyclen der allgemeinen Formel IV sind aus der Patentliteratur bekannt oder können in enger Anlehnung an publizierte Verfahren aus gängigen Ausgangsmaterialien synthetisiert werden.

Chlorkohlensäurederivate der allgemeinen Formel V sind in Anlehnung an gängige Verfahren aus Spirodiaminen der allgemeinen Formel II und Phosgen leicht herstellbar.

Ein weiterer Gegenstand der Erfindung sind Arzneimittel, die ein oder mehrere basisch substituierte Diazepinone der allgemeinen Formel I bzw. deren physiologisch verträgliche Salze enthalten.

Die Verbindungen der allgemeinen Formel I lassen sich hierzu in an sich bekannter Weise in die üblichen pharmazeutischen Zübereitungsformen, z.B. in Lösungen, Suppositorien, Tabletten, Dragees, Kapseln oder Teezübereitungen einarbeiten. Die Tagesdosis liegt bei oraler Gabe im allgemeinen zwischen 0,01 und 10 mg/kg, vorzugsweise 0,02 und 5 mg/kg, insbesondere 0,05 und 2,5 mg/kg Körpergewicht, die gegebenenfalls in Form mehrerer, vorzugsweise 1 bis 3 Einzelgaben, zur Erzielung der gewünschten Ergebnisse verabreicht wird.

Die basisch substituierten kondensierten Diazepinone der allgemeinen Formel I und ihre Säureadditionssalze besitzen wertvolle Eigenschaften; wie bereits eingangs ausgeführt, zeigen sie selektive spasmolytische Eigenschaften auf periphere Organe, insbesondere Ileum und Blase, und sind angesichts im therapeutischen Dosisbereich fehlender herzfrequenzsteigernder, magensäuresekretionshemmender, salivationshemmender und das Akkomodationsvermögen des Auges beeinträchtigender Effekte in der Human- und Veterinärmedizin geeignet zur Therapie cholinerg bedingter Spasmen und Motilitätsstörungen im Magen-Darmtrakt und im Bereich der abführenden Gallengänge, zur symptomatischen Therapie der Cystitis und von Spasmen bei Urelithiasis durch Senkung des pathologisch erhöhten Tonus der Hohlorgane, zur Therapie der relativen Inkontinenz, die auf einem Mißverhältnis von Sphinkter- und Detrusor-Tonus beruht, zur symptomatischen Therapie des Asthma bonchiale und der Bronchitis durch Unterdrückung des muscarinisch vermittelten Anteils der Bronchokonstriktion, sowie zur Therapie ischämischer Herzerkrankungen durch Herzfrequenzsenkung und gleichzeitige Unterdrückung parasympathisch verursachter Coronarspasmen und Senkung des basalen Coronartonus.

Eine günstige Relation zwischen spasmolytischen Wirkungen einerseits und den bei Therapeutica mit anticholinerger Wirkkomponete auftretenden unerwünschten Wirkungen auf die Herzfrequenz, die Pupillenweite, die Tränen-, Speichel- und Magensäuresekretion andererseits ist für die therapeutische Verwendung der Substanzen von besonderer Wichtigkeit. Die folgenden Versuche zeigen, daß die erfindungsgemäßen Verbindungen diesbezüglich überraschend günstige Relationen aufweisen.

A. Untersuchung auf funktionelle Selektivität der anti-muscarinischen Wirkung

Substanzen mit antimuscarinischen Eigenschaften inhibieren die Wirkungen von exogen zugeführten Agonisten oder von Acetylcholin, das aus cholinergen Nervenendigungen freigesetzt wird. Im folgenden wird eine Beschreibung von Methoden wiedergegeben, die zur Erfassung von spasmolytisch wirkenden Antimuscarinica geeignet sind.

"In vitro" Organpräparationen

Dissoziationskonstanten ($K_B$-Werte) wurden "in vitro" am Ileum und spontan schlagenden Vorhof des Meerschweinchens ermittelt. Das Ileum wurde entnommen und im Organbad in Krebs-Henseleit-Lösung inkubiert. Kontraktionen wurden derart durch steigende Konzentrationen von Methacholin (M) hervorgerufen, daß volle Konzentrations-Wirkungskurven aufgenommen werden konnten. Danach wurde M ausgewaschen, die zu untersuchende Substanz beigefügt und 30 Minuten lang in Kontakt gelassen, und wiederum eine Konzentrations-Wirkungskurve mit M aufgenommen.

Aus dem Dosisverhältnis (DR), das ist das Ausmaß der Verschiebung der Konzentrations-Wirkungskurve, wurde die Dissoziationskonstante nach Arunlakshana und Schild (Brit. J. Pharmacol. 14, 48, 1959)

berechnet.

Am isolierten, spontan schlagenden rechten Vorhof reduzierte M konzentrationsabhängig die Herzfrequenz. Durch Zugabe eines Antimuscarinicums wurde dieser Effekt wieder aufgehoben. Dissoziationskonstanten für die muscarinischen Rezeptoren des Vorhofes wurden auf die gleiche Art und Weise wie oben beschrieben gewonnen. Der Vergleich der in beiden Geweben ermittelten Dissoziationskonstanten erlaubte die Identifizierung von selektiv spasmolytisch wirkenden Substanzen. Die Ergebnisse sind in der Tabelle III enthalten.

"In vivo" Methoden

Die angewandten Methoden hatten zum Ziel, die Selektivität der antimuscarinischen Wirkung zu bestätigen. Jene Substan zen, die auf der Basis von "in vitro" Untersuchungen ausgewählt worden waren, wurden auf

1. Selektivität der bronchospasmolytischen Aktivität am Meerschweinchen,
2. Speichelsekretionshemmende Wirkung an der Ratte und
3. In-situ-spasmolytische Aktivität am Meerschweinchen untersucht.

1. Wirkung auf M-Rezeptoren der Bronchien, des Herzens und der Harnblase anaesthetisierter Meerschweinchen

Meerschweinchen beiderlei Geschlechts (550-600 g Körpergewicht) wurden mit Urethan (1,4 g/kg, i.p.) anaesthetisiert. Eine Kanüle wurde zur Injektion der Wirkstoffe in die Jugularvene eingeführt. 220 I.U./kg Heparin wurden intravenös injiziert. Es wurde eine Kanüle in die Trachea eingeführt; die Tiere wurden künstlich mit sauerstoffangereicherter Luft mittels einer positiven Druckpumpe (Braun-Melsungen) bei einer Rate von 80 Schlägen pro Minute beatmet. Ein Ast der Trachealkanüle wurde mit einem Wassermanometer von 10 cm Höhe verbunden. Das Beatmungsvolumen wurde so eingestellt, daß der maximale intratracheale Druck während der Beatmung gerade den Druck einer 10 cm-Wassersäule erreichte.

Sieht man von einigen Modifikationen ab, wurde die Wirkung der Wirkstoffe auf den Bronchialtonus nach der von Konzett und Rössler (1940) beschriebenen Methode gemessen. Das durch Bronchokonstriktion erzeugte Volumen des Beatmungsgasgemisches (Overflow), das das Wassermanometer durchströmte, wur de mittels eines Röhren-Pneumotachometers (FLEISCH, Modell 1000), das mit einem SP 2040D Differentialdruck-Transducer (HSE) verbunden war, gemessen. Die Werte wurden mit einem IFD Aufnahmegerät registriert. Vor dem Versuch wurde die Trachea für eine kurze Zeit abgeklemmt, um den maximal möglichen Grad der Bronchokonstriktion zur Kalibrierung zu erzeugen. Eine Kanüle wurde in die linke große Halsschlagader eingeführt; der arterielle Blutdruck wurde mit Hilfe eines Drucktransducers (Bell and Howell, 4-327 I) in Verbindung mit einem IFD-Aufzeichnungsgerät gemessen. Die Herzfrequenz wurde mit einem Frequenzmesser, der durch arterielle Pulswellen ausgelöst wird, gemessen.

Es wurde ein kleiner medianer Bauchschnitt gesetzt und die Harnblase an einem Kraft-Transducer unter einer Ruhespannung von 1 Gramm angebunden.

Die zu testenden Wirksubstanzen wurden über die Jugularvene injiziert, 5 Minuten später wurde die Spannungszunahme der Blase (in Gramm), der Bronchialwiderstand (in %) und die Abnahme der Herzfrequenz (Schläge pro Minute) nach Gabe von Acetylcholin (50 µg/kg i.v. und i.a.) gemessen. Es wurden dosis-abhängige Kurven durch Wiedergabe der prozentualen Hemmung der Bronchokonstriktion, Bradycardie und der Spannungszunahme der Blase gegen den Logarithmus der Dosis (mol/kg) der zu untersuchenden Wirkstoffe aufgezeichnet. Die Ergebnisse werden angegeben als Mittel- werte (n = 4-6). Ergebnisse siehe Tabelle I.

2. Speichselsekretionshemmende Wirkung an der Ratte

Nach Lavy und Mulder (Arch. int. Pharmacodyn. 178 , 437-445, (1969)) erhielten mit 1,2 g/kg Urethan narkotisierte männliche THOM-Ratten steigende Dosen der Substanz i.v.. Die Speichelsekretion wurde durch s.c. Gabe von 2 mg/kg Pilocarpin ausgelöst. Der Speichel wurde mit Fließpapier aufge saugt, die von ihm eingenommene Fläche alle 5 Minuten planimetrisch bestimmt. Die Dosis der Substanz, die das Speichelvolumen um 50% verminderte, wurde graphisch ermittelt. Ergebnisse siehe Tabelle II.

3. In-situ-spasmolytische Wirkung bei Meerschweinchen

Männliche Meerschweinchen (500 bis 600 g Körpergewicht) wurden mit Urethan (1,2 g/kg, i.p.) anaesthetisiert; es wurden Kanülen in die Trachea, Jugularvene und die linke Halsschlagader eingeführt. Die Tiere wurden künstlich mit sauerstoff angereicherter Luft mittels einer positiven Druckpumpe bei einer Schlagfrequenz von 80 pro Minute beatmet. Es wurde ein 3 bis 4 cm langer Bauchschnitt vorgenommen und etwa 15 cm einer beweglichen Schlinge des Krummdarms (Ileum) wurde unter Erhalt der Blutzirkulation distal abgebunden. Der proximale Teil wurde mit einer Krebs-Ringer-Lösung gefüllt und ein Druckmesser mit einem Millar-Mikrospitzenkatheter (PC-450, 5F) wurde in den Darm eingeführt. Eine Glasröhre wurde vertikal im Abdomen plaziert und an der umgebenden Abdominalwand befestigt in der Art, daß bei Befüllung der Glasröhre mit Krebs-Ringer-Lösung das Tier als sein eigenes Organbad diente.

Die Glasröhre wurde mit Krebs-Ringer-Lösung soweit gefüllt, daß der gesamte Unterbauch eingetaucht war. Die zu prüfenden Wirkstoffe wurden über die Jugularvene injiziert; 5 Minuten später wurden mittels Methacholin (20 µg/kg i.a.) Kontraktionen erzeugt. Durch Aufzeichnen der prozentualen Unterdrückung der durch Methacholin erzeügten Kontraktionen gegen den Logarithmus der Dosismenge (mol/kg) der zu testenden Wirksubstanz wurden Dosis-Wirkungskurven erhalten.

Die Ergebnisse wurden als Mittelwerte (n = 4 bis 8) angegeben (siehe Tabelle II).

Nach den vorstehenden Angaben wurden beispielsweise die folgenden Verbindungen untersucht:

A = 5,11-Dihydro-11-[[7-methyl-2,7-diazaspiro[4,4]non-2-yl]carbonyl]-6H-pyrido[2,3-b][1,4]-benzodiazepin-6-on

B = 5,11-Dihydro-8-methyl-11-[[7-methyl-2,7-diazaspiro[4,4]non-2-yl]carbonyl]-6H-pyrido[2,3-b][1,4]-benzodiazepin-6-on

C = 5,11-Dihydro-11-[[6-methyl-2,6-diazaspiro[3,4]oct-2-yl]carbonyl]-6H-pyrido[2,3-b][1,4]benzodiazepin-6-on

D = 5,11-Dihydro-8-ethyl-11-[[6-methyl-2,6-diazaspiro[3,4]oct-2-yl]carbonyl]-6H-pyrido[2,3-b][1,4]-benzodiazepin-6-on

E = 5,11-Dihydro-8-methyl-11-[[2-methyl-2,6-diazaspiro[3,4]oct-6-yl]carbonyl]-6H-pyrido[2,3-b][1,4]-benzodiazepin6-on und als Vergleichssubstanzen

X = 11-[[2-[(Diethylamino)methyl]-1-piperidinyl]acetyl]-5,11-dihydro-6H-pyrido[2,3-b][1,4]benzodiazepin-6-on (vgl. US-Patent 4 550 107)

Y = 5,11-Dihydro-11-[(4-methyl-1-piperazinyl)acetyl]-6H-pyrido[2,3-b][1,4]benzodiazepin-6-on (Pirenzepin, siehe US-Patent Nr. 3 660 380)

und

Z = Atropin.

Tabelle I:

| Selektivität der bronchospasmolytischen Aktivität am Meerschweinchen: | | | | |
|---|---|---|---|---|
| | | Acetylcholin - Antagonismus | | |
| Substanz | Bronchien -log $ED_{50}$ ($molkg^{-1}$) i.v. | Harnblase -log $ED_{50}$ ($molkg^{-1}$) i.v. | Herz -log $ED_{50}$ ($molkg^{-1}$) i.v. | Verhältnis Beeinflussung von Bradycardie zu Bronchokonstriktion |
| A | 6,44 | 5,48 | 5,10 | 22 |
| B | 6,65 | 6,29 | 5,45 | 16 |
| C | 7,30 | 6,79 | 5,88 | 26 |
| D | 6,97 | 6,82 | 6,01 | 9 |
| E | 7,27 | 6,98 | 5,76 | 32 |
| X | 5,58 | 4,93 | 5,84 | 0,5 |
| Y | 6,57 | 5,84 | 5,90 | 5 |
| Z | 8,09 | 7,28 | 7,57 | 3 |

Tabelle II:

Selektivität der in-situ-spasmolytischen Aktivität in Relation zur speichelsekretionshemmenden Wirkung.

| Substanz | In-situ-Spasmolyse Meerschweinchen-Ileum -log $ED_{50}$ ($molkg^{-1}$) i.v. | Speichelsekretionshemmung Ratte -log $ED_{50}$ ($molkg^{-1}$) i.v. | Verhältnis Speichelsekretionshemmung zur spasmolytischen Aktivität |
|---|---|---|---|
| A | 6,05 | 5,46 | 4 |
| C | 7,32 | 6,37 | 9 |
| E | 6,97 | 6,60 | 2 |
| X | 5,48 | 5,0 | 3 |
| Y | 6,08 | 6,42 | 0,5 |
| Z | 7,28 | 7,60 | 0,5 |

Tabelle III:

| Dissoziationskonstanten ($K_B$-Werte) am Ileum und spontan schlagenden Vorhof des Meerschweinchens: | | | |
|---|---|---|---|
| | $K_B$ [mol/l] | | Selektivität |
| Substanz | Herz | Ileum | $K_B$ Herz zu $K_B$ Ileum |
| A | $1,23.10^{-6}$ | $2,69.10^{-7}$ | 4,6 |
| B | $2,51.10^{-7}$ | $5,89.10^{-8}$ | 4,3 |
| C | $3,39.10^{-7}$ | $7,24.10^{-8}$ | 4,7 |
| D | $1,1\ .10^{-7}$ | $2,24.10^{-8}$ | 4,9 |
| E | $4,68.10^{-7}$ | $6,46.10^{-8}$ | 7,2 |
| X | $1,05.10^{-7}$ | $6,17.10^{-7}$ | 0,17 |
| Y | $1,23.10^{-7}$ | $1,94.10^{-7}$ | 0,63 |
| Z | $1,41.10^{-9}$ | $8,13.10^{-10}$ | 1,7 |

Diskussion der Ergebnisse:

Die Substanzen der allgemeinen Formel I hemmen in niedrigen Dosierungen die Effekte von exogen zugeführtem Acetylcholin oder Methacholin auf die glatte Muskulatur von Bronchien, Blase oder Dünndarm, ohne daß die agonistische Wirkung auf die Herzfrequenz beeinflußt wird (Tabelle I und II). Zum Beispiel zeigen die Substanzen C und E eine sehr ausgeprägte Glattmuskelselektivität; 26- und 32-fach niedrigere Dosierungen sind notwendig, um die durch Acetylcholin ausgelöste Bronchokonstriktion zu hemmen im Vergleich zu der Acetylcholin-induzierten Bradykardie (Tabelle I). Die Substanzen der allgemeinen Formel I zeigen nicht nur Selektivität für die glatte Muskulatur im Vergleich zu Effekten, die durch kardiale Muskarinrezeptoren ausgelöst werden, sondern es sind auch höhere Dosierungen erforderlich, um die Pilocarpininduzierte Speichelsekretion zu hemmen (Tabelle II).

Die beobachtete in-vivo-Selektivität der Substanzen für die glatte Muskulatur stimmt überein mit den invitro-Untersuchungen. Die Substanzen besitzen eine höhere Affinität zu Muskarinrezeptoren des Ileums im Vergleich zu kardialen Muskarinrezeptoren (Tabelle III).

Aus den Daten geht hervor, daß die Substanzen der allgemeinen Formel I die Effekte von Muskarinagonisten auf die glatte Muskulatur, z. B. Bronchien, Blase und Ileum in Dosierungen hemmen, die keinen Einfluß auf die Herzfrequenz oder die Speichelsekretion haben. Die Vergleichssubstanzen Y (Pirenzepin) und Z (Atropin) zeigen keine Selektivität und beeinflussen alle oben genannten Effekte im gleichen Dosisbereich. Die Vergleichssubstanz X zeigt eine höhere Wirksamkeit auf kardiale Muskarinrezeptoren.

Alle Substanzen der allgemeinen Formel I zeichnen sich durch eine ausgesprochene Hydrolyse-Stabilität aus. Dadurch wird es möglich, lagerbeständige Lösungen zur parenteralen Applikation herzustellen.

Die nachfolgenden Beispiele sollen die Erfindung näher erläutern:

"Fp." bedeutet "Schmelzpunkt", "Z." bedeutet "Zersetzung". Für alle Verbindungen liegen befriedigende Elementaranalysen, IR-, UV-, $^1$H-NMR-, häufig auch Massenspektren vor. Prozente sind, sofern nichts anderes ausdrücklich erwähnt, immer Gewichtsprozente.

Beispiel 1

5,11-Dihydro-11-[[7-methyl-2,7-diazaspiro[4,4]non-2-yl]carbonyl]-6H-pyrido[2,3-b][1,4]benzodiazepin-6-on

Die Mischung aus 9,0 g (0,033 mol) 11-(Chlorcarbonyl)-5,11-dihydro-6H-pyrido[2,3-b][1,4]-benzodiazepin-6-on, 4,3 g (0,041 Mol) wasserfreiem Natriumcarbonat, 5,6 g (0,04 Mol) 2-Methyl-2,7-diazaspiro[4,4]nonan und 100 ml Acetonitril wurde 30 Minuten lang bei einer Reaktionstemperatur von 50° C gerührt. Das Lösungsmittel wurde anschließend im Vakuum abdestilliert, der verbleibende hochviskose Rückstand in 30 ml Wasser aufgenommen, natronalkalisch gestellt und mit Dichlormethan erschöpfend extrahiert. Die vereinigten Dichlormethanphasen wurden über Natriumsulfat getrocknet und eingedampft, der Rückstand an Kieselgel (35-70 mesh) unter Verwendung von Dichlormethan/Essigsäureethylester/Cyclohexan/Methanol/konz. Ammoniak 50/11/9/9/1, v/v/v/v/v, zum Eluieren chromatographisch gereinigt. Der nach dem Eindampfen der geeigneten Eluate verbleibende Rückstand wurde aus Wasser/methanol 1/9 umkristallisiert. Man erhielt 5,9 g (48 % der Theorie) an farblosen Kristallen vom Fp. 271-274° C.

| $C_{21}H_{23}N_5O_2$ (377,45). | | | |
|---|---|---|---|
| Ber.: | C 66,83 | H 6,14 | N 18,55 |
| Gef.: | 67,00 | 6,35 | 18,85 |

Beispiel 2

9-Chlor-5,11-dihydro-11-[[7-methyl-2,7-diazaspiro[4,4]non-2-yl]carbonyl]-6H-pyrido[2,3-b][1,4]benzodiazepin-6-on

Hergestellt analog Beispiel 1 aus 9-Chlor-11-(chlorcarbonyl)-5,11-dihydro-6H-pyrido[2,3-b][1,4]-benzodiazepin-6-on und 2-Methyl-2,7-diazaspiro[4,4]nonan in. einer Ausbeute von 41 % der Theorie. Farblose Kristalle vom Fp. 257-259° C (Dimethylacetamid).

| $C_{21}H_{22}N_5O_2$ (411,90). | | | | |
|---|---|---|---|---|
| Ber.: | C 61,24 | H 5,38 | Cl 8,61 | N 17,00 |
| Gef.: | 61,50 | 5,67 | 8,59 | 17,10 |

Beispiel 3

5,11-Dihydro-8-methyl-11-[[7-methyl-2,7-diazaspiro[4,4]non-2-yl]carbonyl]-6H-pyrido[2,3-b][1,4]-benzodiazepin-6-on

Hergestellt analog Beispiel 1 aus 11-(Chlorcarbonyl)-5,11-dihydro-8-methyl-6H-pyrido[2,3-b][1,4]-benzodiazepin-6-on und 2-Methyl-2,7-diazaspiro[4,4]nonan in einer Ausbeute von 71 % der Theorie. Farblose Kristalle vom Fp. 212-214° C (aus Acetonitril unter Verwendung von Aktivkohle).

| $C_{22}H_{25}N_5O_2$ (391,48). | | | |
|---|---|---|---|
| Ber.: | C 67,50 | H 6,44 | N 17,89 |
| Gef.: | 67,89 | 6,20 | 18,00 |

Beispiel 4

8-Chlor-5,11-dihydro-11-[[7-methyl-2,7-diazaspiro[4,4]non-2-yl]carbonyl]-6H-pyrido[2,3-b][1,4]benzodiazepin-6-on

Hergestellt analog Beispiel 1 aus 8-Chlor-11-(chlorcarbonyl)-5,11-dihydro-6H-pyrido[2,3-b][1,4]-benzodiazepin-6-on und 2-Methyl-2,7-diazaspiro[4,4]nonan in einer Ausbeute von 47 % der Theorie. Farblose Kristalle vom Fp. 214-215° C (Acetonitril).

| $C_{21}H_{22}ClN_5O_2$ (411,90). | | | | |
|---|---|---|---|---|
| Ber.: | C 61,24 | H 5,38 | Cl 8,61 | N 17,00 |
| Gef.: | 61,00 | 5,38 | 8,72 | 17,10 |

Beispiel 5

5,11-Dihydro-8-ethyl-11-[[7-methyl-2,7-diazaspiro[4,4]non-2-yl]carbonyl]-6H-pyrido[2,3-b][1,4]benzodiazepin-6-on

Hergestellt analog Beispiel 1 aus 11-(Chlorcarbonyl)-5,11-dihydro-8-ethyl-6H-pyrido[2,3-b][1,4]-benzodiazepin-6-on und 2-Methyl-2,7-diazaspiro[4,4]nonan in einer Ausbeute von 57 % der Theorie. Farblose Kristalle vom Fp. 216-217° C (aus Acetonitril unter Verwendung von Aktivkohle).

| $C_{23}H_{27}N_5O_2$ (405,50). | | | |
|---|---|---|---|
| Ber.: | C 68,13 | H 6,71 | N 17,27 |
| Gef.: | 68,30 | 6,81 | 17,30 |

Beispiel 6

5,11-Dihydro-9-methyl-11-[[7-methyl-2,7-diazaspiro[4,4]non-2-yl]carbonyl]-6H-pyrido[2,3-b][1,4]-benzodiazepin-6-on

Hergestellt analog Beispiel 1 aus 11-(Chlorcarbonyl)-5,11-dihydro-9-methyl-6H-pyrido[2,3-b][1,4]-benzodiazepin-6-on und 2-Methyl-2,7-diazaspiro[4,4]nonan in einer Ausbeute von 9 % der Theorie. Farblose Kristalle vom Fp. 253-255° C (Acetonitril).

| $C_{22}H_{25}N_5O_2$ (391,48). | | | |
|---|---|---|---|
| Ber.: | C 67,50 | H 6,44 | N 17,89 |
| Gef.: | 66,99 | 6,47 | 17,62 |

Beispiel 7

8-Brom-5,11-dihydro-11-[[7-methyl-2,7-diazaspiro[4,4]non-2-yl]carbonyl]-6H-pyrido[2,3-b][1,4]benzodiazepin-6-on

Hergestellt analog Beispiel 1 aus 8-Brom-11-(chlorcarbonyl)5,11-dihydro-6H-pyrido[2,3-b][1,4]-benzodiazepin-6-on und 2-Methyl-2,7-diazaspiro[4,4]nonan in einer Ausbeute von 64 % der Theorie. Farblose Kristalle vom Fp. 227-229° C (Z.) (aus Acetonitril/Ethanol 1/1 v/v).

| $C_{21}H_{22}BrN_5O_2$ (456,36). | | | | |
|---|---|---|---|---|
| Ber.: | C 55,27 | H 4,86 | Br 17,51 | N 15,35 |
| Gef.: | 55,60 | 5,10 | 17,35 | 15,13 |

Beispiel 8

5,11-Dihydro-11-[[7-ethyl-2,7-diazaspiro[4,4]non-2-yl]carbonyl]-6H-pyrido[2,3-b][1,4]benzodiazepin-6-on

Hergestellt analog Beispiel 1 aus 11-(Chlorcarbonyl)-5,11-dihydro-6H-pyrido[2,3-b][1,4]benzodiazepin-6-on und 2-Ethyl-2,7-diazaspiro[4,4]nonan in einer Ausbeute von 85 % der Theorie. Farblose Kristalle vom Fp. 214-215° C (Acetonitril).

| $C_{22}H_{25}N_5O_2$ (391,48). | | | |
|---|---|---|---|
| Ber.: | C 67,50 | H 6,44 | N 17,89 |
| Gef.: | 67,44 | 6,70 | 18,10 |

Beispiel 9

5,11-Dihydro-11-[[7-ethyl-2,7-diazaspiro[4,4]non-2-yl]carbonyl]-8-methyl-6H-pyrido[2,3-b][1,4]benzodiazepin-6-on

Hergestellt analog Beispiel 1 aus 11-(Chlorcarbonyl)-5,11-dihydro-8-methyl-6H-pyrido[2,3-b][1,4]-benzodiazepin-6-on und 2-Ethyl-2,7-diazaspiro[4,4]nonan in einer Ausbeute von 74 % der Theorie. Farblose Kristalle vom Fp. 178-180 °C (Acetonitril).

| $C_{23}H_{27}N_5O_2$ (405,50). | | | |
|---|---|---|---|
| Ber.: | C 68,13 | H 6,71 | N 17,27 |
| Gef.: | 67,94 | 6,70 | 17,57 |

Beispiel 10

5,11-Dihydro-11-[[7-ethyl-2,7-diazaspiro[4,4]non-2-yl]carbonyl]-9-methyl-6H-pyrido[2,3-b][1,4]benzodiazepin-6-on

Hergestellt analog Beispiel 1 aus 11-(Chlorcarbonyl)-5,11-dihydro-9-methyl-6H-pyrido[2,3-b][1,4]-benzodiazepin-6-on und 2-Ethyl-2,7-diazaspiro[4,4]nonan in einer Ausbeute von 85 % der Theorie. Farblose Kristalle vom Fp. 244-246 °C (Acetonitril).

| $C_{23}H_{27}N_5O_2$ (405,50). | | | |
|---|---|---|---|
| Ber.: | C 68,13 | H 6,71 | N 17,27 |
| Gef.: | 67,83 | 6,92 | 17,17 |

Beispiel 11

9-Chlor-5,11-dihydro-11-[[7-ethyl-2,7-diazaspiro[4,4]non-2-yl]carbonyl]-6H-pyrido[2,3-b][1,4]benzodiazepin-6-on

Hergestellt analog Beispiel 1 aus 9-Chlor-11-chlorcarbonyl)-5,11-dihydro-6H-pyrido[2,3-b][1,4]-benzodiazepin-6-on und 2-Ethyl-2,7-diazaspiro[4,4]nonan in einer Ausbeute von 75 % der Theorie. Farblose Kristalle vom Fp. 224,0-225,5 °C (Acetonitril).

| $C_{22}H_{24}ClN_5O_2$ (425,93). | | | | |
|---|---|---|---|---|
| Ber.: | C 62,04 | H 5,68 | Cl 8,32 | N 16,44 |
| Gef.: | 61,89 | 5,65 | 8,35 | 16,34 |

Beispiel 12

5,11-Dihydro-11-[[6-methyl-2,6-diazaspiro[3,4]oct-2-yl]carbonyl]-6H-pyrido[2,3-b][1,4]benzodiazepin-6-on

a.) 4,4-Bis-(ethoxycarbonyl)-1-methyl-2-pyrrolidinon

Die Mischung aus 221,6 g (0,90 mol) 1,1,2-Ethantricarbonsäuretriethylester, 116,3 g (0,90 mol) 1,3,5-Trimethylhexahydro-1,3,5-triazin und 20,6 g (0,18 mol) Trifluoressigsäure wurde 20 Stunden bei einer Reaktionstemperatur von 100 °C gerührt. Nach dem Erkalten wurde die Mischung mit 1 Liter Toluol verdünnt und anschließend mit dreimal je 100 ml 10-proz. wässeriger Salzsäure extrahiert. Die salzsauren wässerigen Extrakte wurden vereinigt und einmal mit 100 ml Ethylacetat ausgezogen. Die erhaltenen organischen Phasen wurden zusammengegeben, einmal mit 200 ml einer gesättigten wässerigen Natriumhydrogencarbonat-Lösung durchge schüttelt und dann noch dreimal mit je 300 ml Wasser gewaschen, über Natriumsulfat getrocknet und im Vakuum eingedampft. Das erhaltene farblose Öl (Ausbeute: 200 g, d. h. 91 % der Theorie) wurde ohne weitere Reinigung in der folgenden Stufe verwendet.

b.) 3,3-Bis-(hydroxymethyl)-1-methylpyrrolidin

Zu der Suspension von 75,0 g (1,976 mol) Lithiumaluminiumhydrid in 1 Liter wasserfreiem Tetrahydrofuran tropfte man die Lösung von 160,0 g (0,658 mol) 4,4-Bis-(ethoxycarbonyl)-1-methyl-2-pyrrolidinon in 700 ml trockenem Tetrahydrofuran so zu, daß die Reaktion unter Kontrolle gehalten werden konnte und das Tetrahydrofuran mäßig siedete. Nach Beendigung der Zugabe kochte man unter Rühren noch 4 Stunden unter Rückfluß. Man ließ erkalten und tropfte unter Rühren und äußerer Kühlung mit Eiswasser nacheinander 75 ml Wasser, 75 ml 15-proz. Natronlauge und 215 ml Wasser ein. Der erhaltene Niederschlag wurde abgenutscht, nochmals mit Tetrahydrofuran aufgeschlämmt und aufgekocht, danach abermals abgenutscht. Die erhaltenen Filtrate wurden vereinigt, sorgfältig über Natriumsulfat getrocknet und im Vakuum eingedampft. Das in einer Ausbeute von 71,4 g (75 % der Theorie) anfallende farblose, viskose Öl wurde ohne weitere Reinigung in der nächsten Stufe weiterverarbeitet.

c.) 3,3-Bis-(brommethyl)-1-methylpyrrolidin

Die Mischung aus 28,0 g (0,193 mol) der vorgenannten Verbindung und 250 ml 63-proz. wässeriger Bromwasserstoffsäure wurde im Bombenrohr 24 Stunden lang auf 180 °C erhitzt. Nach dem Erkalten wurde im Vakuum zur Trockene eingedampft, der Rückstand, der in Wasser relativ schwer löslich war, in 400 ml Wasser aufgenommen und mit überschüssigem Kaliumcarbonat behandelt. Die erhaltene Suspension wurde mit Essigsäureethylester erschöpfend extrahiert, die Extrakte wurden vereinigt und über Natriumsulfat getrocknet. Der nach dem Abdampfen des Lösemittels verbleibende Rückstand (Ausbeute: 49,0 g, das sind 94 % der Theorie), ein farbloses Öl, wurde ohne weitere Reinigung in der nächsten Stufe eingesetzt. $R_F$ 0,9 (Macherey-Nagel, Polygram(R) SIL G/$UV_{254}$, pre-coated plastic sheets for TLC; Fließmittel: Essigsäureethylester/Methanol/konz. Ammoniak 100/30/3, v/v/v).

d.) 6-Methyl-2-[(4-methylphenyl)sulfonyl]-2,6-diazaspiro[3,4]octan

Zu der Lösung von 51,3 g (0,3 mol) p-Toluolsulfonamid und 33,6 g (0,6 mol) Kaliumhydroxid in 160 ml Wasser gab man die Lösung von 80,0 g (0,295 mol) 3,3-Bis-(brommethyl)-1-methylpyrrolidin in 4,8 Liter

Dioxan und kochte die erhaltene Mischung unter Rückfluß. Nach 17 und weiteren 24 Stunden gab man nochmals jeweils 10,0 g (0,0584 mol) p-Toluolsulfonamid und 6,7 g (0,12 mol) Kaliumhydroxid, gelöst in 30 ml Wasser, zu und erhitzte abschließend nochmals für 24 Stunden zum Rückfluß. Die erhaltene Reaktionsmischung wurde im Vakuum eingedampft, der verbleibende Rückstand zwischen Wasser und Ethylacetat verteilt, die organische Schicht einmal mit Wasser gewaschen, über Natriumsulfat getrocknet und am Rotationsverdampfer im Vakuum eingedampft. Der verbleibende Rückstand (64,0 g) wurde mit Diisopropylether verrührt und abgenutscht, der verbleibende Nutschenrückstand schließlich aus heißem Cyclohexan unter Verwendung von Aktivkohle umkristallisiert. Man erhielt 50,5 g (61 % der Theorie) an farblosen Kristallen vom Fp. 83-85 °C.

| $C_{14}H_{20}N_2O_2S$ (280,39) | | | | |
|---|---|---|---|---|
| Ber.: | C 59,97 | H 7,19 | N 9,99 | S 11,43 |
| Gef.: | 59,66 | 7,16 | 10,30 | 11,51 |

e.) 6-Methyl-2,6-diazaspiro[3,4]octan

Die Mischung aus 48,5 g (0,173 mol) 6-Methyl-2-[(4-methylphenyl)-sulfonyl]-2,6-diazaspiro[3,4]octan, 199 ml (ca. 0,7 mol) einer ca. 3,5 molaren Lösung von Natrium-bis-(2-methoxyethoxy)dihydroaluminat in Toluol und 300 ml trockenem Toluol wurde unter Rühren 15 Stunden auf 60 °C und 6 Stunden auf 80 °C erhitzt. Nach Beendigung der Umsetzung tropfte man vorsichtig unter äußerer Kühlung mit Eiswasser 20-proz. wässerige Natronlauge zu, bis die Wasserstoffentwicklung beendet war, trennte die Toluolphase ab, trocknete sie über Natriumsulfat und dampfte unter Verwendung einer Vigreux-Kolonne bei einem Druck von 50 mmHg vorsichtig ein. Die gesuchte Verbindung hatte den Sdp.$_{22\ mmHg}$ 80-85 °C und erwies sich als farblose, leicht bewegliche, aminartig riechende Flüssigkeit. Ausbeute: 7,5 g (34 % der Theorie).

f.) 5,11-Dihydro-11-[[6-methyl-2,6-diazaspiro[3,4]oct-2-yl]-carbonyl]-6H-pyrido[2,3-b][1,4]benzodiazepin-6-on

Hergestellt analog Beispiel 1 aus 11-(Chlorcarbonyl)-5,11-dihydro-6H-pyrido[2,3-b][1,4]benzodiazepin-6-on und 6-Methyl-2,6-diazaspiro[3,4]octan in einer Ausbeute von 26 % der Theorie. Farblose Kristalle vom Fp. 225,5-227,0 °C (Acetonitril).

| $C_{20}H_{21}N_5O_2$ (363,42). | | | |
|---|---|---|---|
| Ber.: | C 66,10 | H 5,82 | N 19,27 |
| Gef.: | 66,18 | 5,82 | 19,17 |

Beispiel 13

5,11-Dihydro-8-ethyl-11-[[6-methyl-2,6-diazaspiro[3,4]oct-2-yl]carbonyl]-6H-pyrido[2,3-b][1,4]benzodiazepin-6-on

Hergestellt analog Beispiel 1 aus 11-(Chlorcarbonyl)-5,11-dihydro-8-ethyl-6H-Pyrido[2,3-b][1,4]-benzodiazepin-6-on und 6-Methyl-2,6-diazaspiro[3,4]octan in einer Ausbeute von 47 % der Theorie. Farblose Kristalle vom Fp. 215-217 °C (Acetonitril).

| $C_{22}H_{25}N_5O_2$ (391,48). | | | |
|---|---|---|---|
| Ber.: | C 67,50 | H 6,44 | N 17,89 |
| Gef.: | 67,27 | 6,41 | 17,88 |

Beispiel 14

5,11-Dihydro-8-methyl-11-[[6-methyl-2,6-diazaspiro[3,4]oct-2-yl]carbonyl]-6H-pyrido[2,3-b][1,4]-benzodiazepin-6-on

Hergestellt analog Beispiel 1 aus 11-(Chlorcarbonyl)-5,11-dihydro-8-methyl-6H-pyrido[2,3-b][1,4]-benzodiazepin-6-on und 6-Methyl-2,6-diazaspiro[3,4]octan in einer Ausbeute von 34 % der Theorie. Farblose Kristalle vom Fp. 220-223 °C (Acetonitril).

| $C_{21}H_{23}N_5O_2$ (377,45). | | | |
|---|---|---|---|
| Ber.: | C 66,83 | H 6,14 | N 18,55 |
| Gef.: | 66,59 | 6,12 | 18,41 |

Beispiel 15

5,11-Dihydro-9-methyl-11-[[6-methyl-2,6-diazaspiro[3,4]oct-2-yl]carbonyl]-6H-pyrido[2,3-b][1,4]-benzodiazepin-6-on

Hergestellt analog Beispiel 1 aus 11-(Chlorcarbonyl)-5,11-dihydro-9-methyl-6H-pyrido[2,3-b][1,4]-benzodiazepin-6-on und 6-Methyl-2,6-diazaspiro[3,4]octan in einer Ausbeute von 41 % der Theorie. Farblose Kristalle vom Fp. 242-245 °C (nach zweimaligem Umkristallisieren aus Acetonitril).

| $C_{21}H_{23}N_5O_2$ (377,45). | | | |
|---|---|---|---|
| Ber.: | C 66,83 | H 6,14 | N 18,55 |
| Gef.: | 66,57 | 6,23 | 18,41 |

Beispiel 16

9-Chlor-5,11-dihydro-11-[[6-methyl-2,6-diazaspiro[3,4]oct-2-yl]carbonyl]-6H-Pyrido[2,3-b][1,4]benzodiazepin-6-on

Hergestellt analog Beispiel 1 aus 9-Chlor-11-(chlorcarbonyl)5,11-dihydro-6H-pyrido[2,3-b][1,4]-benzodiazepin-6-on und 6-Methyl-2,6-diazaspiro[3,4]octan in einer Ausbeute von 37 % der Theorie. Farblose Kristalle vom Fp. 229-230 °C (Acetonitril).

| $C_{20}H_{20}ClN_5O_2$ (397,87). | | | | |
|---|---|---|---|---|
| Ber.: | C 60,38 | H 5,07 | Cl 8,91 | N 17,60 |
| Gef.: | 60,21 | 5,01 | 8,90 | 17,94 |

Beispiel 17

5,11-Dihydro-11-[[2-methyl-2,6-diazaspiro[3,4]oct-6-yl]carbonyl]-6H-pyrido[2,3-b][1,4]benzodiazepin-6-on

a.) 4,4-Bis-(ethoxycarbonyl)-1-(phenylmethyl)-2-pyrrolidinon

Hergestellt analog Beispiel 12a aus 1,1,2-Ethantricarbonsäuretriethylester und 1,3,5-Tris-(phenylmethyl)-hexahydro-1,3,5-triazin in Gegenwart von Trifluoressigsäure in einer Ausbeute von 90 % der Theorie. Farbloses, viskoses Öl, das ohne weitere Reinigung in der nächsten Stufe eingestzt wurde.

b.) 3,3-Bis-(hydroxymethyl)-1-(phenylmethyl)-pyrrolidin

Hergestellt analog Beispiel 12b) aus 4,4-Bis-(ethoxycarbonyl)-1-(Phenylmethyl)-2-pyrrolidinon und Lithiumaluminiumhydrid in einer Ausbeute von 78 % der Theorie. Farbloses, hochviskoses Öl, das nach etwa einwöchigem Stehen bei Raumtemperatur durchkristallisierte und ohne weitere Reinigung in der nächsten Stufe weiterverarbeitet wurde.

c.) 3,3-Bis-(brommethyl)-1-(phenylmethyl)-pyrrolidin-hydrobromid

Die Mischung aus 22,0 g (0,1 mol) 3,3-Bis-(hydroxymethyl)-1-(phenylmethyl)-pyrrolidin und 130 ml 63-proz. wässeriger Bromwasserstoffsäure wurde im Bombenrohr 24 Stunden lang auf 180° C erhitzt. Nach dem Erkalten wurde im Vakuum zur Trockne eingedampft, der kristalline Rückstand mit 100 ml kaltem Wasser verrührt und anschließend abgenutscht. Man kristalli sierte aus siedendem Wasser um und erhielt 35,0 g (82 % der Theorie) an farblosen Kristallen vom Fp. 222-225° C.

| $C_{13}H_{17}Br_2NxHBr$ (428,0). | | | | |
|---|---|---|---|---|
| Ber.: | C 36,48 | H 4.24 | Br 56,01 | N 3,27 |
| Gef.: | 36,56 | 4,10 | 55,73 | 3,02 |

d.) 6-(Phenylmethyl)-2-[(4-methylphenyl)sulfonyl]-2,6-diazaspiro[3,4]octan

Hergestellt analog Beispiel 12d) aus 3,3-Bis-(brommethyl)-1-(Phenylmethyl)-pyrrolidin-hydrobromid, Kaliumhydroxid und p-Toluolsulfonamid in einer Ausbeute von 60 % der Theorie. Die erhaltene kristalline Substanz wurde ohne Umkristallisation oder sonstige Reinigung direkt weiterverarbeitet.

e.) 6-(Phenylmethyl)-2,6-diazaspiro[3,4]octan

Hergestellt analog Beispiel 12e) aus 6-(Phenylmethyl)-2-[(4-methylphenyl)sulfonyl]-2,6-diazaspiro[3,4]-octan und Natrium-bis-(2-methoxyethoxy)-dihydroaluminat in einer Ausbeute von 41 % der Theorie. Farblose Flüssigkeit vom $Sdp._{18\ mmHg}$ 161-170° C und $R_F$ 0,25 (Macherey-Nagel, Polygram(R) SIL G/$UV_{254}$, pre-

coated plastic sheets for TLC; Fließmittel: Dichlormethan/Methanol/konz. wässeriges Ammoniak 68/15/15/2, v/v/v/v).

f.) 2-Methyl-6-(phenylmethyl)-2,6-diazaspiro[3,4]octan

10,8 g (0,0534 mol) 6-(Phenylmethyl)-2,6-diazaspiro[3,4]-octan wurden in 300 ml Ethanol gelöst, mit 5 ml (ca. 0,062 mol) einer 37-proz. wässerigen Formalin-Lösung versetzt und 50 Minuten unter Rückfluß gekocht. Man ließ erkalten, gab 5,0 g Raney-Nickel zu und hydrierte die Mischung 5 Stunden bei Raumtemperatur und 4 bar Wasserstoffdruck. Der Katalysator wurde abfiltriert, das Filtrat im Vakuum eingedampft, der verbleibende Rückstand an Kieselgel mittels HPLC und unter Verwendung von Dichlormethan/Methanol/Cyclohexan/konz. wässerigem Ammoniak 68/15/15/2 zum Eluieren gereinigt. Eindampfen geeigneter Fraktionen ergab die gesuchte Verbindung in Form eines zähflüssigen, farblosen Öls. Ausbeute: 6,1 g (53 % der Theorie). $R_F$ 0,54 (Macherey-Nagel, Polygram(R) SIL G/$UV_{254}$ pre-coated plastic sheets for TLC; Fließmittel: Dichlormethan/ethanol/Cyclohexan/konz. wässeriges Ammoniak 68/15/15/2, v/v/v/v).

g.) 2-Methyl-2,6-diazaspiro[3,4]octan

Zu der Lösung von 6,1 g (0.0282 mol) 2-Methyl-6-(phenylmethyl)-2,6-diazaspiro[3,4]octan in 60 ml Ethanol gab man 4,0 g 10-proz. Palladium/Tierkohle-Katalysator und hydrierte anschließend 5 Stunden bei Raumtemperatur und einem Wasserstoffdruck von 5 bar. Man filtrierte, dampfte das Filtrat bei vermindertem Druck (100 mmHg) ein und erhielt als Rückstand ein farbloses Öl vom $R_F$ 0,1 (Macherey-Nagel, Polygram(R) SIL G/$UV_{254}$, pre-coated plastic sheets for TLC; Fließmittel: Dichlormethan/Methanol/Cyclohexan/konz. wässeriges Ammoniak 68/20/10/5, v/v/v/v).
Ausbeute: 3,2 g (90 % der Theorie).

h.) 5,11-Dihydro-11-[[2-methyl-2,6-diazaspiro[3,4]oct-2-yl]carbonyl]-6H-pyrido[2,3-b][1,4]benzodiazepin-6-on

Hergestellt analog Beispiel 1 aus 11-(Chlorcarbonyl)-5,11-dihydro-6H-pyrido[2,3-b][1,4]benzodiazepin-6-on und 2-Methyl-2,6-diazaspiro[3,4]octan in einer Ausbeute von 46 % der Theorie. Farblose Kristalle vom Fp. 273-275 ° C (Acetonitril).

| $C_{20}H_{21}N_5O_2$ (363,42). | | | |
|---|---|---|---|
| Ber.: | C 66,10 | H 5,82 | N 19,27 |
| Gef.: | 66,25 | 5,89 | 18,90 |

Beispiel 18

5,11-Dihydro-11-[[6-(phenylmethyl)-2,6-diazaspiro[3,4]oct-2-yl]carbonyl]-6H-pyrido[2,3-b][1,4]benzodiazepin-6-on

Hergestellt analog Beispiel 1 aus 11-(Chlorcarbonyl)-5,11-dihydro-6H-pyrido[2,3-b][1,4]benzodiazepin-6-on und 6-(Phenylmethyl)-2,6-diazaspiro[3,4]octan in einer Ausbeute von 20 % der Theorie. Farblose Kristalle vom Fp. 193-195 ° C (Diisopropylether).

| $C_{26}H_{25}N_5O_2$ (439,52). | | | |
|---|---|---|---|
| Ber.: | C 71,05 | H 5,73 | N 15,93 |
| Gef.: | 70,75 | 5,91 | 15,76 |

Beispiel 19

5,11-Dihydro-8-methyl-11-[[2-methyl-2,6-diazaspiro[3,4]oct-6-yl]carbonyl]-6H-pyrido[2,3-b][1,4]-benzodiazepin-6-on

Hergestellt analog Beispiel 1 aus 11-(Chlorcarbonyl)-5,11-dihydro-8-methyl-6H-pyrido[2,3-b][1,4]-benzodiazepin-6-on und 2-Methyl-2,6-diazaspiro[3,4]octan in einer Ausbeute von 43 % der Theorie. Farblose Kristalle vom FP. 184,0-184,5° C (Acetonitril).

| $C_{21}H_{23}N_5O_2$ (377,45). | | | |
|---|---|---|---|
| Ber.: | C 66,83 | H 6,14 | N 18,55 |
| Gef.: | 66,79 | 6,43 | 18,65 |

Beispiel 20

5,11-Dihydro-8-ethyl-11-[[2-methyl-2,6-diazaspiro[3,4]oct-6-yl]carbonyl]-6H-pyrido[2,3-b][1,4]benzodiazepin-6-on

Hergestellt analog Beispiel 1 aus 11-(Chlorcarbonyl)-5,11-dihydro-8-ethyl-6H-pyrido[2,3-b][1,4]-benzodiazepin-6-on und 2-Methyl-2,6-diazaspiro[3,4]octan in einer Ausbeute von 34 % der Theorie. Farblose Kristalle vom Fp.245-246° C (Acetonitril).

| $C_{22}H_{25}N_5O_2$ (391,48). | | | |
|---|---|---|---|
| Ber.: | C 67,50 | H 6,44 | N 17,89 |
| Gef.: | 67,30 | 6,33 | 17,88 |

Beispiel 21

5,11-Dihydro-9-methyl-11-[[2-methyl-2,6-diazaspiro[3,4]oct-6-yl]carbonyl]-6H-pyrido[2,3-b][1,4]-benzodiazepin-6-on

Hergestellt analog Beispiel 1 aus 11-(Chlorcarbonyl)-5,11-dihydro-9-methyl-6H-pyrido[2,3-b][1,4]-benzodiazepin-6-on und 2-Methyl-2,6-diazaspiro[3,4]octan in einer Ausbeute von 41 % der Theorie. Farblose Kristalle vom Fp.284-285° C (Acetonitril).

| $C_{21}H_{23}N_5O_2$ (377,45). | | | |
| --- | --- | --- | --- |
| Ber.: | C 66,83 | H 6,14 | N 18,55 |
| Gef.: | 67,08 | 6,12 | 18,85 |

Beispiel 22

9-Chlor-5,11-dihydro-11-[[2-methyl-2,6-diazaspiro[3,4]oct-6-yl]carbonyl]-6H-pyrido[2,3-b][1,4]benzodiazepin-6-on

Hergestellt analog Beispiel 1 aus 9-Chlor-11-(chlorcarbonyl)-5,11-dihydro-6H-pyrido[2,3-b][1,4]-benzodiazepin-6-on und 2-Methyl-2,6-diazaspiro[3,4]octan in einer Ausbeute von 18 % der Theorie. Farblose Kristalle vom Fp.276-277° C (nach zweimaligem Umkristallisieren aus Acetonitril).

| $C_{20}H_{20}ClN_5O_2$ (397,87). | | | | |
| --- | --- | --- | --- | --- |
| Ber.: | C 60,38 | H 5,07 | Cl 8,91 | N 17,60 |
| Gef.: | 60,74 | 4,92 | 8,91 | 17,77 |

Beispiel 23

8-Chlor-5,11-dihydro-11-[[2-methyl-2,6-diazaspiro[3,4]oct-6-yl]carbonyl]-6H-pyrido[2,3-b][1,4]benzodiazepin-6-on

Hergestellt analog Beispiel 1 aus 8-Chlor-11-(chlorcarbonyl)-5,11-dihydro-6H-pyrido[2,3-b][1,4]-benzodiazepin-6-on und 2-Methyl-2,6-diazaspiro[3,4]octan in einer Ausbeute von 50 % der Theorie. Farblose Kristalle vom Fp.231-232° C (Acetonitril).

| $C_{20}H_{20}ClN_5O_2$ (397,87). | | | | |
| --- | --- | --- | --- | --- |
| Ber.: | C 60,38 | H 5,07 | Cl 8,91 | N 17,60 |
| Gef.: | 60,62 | 5,20 | 8,61 | 17,54 |

Beispiel 24

5,11-Dihydro-11-[[6-ethyl-2,6-diazaspiro[3,4]oct-2-yl]carbonyl]-8-methyl-6H-pyrido[2,3-b][1,4]benzodiazepin-6-on

a.) 4-Cyan-4-(ethoxycarbonyl)-1-ethyl-2-pyrrolidinon

Hergestellt analog Beispiel 12a) aus 2-Cyanbernsteinsäurediethylester und 1,3,5-Triethyl-hexahydro-1,3,5-triazin in gegenwart von Trifluoressigsäure in einer Ausbeute von 89 % der Theorie. Farbloses Öl, das als Rohprodukt ohne Reinigung weiterverarbeitet wurde.

b.) 3-(Aminomethyl)-3-(hydroxymethyl)-1-ethylpyrrolidin

Hergestellt analog Beispiel 12b) aus 4-Cyan-4-(ethoxycarbonyl)-1-ethyl-2-pyrrolidinon und Lithiumaluminiumhydrid in Tetrahydrofuran in einer Ausbeute von 74 % der Theorie. Farbloses Öl vom $R_F$ 0,25 (Macherey-Nagel, Polygram(R) SIL G/$UV_{254}$, pre-coated plastic sheets for TLC; Fließmittel: Essigsäureethylester//Methanol/Cyclohexan/konz. wässeriges Ammoniak 68/15/15/2, v/v/v/v).

c.) 3-(Aminomethyl)-3-(brommethyl)-1-ethylpyrrolidin-dihydrobromid

Hergestellt analog Beispiel 12c) aus 3-(Aminomethyl)-3-(hydroxymethyl)-1-ethylpyrrolidin und 63-proz. wässeriger Bromwasserstoffsäure in einer Ausbeute von 94 % der Theorie. Das rohe, bräunlich gefärbte Salz wurde ohne weitere Reinigung der folgenden Cyclisierungsreaktion unterworfen.

d.) 6-Ethyl-2,6-diazaspiro[3,4]octan

Die Lösung von 149,4 g (0,39 mol) 3-(Aminomethyl)-3-(brommethyl)-1-ethylpyrrolidin in 2 Liter Dioxan wurde vorsichtig mit der Mischung von 130 g (3,25 mol) Natriumhydroxid und 120 ml Wasser versetzt und anschließend unter Rühren und Rückfluß 8 Stunden zum Sieden erhitzt. Nach dem Erkalten wurde filtriert, aus dem Filtrat die wässerige Phase abgetrennt und verworfen, die organische Phase bei leicht vermindertem Druck (100 mmHg) und unter Verwendung einer Vigreux-Kolonne vom Lösungsmittel befreit. Der verbleibende Rückstand ergab bei der Destillation im Wasserstrahlvakuum 15,8 g (29 % der Theorie) eines farblosen Öls vom $Sdp._{20\ mmHg}$ 83-87° C, das durch MS, IR- und $^1$H-NMR-Spektren als die gesuchte Verbindung identifiziert wurde.

e.) 5,11-Dihydro-11-[[6-ethyl-2,6-diazaspiro[3,4]oct-2-yl]carbonyl]-8-methyl-6H-pyrido[2,3-b][1,4]-benzodiazepin6-on

Hergestellt analog Beispiel 1 aus 11-(Chlorcarbonyl)-5,11-dihydro-8-methyl-6H-pyrido[2,3-b][1,4]-benzodiazepin-6-on und 6-Ethyl-2,6-diazaspiro[3,4]octan in einer Ausbeute von 27 % der Theorie. Farblose Kristalle vom Fp. 216-217° C (Acetonitril).

| $C_{22}H_{25}N_5O_2$ (391,48). | | | |
|---|---|---|---|
| Ber.: | C 67,50 | H 6,44 | N 17,89 |
| Gef.: | 67,39 | 6,20 | 17,60 |

Beispiel 25

5,11-Dihydro-11-[[6-ethyl-2,6-diazaspiro[3,4]oct-2-yl]carbonyl]-6H-pyrido[2,3-b][1,4]benzodiazepin-6-on

Hergestellt analog Beispiel 1 aus 11-(Chlorcarbonyl)-5,11-dihydro-6H-pyrido[2,3-b][1,4]benzodiazepin-6-on und 6-Ethyl-2,6-diazaspiro[3,4]octan in einer Ausbeute von 15 % der Theorie. Farblose Kristalle vom Fp. 221-223° C (Acetonitril).

| $C_{21}H_{23}N_5O_2$ (377,45). | | | |
|---|---|---|---|
| Ber.: | C 66,83 | H 6,14 | N 18,55 |
| Gef.: | 66,77 | 6,32 | 18,32 |

Beispiel 26

5,11-Dihydro-11-[[6-ethyl-2,6-diazaspiro[3,4]oct-2-yl]carbonyl]-9-methyl-6H-pyrido[2,3-b][1,4]benzodiazepin-6-on

Hergestellt analog Beispiel 1 aus 11-(Chlorcarbonyl)-5,11-dihydro-9-methyl-6H-pyrido[2,3-b][1,4]-benzodiazepin-6-on und 6-Ethyl-2,6-diazaspiro[3,4]octan in einer Ausbeute von 26 % der Theorie. Farblose Kristalle vom Fp. 169-171 °C (Acetonitril).

| $C_{22}H_{25}N_5O_2$ (391,48). | | | |
|---|---|---|---|
| Ber.:<br>Gef.: | C 67,50<br>67,46 | H 6,44<br>6,09 | N 17,89<br>17,49 |

Beispiel 27

9-Chlor-5,11-dihydro-11-[[6-ethyl-2,6-diazaspiro[3,4]oct-2-yl]carbonyl]-6H-pyrido[2,3-b][1,4]benzodiazepin-6-on

Hergestellt analog Beispiel 1 aus 9-Chlor-11-(chlorcarbonyl)-5,11-dihydro-6H-pyrido[2,3-b][1,4]-benzodiazepin-6-on und 6-Ethyl-2,6-diazaspiro[3,4]octan in einer Ausbeute von 19 % der Theorie. Farblose Kristalle vom Fp. 158-160 °C (Acetonitril) und $R_F$ 0,63 (Macherey-Nagel, Polygram(R) SIL G/$UV_{254}$, pre-coated plastic sheets for TLC; Fließmittel: Dichlormethan/Essigsäureethylester//Methanol/Cyclohexan/konz. wässeriges Ammoniak 62/16,7/10/10/1,3, v/v/v/v/v).

| $C_{21}H_{22}ClN_5O_2$ (411,90). | | | | |
|---|---|---|---|---|
| Ber.:<br>Gef.: | C 61,24<br>61,25 | H 5,38<br>5,36 | Cl 8,61<br>8,63 | N 17,00<br>17,08 |

Beispiel 28

5,11-Dihydro-11-[[6-propyl-2,6-diazaspiro[3,4]oct-2-yl]carbonyl]-6H-pyrido[2,3-b][1,4]benzodiazepin-6-on

a.) 4-Cyan-4-(ethoxycarbonyl)-1-propyl-2-pyrrolidinon

Hergestellt analog Beispiel 12a) aus Cyanbernsteinsäurediethylester und 1,3,5-Tripropyl-hexahydro-1,3,5-triazin in Gegenwart von Trifluoressigsäure in einer Ausbeute von 65 % der Theorie Farblose Flüssigkeit von $R_F$ 0,72 (MachereyNagel, Polygram(R) SIL G/$UV_{254}$, pre-coated plastic sheets for TLC; Fließmittel: Essigsäureethylester/Petrolether 1/1, v/v).

b.) 3-(Aminomethyl)-3-(hydroxymethyl)-1-propylpyrrolidin

Hergestellt analog Beispiel 12b) aus 4-Cyan-4-(ethoxycarbonyl)-1-propyl-2-pyrrolidinon und Lithiumaluminiumhydrid in einer Ausbeute von 28 % der Theorie. Farbloses, viskoses Öl, das ohne weitere Reinigung in der nächsten Stufe eingesetzt wurde.

c.) 3-(Aminomethyl)-3-(brommethyl)-1-propylpyrrolidin-dihydrobromid

Hergestellt analog Beispiel 12c) aus 3-(Aminomethyl)-3-(hydroxymethyl)-1-propylpyrrolidin und 63-proz. wässeriger Bromwasserstoffsäure in einer Ausbeute von 91 % der Theorie. Das bräunlich gefärbte Salz wurde ohne weitere Reinigung in der nächsten Stufe zur Reaktion gebracht.

d.) 6-Propyl-2,6-diazaspiro[3,4]octan

3-(Aminomethyl)-3-(brommethyl)-1-propylpyrrolidin wurde mit Ätznatron, Wasser und Dioxan entsprechend den Angaben im Beispiel 24d) umgesetzt. Das nach Aufarbeitung anfallende Rohprodukt vom $R_F$ 0,5 (Macherey-Nagel, Polygram(R) SIL G/$UV_{254}$, pre-coated plastic sheets for TLC; Fließmittel: Dichlormethan/methanol/Cyclohexan/konz. wässeriges Ammoniak 68/20/10/5, v/v/v/v) wurde säulenchromatographisch an Kieselgel unter Verwendung des vorstehend genannten Eluens gereinigt. Man erhielt in einer Ausbeute von 47 % der Theorie - bezogen auf 3-(Aminomethyl)-3-(hydroxymethyl)-1-propylpyrrolidin, die Titelverbindung, die durch MS und $^1$H-NMR-Spektrum als die gesuchte Verbindung identifiziert wurde.

e.) 5,11-Dihydro-11-[[6-propyl-2,6-diazaspiro[3,4]oct-2-yl]carbonyl]-6H-pyrido[2,3-b][1,4]benzodiazepin-6-on

Hergestellt analog Beispiel 1 aus 11-(Chlorcarbonyl)-5,11-dihydro-6H-pyrido[2,3-b][1,4]benzodiazepin-6-on und 6-Propyl-2,6-diazaspiro[3,4]octan in einer Ausbeute von 74 % der Theorie. Farblose Kristalle vom Fp. 208-209 °C (Acetonitril).

| $C_{22}H_{25}N_5O_2$ (391,48) | | | |
|---|---|---|---|
| Ber.: | C 67,50 | H 6,44 | N 17,89 |
| Gef.: | 67,40 | 6,39 | 17,60 |

Beispiel 29

5,11-Dihydro-8-methyl-11-[[6-propyl-2,6-diazaspiro[3,4]oct-2-yl]carbonyl]-6H-pyrido[2,3-b][1,4]-benzodiazepin-6-on

Hergestellt analog Beispiel 1 aus 11-(Chlorcarbonyl)-5,11-dihydro-8-methyl-6H-pyrido[2,3-b][1,4]-benzodiazepin-6-on und 6-Propyl-2,6-diazaspiro[3,4]octan in einer Ausbeute von 60 % der Theorie. Farblose Kristalle vom Fp. 219-221 °C (Acetonitril).

| $C_{23}H_{27}N_5O_2$ (405,50) | | | |
|---|---|---|---|
| Ber.: | C 68,13 | H 6,71 | N 17,27 |
| Gef.: | 68,02 | 6,74 | 17,00 |

Beispiel 30

8-Chlor-5,11-dihydro-11-[[6-propyl-2,6-diazaspiro[3,4]oct-2-yl]carbonyl]-6H-pyrido[2,3-b][1,4]benzodiazepin-6-on

Hergestellt analog Beispiel 1 aus 8-Chlor-11-(chlorcarbonyl)- 5,11-dihydro-6H-pyrido[2,3-b][1,4]-

benzodiazepin-6-on und 6-Propyl-2,6-diazaspiro[3,4]octan in einer Ausbeute von 44 % der Theorie. Farblose Kristalle vom Fp. 210-212 °C (aus Diisopropylether und Acetonitril).

| $C_{22}H_{24}ClN_5O_2$ (425,93) | | | | |
|---|---|---|---|---|
| Ber.: | C 62,04 | H 5,68 | Cl 8,32 | N 16,44 |
| Gef.: | 61,85 | 5,68 | 8,39 | 16,38 |

Beispiel 31

5,11-Dihydro-8-ethyl-11-[[6-ethyl-2,6-diazaspiro[3,4]oct-2-yl]carbonyl]-6H-pyrido[2,3-b][1,4]benzodiazepin-6-on

Hergestellt analog Beispiel 1 aus 11-(Chlorcarbonyl)-5,11-dihydro-8-ethyl-6H-pyrido[2,3-b][1,4]-benzodiazepin-6-on und 6-Ethyl-2,6-diazaspiro[3,4]octan in einer Ausbeute von 12 % der Theorie. Farblose Kristalle vom Fp. 190-191 °C (Acetonitril) und $R_F$ 0,65 (Macherey-Nagel, Polygram(R) SIL G/$UV_{254}$, pre-coated plastic sheets for TLC; Fließmittel: Dichlormethan/Ethylacetat/Methanol/Cyclohexan/konz. wässeriges Ammoniak 62/16,7/10/10/3, v/v/v/v/v).

| $C_{23}H_{27}N_5O_2$ (405,50) | | | |
|---|---|---|---|
| Ber.: | C 68,13 | H 6,71 | N 17,27 |
| Gef.: | 67,92 | 6,64 | 17,44 |

Beispiel 32

5,11-Dihydro-9-methyl-11-[[6-propyl-2,6-diazaspiro[3,4]oct-2-yl]carbonyl]-6H-pyrido[2,3-b][1,4]-benzodiazepin-6-on

Hergestellt analog Beispiel 1 aus 11-(Chlorcarbonyl)-5,11-dihydro-9-methyl-6H-pyrido[2,3-b][1,4]-benzodiazepin-6-on und 6-Propyl-2,6-diazaspiro[3,4]octan in einer Ausbeute von 39 % der Theorie. Farblose Kristalle vom Fp. 183-184 °C (Acetonitril) und $R_F$ 0,51 (Macherey-Nagel, Polygram(R) SIL G/$UV_{254}$ pre-coated plastic sheets for TLC; Fließmittel: Dichlormethan/Ethylacetat/Methanol/Cyclohexan/konz. wässeriges Ammoniak 63,5/13/11/11/1,5, v/v/v/v/v).

| $C_{23}H_{27}N_5O_2$ (405,50) | | | |
|---|---|---|---|
| Ber.: | C 68,13 | H 6,71 | N 17,27 |
| Gef.: | 68,00 | 6,52 | 17,10 |

Beispiel 33

5,11-Dihydro-8-ethyl-11-[[6-propyl-2,6-diazaspiro[3,4]oct-2-yl]carbonyl]-6H-pyrido[2,3-b][1,4]benzodiazepin-6-on

Hergestellt analog Beispiel 1 aus 11-(Chlorcarbonyl)-5,11-dihydro-8-ethyl-6H-pyrido[2,3-b][1,4]-benzodiazepin-6-on und 6-Propyl-2,6-diazaspiro[3,4]octan in einer Ausbeute von 55 % der Theorie. Farblose Kristalle vom Fp. 170-172 °C (Acetonitril) und $R_F$ 0,52 (Macherey-Nagel, Polygram(R) SIL G/$UV_{254}$ precoated plastic sheets for TLC; Fließmittel: Dichlormethan/Ethylacetat/Methanol/Cyclohexan/konz. wässeriges Ammoniak 63,5/13/11/11/1,5, v/v/v/v/v).

| $C_{24}H_{29}N_5O_2$ (419,53) | | | |
|---|---|---|---|
| Ber.: | C 68,71 | H 6,97 | N 16,69 |
| Gef.: | 68,90 | 7,18 | 16,72 |

Beispiel 34

5,11-Dihydro-11-[[2-ethyl-2,6-diazaspiro[3,4]oct-2-yl]carbonyl]-6H-pyrido[2,3-b][1,4]benzodiazepin-6-on

a.) 2-Acetyl-6-(phenylmethyl)-2,6-diazaspiro[3,4]octan

In die Lösung von 70,8 g (0,35 mol) 6-(Phenylmethyl)-2,6-diazaspiro[3,4]octan in 300 ml Ethanol wurden unter kräftigem Rühren 37,7 ml (0,4 mol) Acetanhydrid eingetropft und die erhaltene Mischung anschließend 3 Stunden unter Rückfluß gekocht. Man dampfte im Vakuum ein, stellte mit 20-proz. wässeriger Natronlauge alkalisch und extrahierte erschöpfend mit Diethylether. Die vereinigten Etherextrakte wurden über Ätzkali getrocknet, vom Lösungsmittel befreit, der verbleibende Rückstand schließlich im Feinvakuum destilliert. Man erhielt die gesuchte Verbindung als farbloses Öl vom $Sdp._{0,035\ mmHg}$ 155-168 °C in einer Ausbeute von 69,3 g (81 % der Theorie).

b.) 2-Ethyl-6-(phenylmethyl)-2,6-diazaspiro[3,4]octan

Hergestellt analog Beispiel 12b) aus 2-Acetyl-6-(phenylmethyl)-2,6-diazaspiro[3,4]octan und Lithiumaluminiumhydrid in wasserfreiem Tetrahydrofuran. Man erhielt die gesuchte Verbindung als farbloses Öl vom $Sdp._{0,2\ mmHg}$ 91-93 °C und $R_F$ 0,6 (Macherey-Nagel, Polygram(R) SIL G/$UV_{254}$, precoated plastic sheets for TLC; Fließmittel: Dichlormethan/methanol/Cyclohexan/konz. wässeriges Ammoniak 68/15/15/2, v/v/v/v) in einer Ausbeute von 73 % der Theorie.

c.) 2-Ethyl-2,6-diazaspiro[3,4]octan

Hergestellt analog Beispiel 17g) aus 2-Ethyl-6-(Phenylmethyl)-2,6-diazaspiro[3,4]octan durch katalytische Hydrierung in Gegenwart von 10-proz. Palladium/Tierkohle. Man erhielt die gesuchte Verbindung als farbloses Öl vom $R_F$ 0,45 (Macherey-Nagel, Polygram(R) SIL G/$UV_{254}$ precoated plastic sheets for TLC; Fließmittel: Dichlormethan/Methanol/Cyclohexan/Ethylacetat/konz. wässeriges Ammoniak 62/10/10/16,7/1,3, v/v/v/v/v) in einer Ausbeute von 96 % der Theorie.

d.) 5,11-Dihydro-11-[[2-ethyl-2,6-diazaspiro[3,4]oct-6-yl]carbonyl]-6H-pyrido[2,3-b][1,4]benzodiazepin-6-on

Hergestellt analog Beispiel 1 aus 11-(Chlorcarbonyl)-5,11-dihydro-6H-pyrido[2,3-b][1,4]benzodiazepin-6-on und 2-Ethyl-2,6-diazaspiro[3,4]octan in einer Ausbeute von 60 % der Theorie. Farblose Kristalle vom Fp. 251-254 °C (Dioxan) und $R_F$ 0,3 (Macherey-Nagel, Polygram(R) SIL G/$UV_{254}$ precoated plastic sheets for TLC; Fließmittel: Dichlormethan/Ethylacetat/Methanol/Cyclohexan/konz. wässeriges Ammoniak 63,5/13/11/11/1,5, v/v/v/v/v).

| $C_{21}H_{23}N_5O_2$ (377,45) | | | |
|---|---|---|---|
| Ber.:<br>Gef.: | C 66,83<br>66,61 | H 6,14<br>6,20 | N 18,55<br>18,40 |

Beispiel 35

5,11-Dihydro-11-[[2-ethyl-2,6-diazaspiro[3,4]oct-6-yl]carbonyl]-8-methyl-6H-pyrido[2,3-b][1,4]benzodiazepin-6-on

Hergestellt analog Beispiel 1 aus 11-(Chlorcarbonyl)-5,11-dihydro-8-methyl-6H-pyrido[2,3-b][1,4]-benzodiazepin-6-on und 2-Ethyl-2,6-diazaspiro[3,4]octan in einer Ausbeute von. 61 % der Theorie. Farblose Kristalle vom Fp. 213-215° C (Acetonitril).

| $C_{22}H_{25}N_5O_2$ (391,48) | | | |
|---|---|---|---|
| Ber.:<br>Gef.: | C 67,50<br>67,19 | H 6,44<br>6,32 | N 17,89<br>18,22 |

Beispiel 36

5,11-Dihydro-11-[[2-ethyl-2,6-diazaspiro[3,4]oct-2-yl]carbonyl]-9-methyl-6H-pyrido[2,3-b][1,4]benzodiazepin-6-on

Hergestellt analog Beispiel 1 aus 11-(Chlorcarbonyl)-5,11-dihydro-9-methyl-6H-pyrido[2,3-b][1,4]-benzodiazepin-6-on und 2-Ethyl-2,6-diazaspiro[3,4]octan in einer Ausbeute von 56 % der Theorie. Farblose Kristalle vom Fp. 251-253° C (Acetonitril).

| $C_{22}H_{25}N_5O_2$ (391,48) | | | |
|---|---|---|---|
| Ber.:<br>Gef.: | C 67,50<br>67,32 | H 6,44<br>6,49 | N 17,89<br>18,31 |

Beispiel 37

9-Chlor-5,11-dihydro-11-[[2-ethyl-2,6-diazaspiro[3,4]oct-2-yl]carbonyl]-6H-pyrido[2,3-b][1,4]benzodiazepin-6-on

Hergestellt analog Beispiel 1 aus 9-Chlor-11-(chlorcarbonyl)-5,11-dihydro-6H-pyrido[2,3-b][1,4]-benzodiazepin-6-on und 2-Ethyl-2,6-diazaspiro[3,4]octan in einer Ausbeute von 45 % der Theorie. Farblose Kristalle vom Fp. 248-250° C (Acetonitril).

| $C_{21}H_{22}ClN_5O_2$ (411,90) | | | | |
|---|---|---|---|---|
| Ber.: | C 61,24 | H 5,38 | Cl 8,61 | N 17,00 |
| Gef.: | 61,31 | 5,22 | 8,90 | 16,98 |

Beispiel 38

5,11-Dihydro-11-[[6-(2-methylpropyl)-2,6-diazaspiro[3,4]oct-2-yl]carbonyl]-6H-pyrido[2,3-b][1,4]-benzodiazepin-6-on

a.) 4-Cyan-4-(ethoxycarbonyl)-1-(2-methylpropyl)-2-pyrrolidinon

Hergestellt analog Beispiel 12a) aus Cyanbernsteinsäure und Hexahydro-1,3,5-tris-(2-methylpropyl)-triazin unter Verwendung von Trifluoressigsäure in einer Ausbeute von 74 % der Theorie. Farbloses Öl fom $R_F$ 0,8 (Macherey-Nagel, Polygram(R) SIL G/$UV_{254}$ pre-coated plastic sheets for TLC; Fließmittel: Ethylacetat/Petrolether 1:1, v/v).

b.) 3-(Aminomethyl)-3-(hydroxymethyl)-1-(2-methylpropyl)pyrrolidin

Hergestellt analog Beispiel 12b) aus 4-Cyan-4-(ethoxycarbonyl)-1-(2-methylpropyl)-2-pyrrolidinon durch Reduktion mit Lithiumaluminiumhydrid in einer Ausbeute von 35 % der Theorie. Farbloses Öl vom $R_F$ 0,26 (Macherey-Nagel, Polygram(R) SIL G/$UV_{254}$, pre-coated plastic sheets for TLC; Fließmittel: Dichlormethan/Methanol/Cyclohexan/konz. wässeriges Ammoniak 68/15/15/2, v/v/v/v).

c.) 3-(Aminomethyl)-3-(brommethyl)-1-(2-methylpropyl)pyrrolidin-dihydrobromid

Hergestellt analog Beispiel 12c) aus 3-(Aminomethyl)-3-hydroxymethyl-1-(2-methylpropyl)pyrrolidin und 63-proz. wässe riger Bromwasserstoffsäure. Das bräunlich gefärbte Salz wurde ohne weitere Reinigung in der folgenden Stufe eingesetzt.

d.) 6-(2-Methylpropyl)-2,6-diazaspiro[3,4]octan

Hergestellt analog Beispiel 28d) aus 3-(Aminomethyl)-3-(brommethyl)-1-(2-methylpropyl)-pyrrolidin-dihydrobromid und Ätznatron in Gegenwart von Wasser und Dioxan in einer Ausbeute von 62 % der Theorie. Farbloses Öl vom $R_F$ 0,45 (Macherey-Nagel, Polygram(R) SIL G/$UV_{254}$ , pre-coated plastic sheets for TLC; Fließmittel: Dichlormethan/Methanol/Cyclohexan/konz. wässeriges Ammoniak 68/20/10/2, v/v/v/v).

e.) 5,11-Dihydro-11-[[6-(2-methylpropyl)-2,6-diazaspiro[3,4]oct 2-yl]carbonyl]-6H-pyrido[2,3-b][1,4]-benzodiazepin-6-on

Hergestellt analog Beispiel 1 aus 11-(Chlorcarbonyl)-5,11-dihydro-6H-pyrido[2,3-b][1,4]benzodiazepin-6-on und 6-(2-Methylpropyl-2,6-diazaspiro[3,4]octan in einer Ausbeute von 49 % der Theorie. Farblose Kristalle vom Fp. 199-201 °C (Acetonitril) und $R_F$ 0,48 (Macherey-Nagel, Polygram(R) SIL G/$UV_{254}$, pre-coated plastic sheets for TLC; Fließmittel: Dichlormethan/Ethylacetat/Methanol/Cyclohexan/konz. wässeriges Ammoniak 63,5/13/11/11/1,5, v/v/v/v/v).

| $C_{23}H_{27}N_5O_2$ (405,50) | | | |
|---|---|---|---|
| Ber.: | C 68,13 | H 6,71 | N 17,27 |
| Gef.: | 67,96 | 6,84 | 17,47 |

Beispiel 39

5,11-Dihydro-8-methyl-11-[[6-(2-methylpropyl)-2,6-diazaspiro[3,4]oct-2-yl]carbonyl]-6H-pyrido[2,3-b][1,4]-benzodiazepin6-on

Hergestellt analog Beispiel 1 aus 11-(Chlorcarbonyl)-5,11-dihydro-8-methyl-6H-pyrido[2,3-b][1,4]-benzodiazepin-6-on und 6-(2-Methylpropyl)-2,6-diazaspiro[3,4]octan in einer Ausbeute von 52 % der Theorie. Farblose Kristalle vom Fp. 186-188 °C (Acetonitril).

| $C_{24}H_{29}N_5O_2$ (419,53) | | | |
|---|---|---|---|
| Ber.: | C 68,71 | H 6,97 | N 16,69 |
| Gef.: | 68,67 | 6,99 | 16,47 |

Beispiel 40

5,11-Dihydro-9-methyl-11-[[6-(2-methylpropyl)-2,6-diazaspiro[3,4]oct-2-yl]carbonyl]-6H-pyrido[2,3-b][1,4]-benzodiazepin-6-on

Hergestellt analog Beispiel 1 aus 11-(Chlorcarbonyl)-5,11-dihydro-9-methyl-6H-pyrido[2,3-b][1,4]-benzodiazepin-6-on und 6-(2-Methylpropyl)-2,6-diazaspiro[3,4]octan in einer Ausbeute von 24 % der Theorie. Farblose Kristalle vom Fp. 163-165 °C (Acetonitril).

| $C_{24}H_{29}N_5O_2$ (419,53) | | | |
|---|---|---|---|
| Ber.: | C 68,71 | H 6,97 | N 16,69 |
| Gef.: | 68,62 | 6,72 | 16,67 |

Beispiel 41

9-Chlor-5,11-dihydro-11-[[6-(2-methylpropyl)-2,6-diazaspiro[3,4]oct-2-yl]carbonyl]-6H-pyrido[2,3-b][1,4]-benzodiazepin6-on

Hergestellt analog Beispiel 1 aus 9-Chlor-11-(chlorcarbonyl)-5,11-dihydro-6H-pyrido[2,3-b][1,4]-benzodiazepin-6-on und 6-(2-Methylpropyl)-2,6-diazaspiro[3,4]octan in einer Ausbeute von 45 % der Theorie. Farblose Kristalle vom Fp. 177-179 °C (Diisopropylether).

| $C_{23}H_{26}ClN_5O_2$ (439,94) | | | | |
|---|---|---|---|---|
| Ber.: | C 62,79 | H 5,96 | Cl 8,06 | N 15,92 |
| Gef.: | 62,98 | 6,29 | 8,07 | 16,08 |

Beispiel 42

6,11-Dihydro-11-[[7-methyl-2,7-diazaspiro[4,4]non-2-yl]carbonyl]-5H-pyrido[2,3-b][1,5]benzodiazepin-5-on

Hergestellt analog Beispiel 1 aus 11-(Chlorcarbonyl)-6,11-dihydro-5H-pyrido[2,3-b][1,5]benzodiazepin-5-on und 2-Methyl-2,7-diazaspiro[4,4]nonan in einer Ausbeute von 84 % der Theorie. Farblose Kristalle vom Fp. 212-214 °C (Acetonitril).

| $C_{21}H_{23}N_5O_2$ (377,45) | | | |
|---|---|---|---|
| Ber.: | C 66,83 | H 6,14 | N 18,55 |
| Gef.: | 66,86 | 6,33 | 18,73 |

Beispiel 43

6,11-Dihydro-11-[[7-ethyl-2,7-diazaspiro[4,4]non-2-yl]carbonyl]-5H-pyrido[2,3-b][1,5]benzodiazepin-5-on

Hergestellt analog Beispiel 1 aus 11-(Chlorcarbonyl)-6,11-dihydro-5H-pyrido[2,3-b][1,5]benzodiazepin-5-on und 2-Ethyl-2,7-diazaspiro[4,4]nonan in einer Ausbeute von 68 % der Theorie. Farblose Kristalle vom Fp. 151,0-152,5 °C (Acetonitril).

| $C_{22}H_{25}N_5O_2$ (391,48) | | | |
|---|---|---|---|
| Ber.: | C 67,50 | H 6,44 | N 17,89 |
| Gef.: | 67,65 | 6,55 | 18,04 |

Beispiel 44

6,11-Dihydro-11-[[6-methyl-2,6-diazaspiro[3,4]oct-2-yl]carbonyl]-5H-pyrido[2,3-b][1,5]benzodiazepin-5-on

Hergestellt analog Beispiel 1 aus 11-(Chlorcarbonyl)-6,11-dihydro-5H-pyrido[2,3-b][1,5]benzodiazepin-5-on und 6-Methyl-2,6-diazaspiro[3,4]octan in einer Ausbeute von 38 % der Theorie. Farblose Kristalle vom Fp. 239-241 °C (Acetonitril).

| $C_{20}H_{21}N_5O_2$ (363,42) | | | |
|---|---|---|---|
| Ber.: | C 66,10 | H 5,82 | N 19,27 |
| Gef.: | 65,99 | 5,77 | 19,17 |

Beispiel 45

6,11-Dihydro-11-[[2-methyl-2,6-diazaspiro[3,4]oct-6-yl]carbonyl]-5H-pyrido[2,3-b][1,5]benzodiazepin-5-on

Hergestellt analog Beispiel 1 aus 11-(Chlorcarbonyl)-6,11-dihydro-5H-Pyrido[2,3-b][1,5]benzodiazepin-5-on und 2-Methyl-2,6-diazaspiro[3,4]octan in einer Ausbeute von 50 % der Theorie. Farblose Kristalle vom Fp. 220-221 ° C (Acetonitril).

| $C_{20}H_{21}N_5O_2$ (363,42) | | | |
|---|---|---|---|
| Ber.: | C 66,10 | H 5,82 | N 19,27 |
| Gef.: | 65,88 | 6,08 | 19,00 |

Beispiel 46

6,11-Dihydro-11-[[6-ethyl-2,6-diazaspiro[3,4]oct-2-yl]carbonyl]-5H-pyrido[2,3-b][1,5]benzodiazepin-5-on

Hergestellt analog Beispiel 1 aus 11-(Chlorcarbonyl)-6,11-dihydro-5H-pyrido[2,3-b][1,5]benzodiazepin-5-on und 6-Ethyl-2,6-diazaspiro[3,4]octan in einer Ausbeute von 10 % der Theorie. Farblose Kristalle vom Fp. 196-200 ° C (Acetonitril) und $R_F$ 0,62 (Macherey-Nagel, Polygram(R) SIL G/$UV_{254}$, pre-coated plastic sheets for TLC; Fließmittel: Dichlormethan/Ethylacetat/Methanol/Cyclohexan/konz. wässeriges Ammoniak 62/16,7/10/10/1,3, v/v/v/v/v).

| $C_{21}H_{23}N_5O_2$ (377,45) | | | |
|---|---|---|---|
| Ber.: | C 66,83 | H 6,14 | N 18,55 |
| Gef.: | 66,26 | 6,11 | 18,14 |

Beispiel 47

6,11-Dihydro-11-[[6-propyl-2,6-diazaspiro[3,4]oct-2-yl]carbonyl]-5H-pyrido[2,3-b][1,5]benzodiazepin-5-on

Hergestellt analog Beispiel 1 aus 11-(Chlorcarbonyl)-6,11-dihydro-5H-pyrido[2,3-b][1,5]benzodiazepin-5-on und 6-Propyl-2,6-diazaspiro[3,4]octan in einer Ausbeute von 51 % der Theorie. Farblose Kristalle vom Fp. 195-197 ° C (Acetonitril) und $R_F$ 0,58 (Bedingungen wie im Beispiel 46).

| $C_{22}H_{25}N_5O_2$ (391,48) | | | |
|---|---|---|---|
| Ber.: | C 67,50 | H 6,44 | N 17,89 |
| Gef.: | 67,46 | 6,36 | 17,98 |

Beispiel 48

6,11-Dihydro-11-[[2-ethyl-2,6-diazaspiro[3,4]oct-6-yl]carbonyl]-5H-pyrido[2,3-b][1,5]benzodiazepin-5-on

Hergestellt analog Beispiel 1, jedoch unter Verwendung von Tetrahydrofuran an Stelle von Acetonitril als Lösungsmittel, aus 11-(Chlorcarbonyl)-6,11-dihydro-5H-pyrido[2,3-b][1,5]benzodiazepin-5-on und 2-Ethyl-2,6-diazaspiro[3,4]octan in einer Ausbeute von 61 % der Theorie. Farblose Kristalle vom Fp. 186-189 °C (Acetonitril) und $R_F$ 0,32 (Macherey-Nagel, Polygram(R) SIL G/$UV_{254}$ , pre-coated plastic sheets for TLC; Fließmittel: Dichlormethan/Ethylacetat/Methanol/Cyclohexan/konz. wässeriges Ammoniak 63,5/13/11/11/1,5, v/v/v/v/v).

| $C_{21}H_{23}N_5O_2$ (377,45) | | | |
|---|---|---|---|
| Ber.: | C 66,83 | H 6,14 | N 18,55 |
| Gef.: | 67,00 | 6,17 | 18,73 |

Beispiel 49

6,11-Dihydro-11-[[6-(2-methylpropyl)-2,6-diazaspiro[3,4]oct-2-yl]carbonyl]-5H-pyrido[2,3-b][1,5]-benzodiazepin-5-on

Hergestellt analog Beispiel 1 aus 11-(Chlorcarbonyl)-6,11-dihydro-5H-pyrido[2,3-b][1,5]benzodiazepin-5-on und 6-(2-Methylpropyl)-2,6-diazaspiro[3,4]octan in einer Ausbeute von 60 % der Theorie. Farblose Kristalle vom Fp. 187-189 °C (Acetonitril).

| $C_{23}H_{27}N_5O_2$ (405,50) | | | |
|---|---|---|---|
| Ber.: | C 68,13 | H 6,71 | N 17,27 |
| Gef.: | 67,95 | 6,77 | 17,47 |

Beispiel 50

5,10-Dihydro-5-[[7-methyl-2,7-diazaspiro[4,4]non-2-yl]carbonyl]-11H-dibenzo[b,e][1,4]diazepin-11-on

Hergestellt analog Beispiel 1 aus 5-(Chlorcarbonyl)-5,10-dihydro-11H-dibenzo[b,e][1,4]diazepin-11-on und 2-Methyl-2,7-diazaspiro[4,4]nonan in einer Ausbeute von 75 % der Theorie. Farblose Kristalle vom Fp. 190 °C (Acetonitril).

| $C_{22}H_{24}N_4O_2$ (376,46) | | | |
|---|---|---|---|
| Ber.: | C 70,19 | H 6,43 | N 14,88 |
| Gef.: | 70,14 | 6,43 | 14,86 |

Beispiel 51

4,9-Dihydro-3-methyl-4-[[7-methyl-2,7-diazaspiro[4,4]non-2-yl]carbonyl]-10H-thieno[3,4-b][1,5]-benzodiazepin-10-on

Hergestellt analog Beispiel 1, jedoch unter Verwendung von Dichlormethan an Stelle von Acetonitril, aus 4-(Chlorcarbonyl)-4,9-dihydro-3-methyl-10H-thieno[3,4-b][1,5]benzodiazepin-10-on und 2-Methyl-2,7-diazaspiro[4,4]nonan in einer Ausbeute von 44 % der Theorie. Farblose Kristalle vom Fp. 213-214 °C (Acetonitril).

| $C_{21}H_{24}N_4O_2S$ (396,51) | | | | |
|---|---|---|---|---|
| Ber.: | C 63,61 | H 6,10 | N 14,13 | S 8,09 |
| Gef.: | 63,30 | 6,19 | 14,21 | 7,94 |

Beispiel 52

4,9-Dihydro-4-[[7-ethyl-2,7-diazaspiro[4,4]non-2-yl]carbonyl]-3-methyl-10H-thieno[3,4-b][1,5]benzodiazepin-10-on

Hergestellt analog Beispiel 51 aus 4-(Chlorcarbonyl)-4,9-dihydro-3-methyl-10H-thieno[3,4-b][1,5]-benzodiazepin-10-on und 2-Ethyl-2,7-diazaspiro[4,4]nonan in einer Ausbeute von 65 % der Theorie. Farblose Kristalle vom Fp. 215-216 °C (Acetonitril).

| $C_{22}H_{26}N_4O_2S$ (410,54) | | | | |
|---|---|---|---|---|
| Ber.: | C 64,36 | H 6,38 | N 13,65 | S 7,81 |
| Gef.: | 64,28 | 6,33 | 13,87 | 7,91 |

Beispiel 53

4,9-Dihydro-3-methyl-4-[[6-methyl-2,6-diazaspiro[3,4]oct-2-yl]carbonyl]-10H-thieno[3,4-b][1,5]benzodiazepin-10-on

Hergestellt analog Beispiel 1 aus 4-(Chlorcarbonyl)-4,9-dihydro-3-methyl-10H-thieno[3,4-b][1,5]-benzodiazepin-10-on und 6-Methyl-2,6-diazaspiro[3,4]octan in einer Ausbeute von 52 % der Theorie. Farblose Kristalle vom Fp. 200-203 °C (Acetonitril).

| $C_{20}H_{22}N_4O_2S$ (382,49) | | | | |
|---|---|---|---|---|
| Ber.: | C 62,80 | H 5,80 | N 14,65 | S 8,38 |
| Gef.: | 62,51 | 5,68 | 14,67 | 8,40 |

Beispiel 54

4,9-Dihydro-3-methyl-4-[[2-methyl-2 6-diazaspiro[3,4]oct-6-yl]carbonyl]-10H-thieno[3,4-b][1,5]benzodiazepin-10-on

Hergestellt analog Beispiel 1 aus 4-(Chlorcarbonyl)-4,9-dihydro-3-methyl-10H-thieno[3,4-b][1,5]-benzodiazepin-10-on und 2-Methyl-2,6-diazaspiro[3,4]octan in einer Ausbeute von 28 % der Theorie.

Farblose Kristalle vom Fp. 135-137 °C (Acetonitril).

| $C_{20}H_{22}N_4O_2S$ (382,49) | | | | |
|---|---|---|---|---|
| Ber.: | C 62,80 | H 5,80 | N 14,65 | S 8,38 |
| Gef.: | 62,62 | 5,81 | 14,70 | 8,54 |

Beispiel 55

4,9-Dihydro-4-[[6-ethyl-2,6-diazaspiro[3,4]oct-2-yl]carbonyl]-3-methyl-10H-thieno[3,4-b][1,5]benzodiazepin-10-on

Hergestellt analog Beispiel 1 aus 4-(Chlorcarbonyl)-4,9-dihydro-3-methyl-10H-thieno[3,4-b][1,5]-benzodiazepin-10-on und 6-Ethyl-2,6-diazaspiro[3,4]octan in einer Ausbeute von 30 % der Theorie. Farblose Kristalle vom Fp. 208-210 °C (Acetonitril).

| $C_{21}H_{24}N_4O_2S$ (396,51) | | | | |
|---|---|---|---|---|
| Ber.: | C 63,61 | H 6,10 | N 14,13 | S 8,09 |
| Gef.: | 63,35 | 6,25 | 14,32 | 8,19 |

Beispiel 56

4,9-Dihydro-3-methyl-4-[[6-propyl-2,6-diazaspiro[3,4]oct-2-yl]carbonyl]-10H-thieno[3,4-b][1,5]benzodiazepin-10-on

Hergestellt analog Beispiel 1 aus 4-(Chlorcarbonyl)-4,9-dihydro-3-methyl-10H-thieno[3,4-b][1,5]-benzodiazepin-10-on und 6-Propyl-2,6-diazaspiro[3,4]octan in einer Ausbeute von 44 % der Theorie. Farblose Kristalle vom Fp. 180-181 °C (Acetonitril) und $R_F$ 0,54 (Macherey-Nagel, Polygram.(R) SIL G/$UV_{254}$ pre-coated plastic sheets for TLC; Fließmittel: Dichlormethan/Ethylacetat/Methanol/Cyclohexan/konz. wässeriges Ammoniak. 63,5/13/11/11/1,5, v/v/v/v/v).

| $C_{22}H_{26}N_4O_2S$ (410,54) | | | | |
|---|---|---|---|---|
| Ber.: | C 64,36 | H 6,38 | N 13,65 | S 7,81 |
| Gef.: | 64,26 | 6,35 | 13,64 | 7,71 |

Beispiel 57

4,9-Dihydro-4-[[2-ethyl-2,6-diazaspiro[3,4]oct-6-yl]carbonyl]-3-methyl-10H-thieno[3,4-b][1,5]benzodiazepin-10-on

Hergestellt analog Beispiel 1 aus 4-(Chlorcarbonyl)-4,9-dihydro-3-methyl-10H-thieno[3,4-b][1,5]-benzodiazepin-10-on und 2-Ethyl-2,6-diazaspiro[3,4]octan in einer Ausbeute von 61 % der Theorie. Farblose Kristalle vom Fp. 216-217 °C (Acetonitril) und $R_F$ 0,47 (Bedingungen wie im Beispiel 56).

| $C_{21}H_{24}N_4O_2S$ (396,51) | | | | |
|---|---|---|---|---|
| Ber.:<br>Gef.: | C 63,61<br>63,51 | H 6,10<br>6,10 | N 14,13<br>14,27 | S 8,09<br>8,08 |

Beispiel 58

4,9-Dihydro-3-methyl-4-[[6-(2-methylpropyl)-2,6-diazaspiro[3,4]oct-2-yl]carbonyl]-10H-thieno[3,4-b][1,5]-benzodiazepin-10-on

Hergestellt analog Beispiel 1 aus 4-(Chlorcarbonyl)-4,9-dihydro-3-methyl-10H-thieno[3,4-b][1,5]-benzodiazepin-10-on und 6-(2-Methylpropyl)-2,6-diazaspiro[3,4]octan in einer Ausbeute von 71 % der Theorie. Farblose Kristalle vom Fp. 186-188 °C (Acetonitril).

| $C_{23}H_{28}N_4O_2S$ (424,56) | | | | |
|---|---|---|---|---|
| Ber.:<br>Gef.: | C 65,07<br>65,07 | H 6,65<br>6,61 | N 13,20<br>13,30 | S 7,55<br>7,42 |

Beispiel 59

3-Chlor-1-methyl-4-[[7-methyl-2,7-diazaspiro[4,4]non-2-yl]carbonyl]-1,4,9,10-tetrahydropyrrolo[3,2-b][1,5]-benzodiazepin-10-on

Hergestellt analog Beispiel 1 aus 3-Chlor-4-(chlorcarbonyl)-1-methyl-1,4,9,10-tetrahydropyrrolo[3,2-b]-[1,5]benzodiazepin-10-on und 2-Methyl-2,7-diazaspiro[4,4]nonan in einer Ausbeute von 80 % der Theorie. Farblose Kristalle vom Fp. 205-206 °C (Acetonitril).

| $C_{21}H_{24}ClN_5O_2$ (413,92) | | | | |
|---|---|---|---|---|
| Ber.:<br>Gef.: | C 60,94<br>61,13 | H 5,84<br>5,66 | Cl 8,57<br>8,78 | N 16,92<br>17,20 |

Beispiel 60

1-Methyl-4-[[7-methyl-2,7-diazaspiro[4,4]non-2-yl]carbonyl]-1,4,9,10-tetrahydropyrrolo[3,2-b][1,5]-benzodiazepin-10-on

3,5 g (8,46 mMol) 3-Chlor-1-methyl-4-[[7-methyl-2,7-diazaspiro[4,4]non-2-yl]carbonyl]-1,4,9,10-tetrahydropyrrolo[3,2-b][1,5]benzodiazepin-10-on wurden in 350 ml heißem Ethanol gelöst und nach Zusatz von 3 g Palladium auf Tierkohle (20-proz.) 2 Stunden bei einem Wasserstoffdruck von 50 bar und einer Temperatur von 40 °C hydriert. Man filtrierte vom Katalysator ab, engte das Filtrat im Vakuum ein, nahm das kristalline Hydrochlorid in 20 ml Wasser auf, stellte die erhaltene Lösung natronalkalisch und extrahierte erschöpfend mit Dichlormethan. Die vereinigten Extrakte wurden über Natriumsulfat getrocknet und eingedampft und der verbleibende Rückstand aus n-Propanol umkristallisiert. Man er hielt 1,1 g (34 % der

Theorie) an farblosen Kristallen vom Fp. 223-225° C.

| $C_{21}H_{25}N_5O_2$ (379,47) | | | |
|---|---|---|---|
| Ber.: | C 66,47 | H 6,64 | N 18,46 |
| Gef.: | 66,43 | 6,84 | 18,44 |

Beispiel 61

3-Chlor-1-methyl-4-[[6-propyl-2,6-diazaspiro[3,4]oct-2-yl]-carbonyl]-1,4,9,10-tetrahydropyrrolo[3,2-b][1,5]-benzodiazepin-10-on

Hergestellt analog Beispiel 1 aus 3-Chlor-4-(chlorcarbonyl)-1-methyl-1,4,9,10-tetrahydropyrrolo[3,2-b]-[1,5]benzodiazepin-10-on und 6-Propyl-2,6-diazaspiro[3,4]octan in einer Ausbeute von 51 % der Theorie. Farblose Kristalle vom Fp. 164-165° C (Acetonitril).

| $C_{22}H_{26}ClN_5O_2$ (427,94) | | | | |
|---|---|---|---|---|
| Ber.: | C 61,75 | H 6,12 | Cl 8,28 | N 16,37 |
| Gef.: | 61,48 | 5,99 | 8,42 | 16,37 |

Beispiel 62

3-Chlor-1-methyl-4-[[6-(2-methylpropyl)-2,6-diazaspiro[3,4]oct-2-yl]carbonyl]-1,4,9,10-tetrahydropyrrolo[3,2-b][1,5]benzodiazepin-10-on

Hergestellt analog Beispiel 1 aus 3-Chlor-4-(chlorcarbonyl)-1-methyl-1,4,9,10-tetrahydropyrrolo[3,2-b]-[1,5]benzodiazepin-10-on und 6-(2-Methylpropyl)-2,6-diazaspiro[3,4]octan in einer Ausbeute von 23 % der Theorie. Farblose Kristalle vom Fp. 163-165° C (Acetonitril).

| $C_{23}H_{28}ClN_5O_2$ (441,96) | | | | . |
|---|---|---|---|---|
| Ber.: | C 62,51 | H 6,39 | Cl 8,02 | N 15,85 |
| Gef.: | 62,47 | 6,39 | 8,18 | 15,89 |

Beispiel 63

3-Chlor-1-methyl-4-[[6-methyl-2,6-diazaspiro[3,4]oct-2-yl]carbonyl]-1,4,9,10-tetrahydropyrrolo[3,2-b][1,5]-benzodiazepin-10-on

Hergestellt analog Beispiel 1 aus 3-Chlor-4-(chlorcarbonyl)-1-methyl-1,4,9,10-tetrahydropyrrolo[3,2-b]-[1,5]benzodiazepin-10-on und 6-Methyl-2,6-diazaspiro[3,4]octan in einer Ausbeute von 61 % der Theorie. Farblose Kristalle vom Fp. 215-217° C (Acetonitril) und $R_F$ 0,7 (Macherey-Nagel, Polygram(R) SIL G/$UV_{254}$ pre-coated plastic sheets for TLC; Fließmittel: Dichlormethan/methanol/Cyclohexan/konz. wässeriges Ammoniak 68/15/15/2, v/v/v/v).

| $C_{20}H_{22}ClN_5O_2$ (399,88) | | | | |
|---|---|---|---|---|
| Ber.: | C 60,07 | H 5,55 | Cl 8,87 | N 17,51 |
| Gef.: | 60,13 | 5,39 | 9,02 | 17,64 |

Beispiel 64

5,11-Dihydro-11-[[6-methyl-2,6-diazaspiro[3,4]oct-2-yl]carbonyl]-6H-pyrido[2,3-b][1,4]benzodiazepin-6-on

In eine Mischung, bestehend aus 22,5 ml einer 20-proz. Lösung von Phosgen in Toluol, 100 ml Acetonitril und 4,75 g (0,045 Mol) wasserfreiem Natriumcarbonat wurden unter äußerer Kühlung mit Eis 5,36 g (0,0425 mol) 6-Methyl-2,6-diazaspiro[3,4]octan zugetropft. Man rührte noch 60 Minuten bei Zimmertemperatur, trug dann 9,0 g (0,0428 Mol) 5,11-Dihydro-6H-pyrido[2,3-b][1,4]benzodiazepin-6-on in die Reaktionsmischung ein und kochte anschließend 4 Stunden unter Rückfluß. Man filtrierte die siedend heiße Mischung, wusch den Niederschlag mit dreimal je 10 ml heißem Acetonitril gründlich durch und engte die vereinigten Filtrate im Vakuum auf ein Gesamtvolumen von 50 ml ein. Man ließ erkalten und hielt unter gelegentlichem Umrühren mit einem Glasstab 2 Stunden bei 0 bis 5 °C, nutschte den entstandenen Kristallbrei ab, kristallisierte nochmals aus Acetonitril um und erhielt farblose Kristalle vom Fp. 225,5-227,0 °C, nach Mischschmelzpunkt, IR- und $^1$H-NMR-Spektrum identisch mit einem nach Beispiel 12 hergestellten Präparat.
Ausbeute: 5,4 g (35 % der Theorie).

Entsprechend erhielt man:
(±)-5,11-Dihydro-11-[[7-methyl-2,7-diazaspiro[4,4]non-2-yl]carbonyl]-6H-pyrido[2,3-b][1,4]benzodiazepin-6-on vom Fp. 272-274 °C (Acetonitril);
5,11-Dihydro-8-methyl-11-[[7-methyl-2,7-diazaspiro[4,4]non-2-yl]carbonyl]-6H-pyrido[2,3-b][1,4]-benzodiazepin-6-on vom Fp. 212-214 °C (Acetonitril);
5,11-Dihydro-8-ethyl-11-[[6-methyl-2,6-diazaspiro[3,4]oct-2-yl]carbonyl]-6H-pyrido[2,3-b][1,4]benzodiazepin-6-on vom Fp. 215-217 °C (Acetonitril);
5,11-Dihydro-8-methyl-11-[[2-methyl-2,6-diazaspiro[3,4]oct-6-yl]carbonyl]-6H-pyrido[2,3-b][1,4]-benzodiazepin-6-on vom Fp. 184,0-184,5 °C (Acetonitril).

Beispiel 65

5,11-Dihydro-11-[[6-methyl-2,6-diazaspiro[3,4]oct-2-yl]carbonyl]-6H-pyrido[2,3-b][1,4]benzodiazepin-6-on

Zu der Suspension von 2,6 g (9,50 mMol) II-(Chlorcarbonyl)-5,11-dihydro-6H-pyrido[2,3-b][1,4]-benzodiazepin-6-on in 30 ml Dimethylformamid tropfte man bei Zimmertemperatur und unter Rühren die Lösung von 1,2 g (9,51 mMol)) 6-Methyl-2,6-diazaspiro[3,4]octan in 10 ml Dimethylformamid. Die anfangs klare Lösung trübte sich innerhalb weniger Minuten erneut ein. Nach halbstündigem Rühren bei Raumtemperatur wurde der farblose Feststoff abgenutscht und mit dreimal je 3 ml eiskaltem Ethanol gründlich ausgewaschen. Das erhaltene farblose Monohydrochlorid der gesuchten Verbindung wurde in 10 ml Wasser gelöst, mit einer gesättigten wäßrigen Kaliumcarbonatlösung bis zur deutlich alkalischen Reaktion versetzt und anschließend filtriert. Der erhaltene Feststoff wurde mit Wasser gründlich gewaschen, anschließend im Vakuumtrockenschrank bei 50 °C und über di-Phosphorpentoxid getrocknet; aus heißem Acetonitril umkristallisiert und erneut im Vakuum getrocknet. Man erhielt 2,35 g (68 % der Theorie) an farblosen Kristallen vom Fp. 225,5-227,0 °C, nach Mischschmelzpunkt, IR- und $^1$H-NMR-Spektrum identisch mit einem nach Beispiel 12 hergestellten Präparat.

Beispiel 66

1-Methyl-4-[[7-methyl-2,7-diazaspiro[4,4]non-2-yl]carbonyl]-1,4,9,10-tetrahydropyrrolo[3,2-b][1,5]-benzodiazepin-10-on

4,02 g (9,71 mMol) 3-Chlor-1-methyl-4-[[7-methyl-2,7-diazaspiro[4,4]non-2-yl]carbonyl]-1,4,9,10-tetrahydropyrrolo [3,2-b][1,5]benzodiazepin-10-on wurden in einem Gemisch aus 5 ml 85-Proz. Ameisensäure und 25 ml Dimethylformamid gelöst und nach Zusatz von 0,5 g 10-proz. Palladium/Aktivkohle 3 Stunden unter Rückfluß gekocht. Man gab 7,0 ml Ameisensäure zu, kochte weitere 6 Stunden unter Rückfluß und erhitzte nach Versetzen mit weiteren 4,0 ml Ameisensäure und 0,8 g 10-proz. Palladium/Aktivkohle abschließend nochmals 8 Stunden unter Rückfluß. Die Mischung wurde heiß filtriert, das Filtrat im Vakuum eingedampft und der Rückstand säulenchromatographisch (Kieselgel; Dichlormethan/Essigsäureethylester/methanol/konz. Ammoniak 3,5:1,5:0,46:0,06, v/v/v/v) gereinigt. Man erhielt 1,14 g (31 % der Theorie) an farblosen Kristallen vom Fp. 223-225 °C (n-Propanol), nach Dünnschichtchromatogramm, IR-, UV- und $^1$H-NMR-Spektren identisch mit einem nach Beispiel 60 erhaltenen Präparat.

Beispiel 67

1-Methyl-4-[[7-methyl-2,7-diazaspiro[4,4]non-2-yl]carbonyl]-1,4,9,10-tetrahydropyrrolo[3,2-b][1,5]-benzodiazepin-10-on

Die Mischung aus 4,14 g (0,01 Mol) 3-Chlor-4-[[7-methyl-2,7-diazaspiro[4,4]non-2-yl]carbonyl]-1,4,9,10-tetrahydropyrrolo[3,2-b][1,5]benzodiazepin-10-on, 83,3 mg (0,001 Mol) 2:1-Tris(o-tolyl)phosphin-Palladiumacetat-Katalysator, 2,025 g (0,044 Mol) Ameisensäure und 5,77 g (0,057 Mol) Triethylamin in 200 ml Tetrahydrofuran wurde unter Stickstoff-Atmosphäre im Autoklaven 40 Stunden auf 100 °C erhitzt. Die Mischung wurde filtriert und im Vakuum eingedampft, der Rückstand natronalkalisch gestellt und erschöpfend mit Dichlormethan extrahiert. Die getrockneten und eingedampften organischen Phasen wurden wie in Beispiel 66 säulenchromatographisch gereinigt. Man erhielt 1,44 g (38 % der Theorie) an farblosen Kristallen vom Fp. 223-225 °C (n-Propanol), nach Dünnschichtchromatogramm, Mischschmelzpunkt und IR-Spektrum identisch mit einem nach Beispiel 60 hergestellten Präparat.

Beispiel 68

5,11-Dihydro-8,9-dimethyl-11-[[6-methyl-2,6-diazaspiro[3,4]oct-2-yl]carbonyl]-6H-pyrido[2,3-b][1,4]-benzodiazepin-6-on

Hergestellt analog Beispiel 1 aus 11-(Chlorcarbonyl)-5,11-dihydro-8,9-dimethyl-6H-pyrido[2,3-b][1,4]-benzodiazepin-6-on und 6-Methyl-2,6-diazaspiro[3,4]octan in einer Ausbeute von 59 % der Theorie. Farblose Kristalle vom Fp. 247-249 °C.

| $C_{22}H_{25}N_5O_2$ (391,48). | | | |
|---|---|---|---|
| Ber.: | C 67,50 | H 6,44 | N 17,89 |
| Gef.: | 67,25 | 6,52 | 18,10 |

Beispiel 69

5,11-Dihydro-8,9-dimethyl-11-[[2-ethyl-2,6-diazaspiro[3,4]oct-6-yl]carbonyl]-6H-pyrido[2,3-b][1,4]-benzodiazepin-6-on-hydrochlorid

3,01 g (0,00998 mol) 11-(Chlorcarbonyl)-5,11-dihydro-8,9-dimethyl-6H-pyrido[2,3-b][1,4]benzodiazepin-6-on, 1,4 g (0,00998 mol) 2-Ethyl-2,6-diazaspiro[3,4]octan und 100 ml wasserfreies Acetonitril wurden unter

Rühren 2 Stunden lang auf 60° C erhitzt. Der nach dem Erkalten ausfallende farblose, kristalline Niederschlag wurde abgenutscht und aus trockenem siedenden Ethanol umkristallisiert. Man erhielt 2,3 g (52 % der Theorie) an farblosen Kristallen vom Fp. 268-270° C.

| $C_{23}H_{27}N_5O_2xHCl$ (441,97). | | | | |
|---|---|---|---|---|
| Ber.: | C 62,51 | H 6,39 | Cl 8,02 | N 15,85 |
| Gef.: | 62,38 | 6,34 | 8,16 | 15,61 |

Beispiel 70

5,11-Dihydro-8,9-dimethyl-11-[[2-methyl-2,6-diazaspiro[3,4]oct-6-yl]carbonyl]-6H-pyrido[2,3-b][1,4]-benzodiazepin-6-on

Hergestellt analog Beispiel 1 aus 11-(Chlorcarbonyl)-5,11-dihydro-8,9-dimethyl-6H-pyrido[2,3-b][1,4]-benzodiazepin-6-on und 2-Methyl-2,6-diazaspiro[3,4]octan in einer Ausbeute von 30 % der Theorie. Farblose Kristalle vom Fp. 292-294° C.

| $C_{22}H_{25}N_5O_2$ (391,48). | | | |
|---|---|---|---|
| Ber.: | C 67,50 | H 6,44 | N 17,89 |
| Gef.: | 66,91 | 6,39 | 17,71 |

Beispiel 71

5,11-Dihydro-11-[[6-methyl-2,6-diazaspiro[3,3]hept-2-yl]-carbonyl]-6H-pyrido[2,3-b][1,4]benzodiazepin-6-on

a) 2,2-Bis(brommethyl)-1,3-propandiamin-dihydrobromid

40,65 g (0,1 mol) 2,6-Bis-[(4-methylphenyl)sulfonyl]-2,6-diazaspiro[3,3]heptan und 300 ml konzentrierte wässerige Bromwasserstoffsäure wurden in einem Glasautoklaven unter Schütteln 12 Stunden lang auf 180° C erhitzt. Die erkaltete Reaktionsmischung wurde mit Wasser auf das dreifache Volumen verdünnt und anschließend filtriert. Das Filtrat wurde im Wasserstrahlvakuum eingedampft, der verbleibende hellbraune Rückstand mit 2x je 100 ml absolutem Ethanol sorgfältig ver rieben und abgenutscht, das erhaltene Kristallisat schließlich aus einer siedenden Mischung von 3 Teilen Ethanol und einem Teil Wasser umkristallisiert. Farblose Kristalle vom Fp. 283-285° C. Ausbeute: 35,7 g (85 % der Theorie).

| $C_5H_{12}Br_2N_2x2HBr$ (421,80). | | | | |
|---|---|---|---|---|
| Ber.: | C 14,24 | H 3,35 | Br 75,78 | N 6,64 |
| Gef.: | 14,39 | 3,36 | 75,32 | 6,36 |

b) 5,11-Dihydro-11-[[6-methyl-2,6-diazaspiro[3,3]hept-2-yl]carbonyl]-6H-pyrido[2,3-b][1,4]benzodiazepin-6-on

Die Suspension von 16,87 g (0,04 mol) 2,2-Bis-(brommethyl)-1,3-propandiamin-dihydrobromid in 500 ml

Acetonitril wurde unter Rühren und bei Zimmertemperatur tropfenweise mit der Lösung aus 6,4 g (0,16 mol) Natriumhydroxid in 25 ml Wasser versetzt. Nach 4-stündigem Rühren bei Raumtemperatur wurde mit wasserfreiem Natriumcarbonat getrocknet und filtriert. Zum Filtrat gab man 9,0 g (0,033 mol) 11-(Chlorcarbonyl)-5,11-dihydro-6H-pyrido[2,3-b][1,4]benzodiazepin-6-on und 4,3 g (0,041 mol) wasserfreies Natriumcarbonat zu und rührte 30 Minuten lang bei einer Reaktionstemperatur von 50 °C. Das Lösungsmittel wurde anschließend im Vakuum abdestilliert, der verbleibende hochviskose Rückstand in 300 ml Ethanol gelöst, mit 4 ml (ca. 0,05 mol) einer 37-proz. wässerigen Formalin-Lösung versetzt und 50 Minuten unter Rückfluß gekocht. Man ließ erkalten, fügte 5,0 g Raney-Nickel zu und hydrierte die Mischung 5 Stunden bei Raumtemperatur und 4 bar Wasserstoffdruck. Der Katalysator wurde abfiltriert, das Filtrat im Vakuum eingedampft, der verbleibende Rückstand an Kieselgel (35-70 mesh) unter Verwendung von Dichlormethan/Essigsäureethylester/Cyclohexan/Methanol/konz. Ammoniak 50/11/9/9/1 v/v/v/v/v zum Eluieren chromatographisch gereinigt. Der nach dem Eindampfen der geeigneten Eluate verbleibende Rückstand wurde aus heißem Acetonitril umkristallisiert. Man erhielt 3,3 g (29 % der Theorie) an farblosen Kristallen vom Fp. 226-228 °C.

| $C_{19}H_{19}N_5O_2$ (349,39). | | | |
|---|---|---|---|
| Ber.: | C 65,32 | H 5,48 | N 20,04 |
| Gef.: | 65,08 | 5,29 | 20,31 |

Entsprechend wurde erhalten:

5,11-Dihydro-8,9-dimethyl-11-[[6-methyl-2,6-diazaspiro[3,3]hept-2-yl]carbonyl]-6H-pyrido[2,3-b][1,4]-benzodiazepin-6-on vom Fp. 296-298 °C.

| $C_{21}H_{23}N_5O_2$ (377,45). | | | |
|---|---|---|---|
| Ber.: | C 66,83 | H 6,14 | N 18,55 |
| Gef.: | 67,05 | 6,25 | 18,75 |

## Beispiel 72

### 5,11-Dihydro-11-[[7-methyl-2,7-diazaspiro[3,5]non-2-yl]carbonyl]-6H-pyrido[2,3-b][1,4]benzodiazepin-6-on

#### a) 4-(Aminomethyl)-1-methyl-4-piperidincarbonsäureethylester

52,0 g (0,265 mol) 4-Cyan-1-methyl-4-piperidincarbonsäureethylester wurden in 400 ml Eisessig gelöst und nach Zugabe von 10 ml konzentrierter Schwefelsäure in Gegenwart von 3 g Platin(IV)-oxid bei einem anfänglichen Wasserstoffdruck von 40 bar bis zur Beendigung der Wasserstoffaufnahme bei Raumtemperatur hydriert. Der Katalysator wurde abgetrennt, das Filtrat im Vakuum vom Lösungsmittel befreit, der Rückstand mit 100 g Eis versetzt und unter Rühren und guter äußerer Kühlung mit Eis-Kochsalz-Gemisch tropfenweise mit konzentrierter Natronlauge versetzt, bis die Mischung einen pH von 8 erreichte. Unter weiterer äußerer Kühlung wurden dann portionsweise 250 g wasserfreies Kaliumcarbonat eingetragen, der dabei entstandene steife Brei mit Ether erschöpfend extrahiert. Die vereinigten Etherlösungen wurden über Kaliumcarbonat getrocknet und eingedampft, der verbleibende Rückstand ohne weitere Reinigung in der folgenden Stufe umgesetzt. Ausbeute: 40,0 g (75 % der Theorie) eines farblosen Öls.

#### b) 7-Methyl-2,7-diazaspiro[3,5]nonan

In eine auf 0 bis 5 °C gekühlte Grignard-Lösung aus 13,6 g (0,559 g-Atom) Magnesium, 61,1 g (0,561 mol) Bromethan und 350 ml Diethylether tropfte man langsam unter Rühren 37,5 g (0,187 mol) 4-(Aminomethyl)-1-methyl-4-piperidincarbonsäureethylester in 100 ml wasserfreiem Diethylether. Man rührte noch 2 Stunden bei 0 °C und 4 Stunden bei Raumtemperatur. Die erneut auf 0 bis 5 °C gekühlte Mischung wurde dann sehr langsam und unter Rühren mit 50 ml 10-proz. wässeriger Ammoniumchlorid-Lösung versetzt und mit 50 ml konz. Ammoniak deutlich ammoniakalisch gestellt. Die Ether-Schicht wurde abgetrennt, die wässerige Phase erschöpfend ausgeethert, die vereinigten Etherauszüge zweimal mit je 50 ml Wasser gewaschen, über Natriumsulfat getrocknet und im Vakuum eingedampft. Der ölige Rückstand wurde in 80 ml wasserfreiem Diethylether aufgenommen, die so erhaltene Lösung zur Suspension von 5,9 g (0,16 mol) Lithiumaluminiumhydrid in 200 ml trockenem Diethylether derart zugetropft, daß der Ether nur mäßig siedete. Nach Beendigung der Zugabe rührte man weitere 4 Stunden bei Raumtemperatur und kochte eine Stunde unter Rückfluß. Man ließ erkalten und tropfte unter Rühren und äußerer Kühlung mit Eiswasser nacheinander 6 ml Wasser, 6 ml 15-proz. Natronlauge und 18 ml Wasser ein. Der erhaltene Niederschlag wurde abgenutscht, nochmals mit Diethylether aufgeschlämmt und aufgekocht, danach abermals abgenutscht.

Die vereinigten Filtrate wurden über Natriumsulfat getrocknet und eingedampft. Das in einer Ausbeute von 19,0 g (72 % der Theorie) anfallende farblose Öl wurde ohne weitere Reinigung in der nächsten Stufe weiterverarbeitet.

c) 5,11-Dihydro-11-[[7-methyl-2,7-diazaspiro[3,5]non-2-yl]carbonyl]-6H-pyrido[2,3-b][1,4]benzodiazepin-6-on

Hergestellt analog Beispiel 1 aus 11-(Chlorcarbonyl)-5,11-dihydro-6H-pyrido[2,3-b][1,4]benzodiazepin-6-on und 7-Methyl. 2,7-diazaspiro[3,5]nonan in einer Ausbeute von 61 % der Theorie. Farblose Kristalle vom Fp. 201-203 °C (Acetonitril).

| $C_{21}H_{23}N_5O_2$ (377,45). | | | |
|---|---|---|---|
| Ber.: | C 66,83 | H 6,14 | N 18,55 |
| Gef.: | 66,95 | 6,27 | 18,80 |

Beispiel 73

5,11-Dihydro-8,9-dimethyl-11-[[7-methyl-2,7-diazaspiro[3,5]non-2-yl]carbonyl]-6H-pyrido[2,3-b][1,4]-benzodiazepin-6-on

Hergestellt analog Beispiel 1 aus 11-(Chlorcarbonyl)-5,11-dihydro-8,9-dimethyl-6H-pyrido[2,3-b][1,4]-benzodiazepin-6-on und 7-Methyl-2,7-diazaspiro[3,5]nonan in einer Ausbeute von 46 % der Theorie. Farblose Kristalle vom Fp. 222-224 °C (Acetonitril).

| $C_{23}H_{27}N_5O_2$ (405,50). | | | |
|---|---|---|---|
| Ber.: | C 68,13 | H 6,71 | N 17,27 |
| Gef.: | 67,95 | 6,80 | 17,49 |

Beispiel 74

5,11-Dihydro-11-[[2-methyl-2,7-diazaspiro[3,5]non-7-yl]carbonyl]-6H-pyrido[2,3-b][1,4]benzodiazepin-6-on

a) 2-Methyl-7-(phenylmethyl)-2,7-diazaspiro[3,5]nonan

11,55 g (0,0534 mol) 7-(Phenylmethyl)-2,7-diazaspiro[3,5]nonan, erhalten analog Beispiel 72a) und 72b), wurden in 300 ml Ethanol gelöst, mit 5 ml (ca. 0,062 mol) einer 37-proz. wässerigen Formalin-Lösung versetzt und 50 Minuten unter Rückfluß gekocht. Man ließ erkalten, gab 5,0 g Raney-Nickel zu und hydrierte die Mischung 5 Stunden bei Raumtemperatur und 4 bar Wasserstoffdruck. Der Katalysator wurde abfiltriert, das Filtrat im Vakuum eingedampft, der verbleibende Rückstand an Kieselgel mittels HPLC und unter Verwendung von Dichlormethan/Methanol/Cyclohexan/konz. wässerigem Ammoniak 68/15/15/2 zum Eluieren gereinigt. Eindampfen der geeigneten Fraktionen ergab die gesuchte Verbindung in Form eines zähflüssigen, farblosen Öls. Ausbeute: 6,7 g (54 % der Theorie). $R_F$ 0,58 (Macherey-Nagel, Polygram(R) SIL G/$UV_{254}$ precoated plastic sheets for TLC; Fließmittel: Dichlormethan/Methanol/Cyclohexan/konz. wässriges Ammoniak 68/15/15/2 v/v/v/v/v).

b) 2-Methyl-2,7-diazaspiro[3,5]nonan

Zu der Lösung von 6,5 g (0,0282 mol) 2-Methyl-6-(phenylmethyl)-2,6-diazaspiro[3,4]octan in 60 ml Ethanol gab man 4,0 g 10-proz. Palladium/Tierkohle-Katalysator und hydrierte anschließend 5 Stunden bei Raumtemperatur und einem Wasserstoffdruck von 5 bar. Man filtrierte, dampfte das Filtrat bei vermindertem Druck (100 mmHg) ein und erhielt als Rück stand ein farbloses Öl vom $R_F$ 0,13 (Macherey-Nagel, Polygram(R) SIL G/$UV_{254}$, pre-coated plastic sheets for TLC; Fließmittel: Dichlormethan/Methanol/Cyclohexan/konz. wässeriges Ammoniak 68/20/10/5, v/v/v/v).
Ausbeute: 3,2 g (81 % der Theorie).

c) 5,11-Dihydro-11-[[2-methyl-2,7-diazaspiro[3,5]non-7-yl]carbonyl]-6H-pyrido[2,3-b][1,4]benzodiazepin-6-on

Hergestellt analog Beispiel 1 aus 11-(Chlorcarbonyl)-5,11-dihydro-6H-pyrido[2,3-b][1,4]benzodiazepin-6-on und 2-Methyl-2,7-diazaspiro[3,5]nonan in einer Ausbeute von 59 % der Theorie. Farblose Kristalle vom Fp. 186-188 °C (Diisopropylether).

| $C_{21}H_{23}N_5O_2$ (377,45). | | | |
|---|---|---|---|
| Ber.: | C 66,83 | H 6,14 | N 18,55 |
| Gef.: | 67,05 | 6,15 | 18,79 |

Beispiel 75

5,11-Dihydro-8,9-dimethyl-11-[[2-methyl-2,7-diazaspiro[3,5]non-7-yl]carbonyl]-6H-pyrido[2,3-b][1,4]-benzodiazepin-6-on

Hergestellt analog Beispiel 1 aus 11-(Chlorcarbonyl)-5,11-dihydro-8,9-dimethyl-6H-pyrido[2,3-b][1,4]-benzodiazepin-6-on und 2-Methyl-2,7-diazaspiro[3,5]nonan in einer Ausbeute von 43 % der Theorie. Farblose Kristalle vom Fp. 245-247 °C (Acetonitril).

| $C_{23}H_{27}N_5O_2$ (405,50). | | | |
|---|---|---|---|
| Ber.: | C 68,13 | H 6,71 | N 17,27 |
| Gef.: | 68,05 | 6,86 | 17,41 |

Im folgenden wird die Herstellung pharmazeutischer Anwendungsformen anhand einiger Beispiele beschrieben:

Beispiel I

Tabletten mit 5 mg 5,11-Dihydro-11-[[6-methyl-2,6-diazaspiro[3,4]oct-2-yl]carbonyl]-6H-pyrido[2,3-b][1,4]-benzodiazepin-6-on

Zusammensetzung:

1 Tablette enthält:

| | |
|---|---|
| Wirkstoff | 5,0 mg |
| Milchzucker | 148,0 mg |
| Kartoffelstärke | 65,0 mg |
| Magnesiumstearat | 2,0 mg |
| | 220,0 mg |

Herstellungsverfahren:

Aus Kartoffelstärke wird durch Erwärmen ein 10%iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45° C getrocknet, nochmals durch obiges Sieb gerieben, mit Magnesiumstearat vermischt und zu Tabletten verpreßt.
Tablettengewicht: 220 mg
Stempel: 9 mm

Beispiel II

Dragées mit 5 mg 5,11-Dihydro-11-[[6-methyl-2,6-diazaspiro[3,4]oct-2-yl]carbonyl]-6H-pyrido[2,3-b][1,4]-benzodiazepin-6-on

Die nach Beispiel I hergestellten Tabletten werden nach bekanntem Verfahren mit einer Hülle überzogen, die im wesentlichen aus Zucker und Talkum besteht. Die fertigen Dragées werden mit Hilfe von Bienenwachs poliert.
Dragéegewicht: 300 mg

Beispiel III

Ampullen mit 10 mg 5,11-Dihydro-11-[[6-methyl-2,6-diazaspiro[3,4]oct-2-yl]carbonyl]-6H-pyrido[2,3-b][1,4]-benzodiazepin-6-on

Zusammensetzung:

1 Ampulle enthält:

| | | |
|---|---|---|
| Wirkstoff | | 10,0 mg |
| Natriumchlorid | | 8,0 mg |
| Dest. Wasser | ad | 1 ml |

Herstellungsverfahren:

Die Wirksubstanz und Natriumchlorid werden in dest. Wasser gelöst und anschließend auf das gegebene Volumen aufgefüllt. Die Lösung wird sterilfiltriert und in 1 ml-Ampullen abgefüllt.
Sterilisation: 20 Minuten bei 120° C.

Beispiel IV

Suppositorien mit 20 mg 5,11-Dihydro-11-[[6-methyl-2,6-diazaspiro[3,4]oct-2-yl]carbonyl]-6H-pyrido[2,3-b]-[1,4]benzodiazepin-6-on

Zusammensetzung:

1 Zäpfchen enthält:

| | |
|---|---|
| Wirkstoff | 20,0 mg |
| Zäpfchenmasse (z.B. Witepsol W 45(R)) | 1 680,0 mg |
| | 1 700,0 mg |

Herstellungsverfahren:

Die feinpulverisierte Wirksubstanz wird in der geschmolzenen und auf 40° C abgekühlten Zäpfchenmasse suspendiert. Man gießt die Masse bei 37° C in leicht vorgekühlte Zäpfchenformen aus.
Zäpfchengewicht 1,7 g

Beispiel V

Tropfen mit 5,11-Dihydro-11-[[6-methyl-2,6-diazaspiro[3,4]oct-2-yl]carbonyl]-6H-pyrido[2,3-b][1,4]-benzodiazepin-6-on

Zusammensetzung:

100 ml Tropflösung enthalten:

| | | |
|---|---|---|
| p-Hydroxybenzoesäuremethylester | | 0,035 g |
| p-Hydroxybenzoesäurepropylester | | 0,015 g |
| Anisöl | | 0,05 g |
| Menthol | | 0,06 g |
| Ethanol rein | | 10,0 g |
| Wirkstoff | | 0,5 g |
| Natriumcyclamat | | 1,0 g |
| Glycerin | | 15,0 g |
| Dest. Wasser | ad | 100,0 ml |

Herstellungsverfahren:

Die Wirksubstanz und Natriumcyclamat werden in ca. 70 ml Wasser gelöst und Glycerin zugefügt. Man löst p-Hydroxybenzoesäureester, Anisöl sowie Menthol in Ethanol und fügt diese Lösung unter Rühren der wäßrigen Lösung zu. Abschließend wird mit Wasser auf 100 ml aufgefüllt und schwebeteilchenfrei filtriert.

## Ansprüche

1.) Kondensierte Diazepinone der allgemeinen Formel I

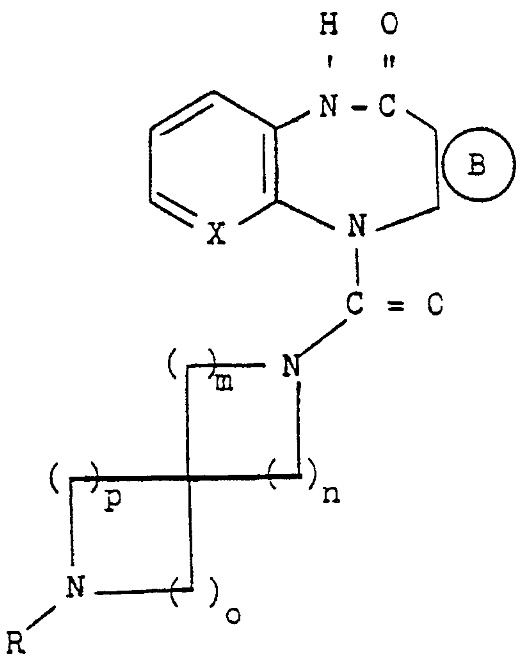

(I),

in der

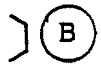 einen der zweiwertigen Reste

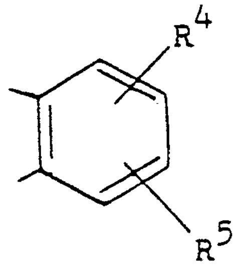 , 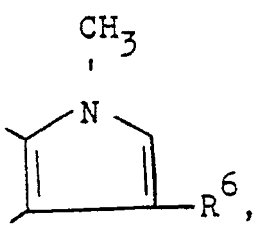 , 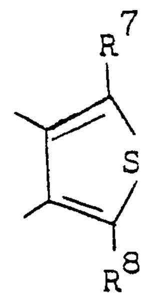 , 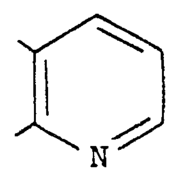

(S) (T) (U) (V)

darstellt und

X, R, $R^4$, $R^5$, $R^6$, $R^7$, $R^8$, m, n, o und p die folgenden Bedeutungen besitzen:

X ist eine =CH-Gruppe oder ein Stickstoffatom,

R ist ein geradkettiger oder verzweigter Alkylrest mit 1 bis 4 Kohlenstoffatomen, der auch gegebenenfalls noch durch ein durch Chlor, Brom, Fluor, Methyl oder Methoxy mono- oder disubstituiertes Phenyl substituiert sein kann,

$R^4$ und $R^5$, die gleich oder voneinander verschieden sein können, bedeuten ein Wasserstoff-, Fluor-, Chlor- oder Bromatom oder eine Alkylgruppe mit 1 bis 4 Kohlenstoffatomen,

$R^6$ ist ein Wasserstoff- oder Chloratom oder eine Methylgruppe,

$R^7$ und $R^8$, die gleich oder voneinander verschieden sein können, bedeuten Wasserstoffatome oder Alkylgruppen mit 1 bis 4 Kohlenstoffatomen, $R^8$ kann aber auch zusätzlich ein Halogenatom bedeuten,

m, n, o und p bedeuten jeweils die Zahlen 1 oder 2 mit der Einschränkung, daß die Summe aus m+n+o+p gleich oder kleiner als 6 sein muß,

ihre Isomeren und ihre physiologisch verträglichen Salze mit anorganischen oder organischen Säuren.

2.) Kondensierte Diazepinone der allgemeinen Formel I gemäß Anspruch 1, in der entweder

X ein Stickstoffatom und

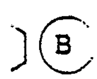

den Rest (S) oder
X die =CH-Gruppe und

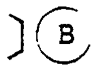

den Rest (V) bedeuten,
R die Methylgruppe,
$R^4$ und $R^5$, die gleich oder voneinander verschieden sein können, jeweils ein Wasserstoff-, Fluor- oder Chloratom, die Methyl- oder Ethylgruppe darstellen und
m und p jeweils den Zahlenwert 1 und
n und o jeweils unabhängig voneinander die Werte 1 oder 2 annehmen,
ihre Isomeren und ihre physiologisch verträglichen Salze mit anorganischen oder organischen Säuren.
3.) Als kondensierte Diazepinone der allgemeinen Formel I gemäß Anspruch 1 die Verbindungen
5,11-Dihydro-11-[[7-methyl-2,7-diazaspiro[4,4]non-2-yl]carbonyl]-6H-pyrido[2,3-b][1,4]benzodiazepin-6-on
5,11-Dihydro-8-methyl-11-[[7-methyl-2,7-diazaspiro[4,4]non-2-yl]carbonyl]-6H-pyrido[2,3-b][1,4]-benzodiazepin-6-on
5,11-Dihydro-11-[[6-methyl-2,6-diazaspiro[3,4]oct-2-yl]carbonyl]-6H-pyrido[2,3-b][1,4]benzodiazepin-6-on
5,11-Dihydro-8-ethyl-11-[[6-methyl-2,6-diazaspiro[3,4]oct-2-yl]carbonyl]-6H-pyrido[2,3-b][1,4]benzodiazepin-6-on
5,11-Dihydro-8-methyl-11-[[2-methyl-2,6-diazaspiro[3,4]-oct-6-yl]carbonyl]-6H-pyrido[2,3-b][1,4]-benzodiazepin-6-on
ihre Isomeren und ihre physiologisch verträglichen Salze mit anorganischen oder organischen Säuren.
4.) Arzneimittel enthaltend eine oder mehrere Verbindungen gemäß den Ansprüchen 1 bis 3 neben den üblichen Träger-und/oder Hilfsstoffen.
5.) Verwendung von Verbindungen gemäß Anspruch 1 bis 3 zur Herstellung von Arzneimitteln zur Therapie cholinerg bedingter Spasmen und Motilitätsstörungen im Magen-Darm-Trakt und im Bereich der abführenden Gallengänge, zur Therapie der Cystitis und von Spasmen bei Urelithiasis, zur Therapie der relativen Inkontinenz, zur Therapie des Asthma bronchiale und der Bronchitis und zur Therapie ischämischer Herzerkrankungen.
6.) Verfahren zur Herstellung von kondensierten Diazepinonen der allgemeinen Formel I

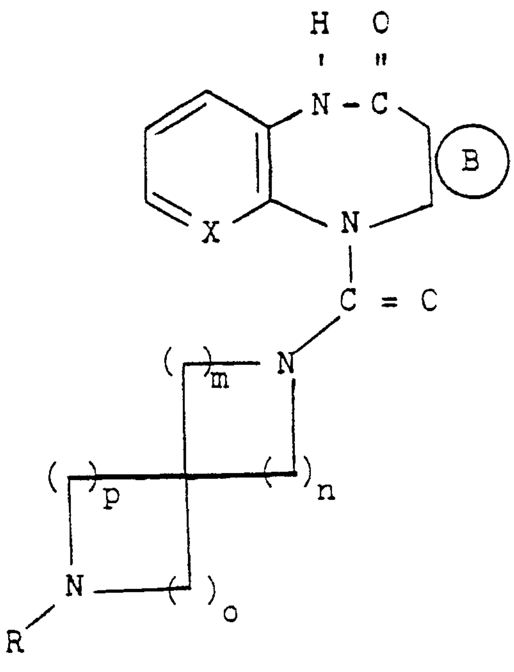

(I),

in der

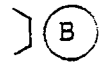 einen der zweiwertigen Reste

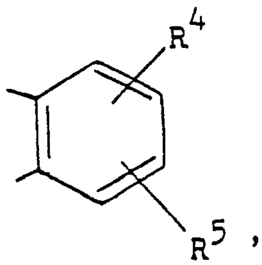

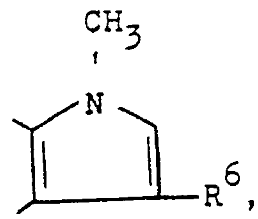

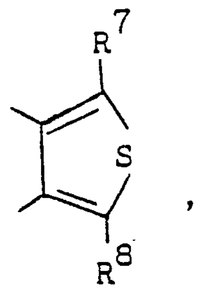

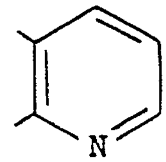

(S) (T) (U) (V)

darstellt und

X, R, $R^4$, $R^5$, $R^6$, $R^7$, $R^8$, m, n, o und p die folgenden Bedeutungen besitzen:

X ist eine =CH-Gruppe oder ein Stickstoffatom,

R ist ein geradkettiger oder verzweigter Alkylrest mit 1 bis 4 Kohlenstoffatomen, der auch gegebenenfalls noch durch ein durch Chlor, Brom, Fluor, Methyl oder Methoxy mono- oder disubstituiertes Phenyl substituiert sein kann,

$R^4$ und $R^5$, die gleich oder voneinander verschieden sein können, bedeuten ein Wasserstoff-, Fluor-, Chlor- oder Bromatom oder eine Alkylgruppe mit 1 bis 4 Kohlenstoffatomen,

$R^6$ ist ein Wasserstoff- oder Chloratom oder eine Methylgruppe,

$R^7$ und $R^8$, die gleich oder voneinander verschieden sein können, bedeuten Wasserstoffatome oder Alkylgruppen mit 1 bis 4 Kohlenstoffatomen, $R^8$ kann aber auch zusätzlich ein Halogenatom bedeuten,

m, n, o und p bedeuten jeweils die Zahlen 1 oder 2 mit der Einschränkung, daß die Summe aus m.n+o+p gleich oder kleiner als 6 sein muß,

ihrer Isomeren und ihrer Salze mit anorganischen oder organischen Säuren,

dadurch gekennzeichnet, daß

a.) zur Herstellung basisch substituierter kondensierter Diazepinone der allgemeinen Formel Ia

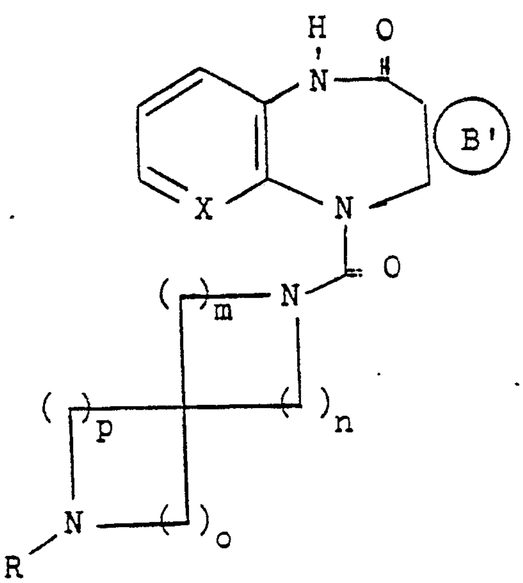

(Ia),

in der

X, R, m, n, o und p die oben angegebenen Bedeutungen haben und

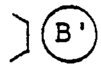

einen der zweiwertigen Reste (S), (U), (V) oder (T′)

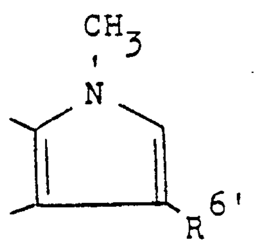

(T'),

darstellt, worin $R^4$, $R^5$, $R^7$ und $R^8$ wie oben definiert sind und $R^{6'}$ ein Chloratom oder eine Methylgruppe bedeutet,
ein Kohlensäurederivat der allgemeinen Formel II

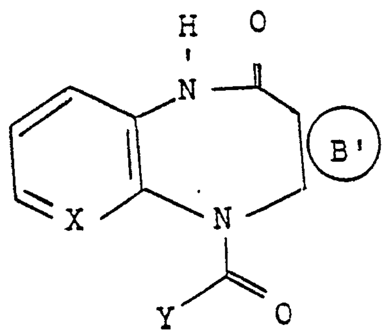

(II),

in der

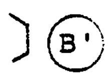

und X wie oben definiert sind und Y ein Halogenatom oder den Rest $-OR^{11}$ bedeutet, in dem $R^{11}$ einen gegebenenfalls halogensubstituierten Alkylrest mit 1 bis 5 Kohlenstoffatomen, einen gegebenenfalls durch Halogenatome oder Nitrogruppen substituierten Phenylrest oder einen Aralkylrest mit 7 bis 15 Kohlenstoffatomen darstellt,
mit einer Verbindung der allgemeinen Formel III

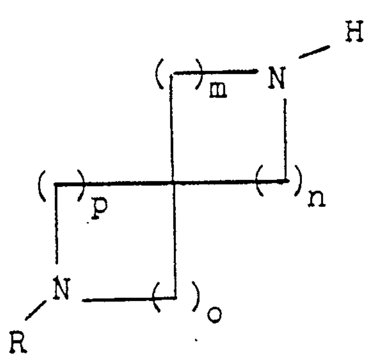

(III),

oder mit ihrer Metallverbindung der allgemeinen Formel IIIa

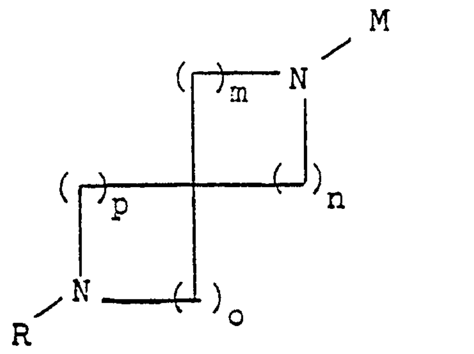

(IIIa),

in welchen R, m, n, o, p wie oben definiert sind und M ein Alkalimetallatom oder 1 Äquivalent eines Erdalkalimetallatoms bedeutet, gegebenenfalls in Anwesenheit eines Lösungsmittels, bei Temperaturen zwischen -10 ° C und dem Siedepunkt des Reaktionsgemisches, gegebenenfalls in Gegenwart einer Base oder eines Überschusses der Verbindung der allgemeinen Formel III, umgesetzt wird oder

b.) zur Herstellung basisch substituierter kondensierter Diazepinone der vorgenannten Formel Ia ein Tricyclus der allgemeinen Formel IV

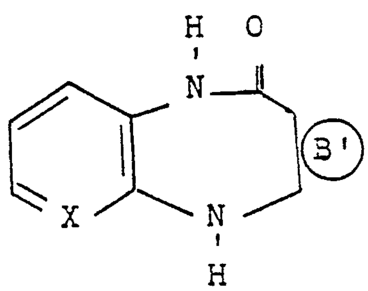

(IV),

in der

X und

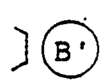

wie oben definiert sind, mit einem Chlorkohlensäurederivat der allgemeinen Formel V

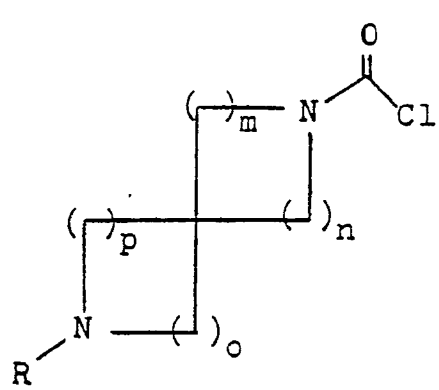

(V),

in der
R, m, n, o und p die oben erwähnten Bedeutungen haben, in inerten Lösungsmitteln, vorzugsweise in Gegenwart von Basen, bei Temperaturen zwischen +30 und +100 °C umgesetzt wird, oder
c.) zur Herstellung von unter die allgemeine Formel I fallenden Pyrrolo-kondensierten Diazepinonen der allgemeinen Formel Ib

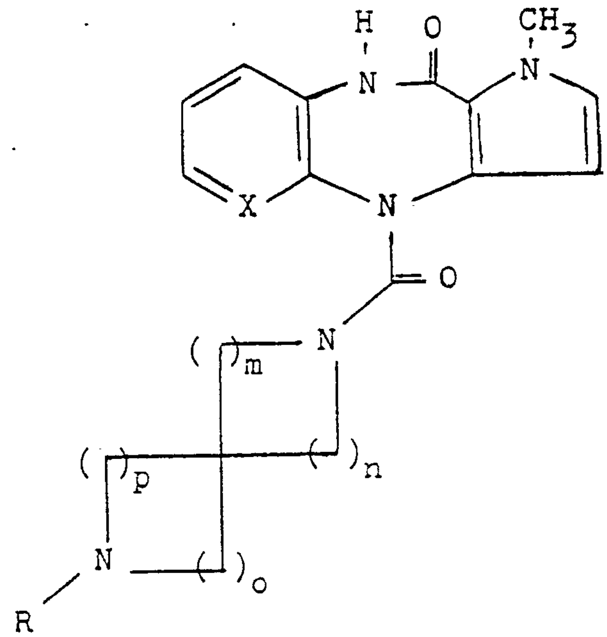

(Ib),

in der
X, R, m, n, o und p die oben erwähnten Bedeutungen haben, Verbindungen der allgemeinen Formel Ia, in der

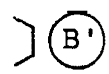

den zweiwertigen Rest (T′)

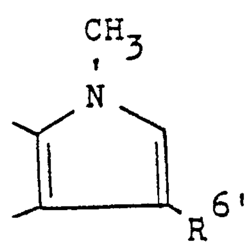

(T'),

bedeutet, worin $R^{6'}$ ein Chloratom darstellt, einer Hydrogenolyse unterworfen werden, und, gegebenenfalls anschließend, so erhaltene Verbindungen in ihre Isomeren aufgetrennt und/oder mit anorganischen oder organischen Säuren in ihre Salze übergeführt werden.

7.) Verfahren gemäß Anspruch 6c, dadurch gekennzeichnet, daß die Hydrogenolyse in Gegenwart von Katalysatoren von Metallen der VIII. Nebengruppe des Periodensystem, bei Wasserstoffdrucken von 1 bis 300 bar und Temperaturen von 0° bis 130 °C in Gegenwart von Lösungsmitteln durchgeführt wird.

8.) Verfahren gemäß Anspruch 6c, dadurch gekennzeichnet, daß die Hydrogenolyse
a.) mit Ameisensäure und einem Palladium-auf-Kohle-Katalysator bei Temperaturen zwischen 70 und 110 °C in Gegenwart eines Lösungsmittels oder
b.) mit Triethylammoniumformiat in Gegenwart überschüssigen Triethylamins und von Palladium auf

Tierkohle oder
c.) mit Palladiumacetat und Triarylphosphinen bei Temperaturen zwischen 40 und 110 °C erfolgt.

Patentansprüche Für folgende Vertragsstaaten: ES, GR

1.) Verfahren zur Herstellung von kondensierten Diazepinonen der allgemeinen Formel I

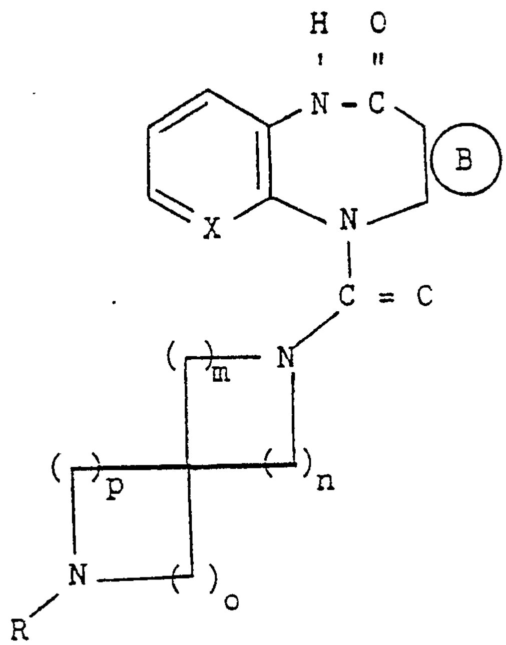

(I),

in der

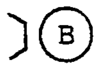 einen der zweiwertigen Reste

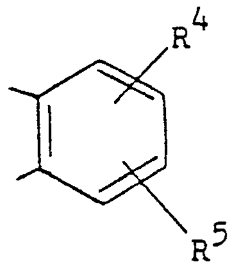 ,

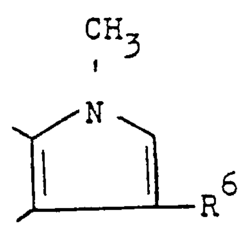 ,

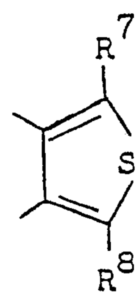 ,

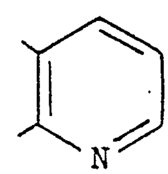

(S) (T) (U) (V)

darstellt und
X, R, $R^4$, $R^5$, $R^6$, $R^7$, $R^8$, m, n, o und p die folgenden Bedeutungen besitzen:
X ist eine =CH-Gruppe oder ein Stickstoffatom,
R ist ein geradkettiger oder verzweigter Alkylrest mit 1 bis 4 Kohlenstoffatomen, der auch gegebenenfalls noch durch ein durch Chlor, Brom, Fluor, Methyl oder Methoxy mono- oder disubstituiertes Phenyl substituiert sein kann,
$R^4$ und $R^5$, die gleich oder voneinander verschieden sein können, bedeuten ein Wasserstoff-, Fluor-, Chlor- oder Bromatom oder eine Alkylgruppe mit 1 bis 4 Kohlenstoffatomen,
$R^6$ ist ein Wasserstoff- oder Chloratom oder eine Methylgruppe,

$R^7$ und $R^8$, die gleich oder voneinander verschieden sein können, bedeuten Wasserstoffatome oder Alkylgruppen mit 1 bis 4 Kohlenstoffatomen, $R^8$ kann aber auch zusätzlich ein Halogenatom bedeuten, m, n, o und p bedeuten jeweils die Zahlen 1 oder 2 mit der Einschränkung, daß die Summe aus $m+n+o+p$ gleich oder kleiner als 6 sein muß,
ihrer Isomeren und ihrer Salze mit anorganischen oder organischen Säuren,
dadurch gekennzeichnet, daß
a.) zur Herstellung basisch substituierter kondensierter Diazepinone der allgemeinen Formel Ia

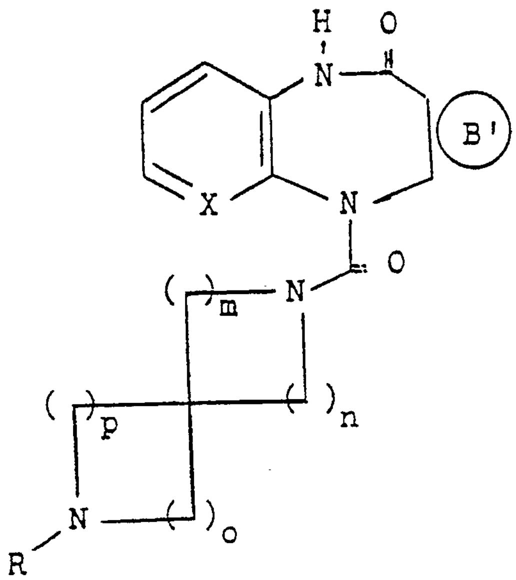

(Ia),

in der
X, R, m, n, o und p die oben angegebenen Bedeutungen haben und

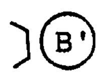

einen der zweiwertigen Reste (S), (U), (V) oder (T')

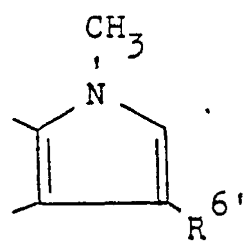

(T'),

darstellt, worin $R^4$, $R^5$, $R^7$ und $R^8$ wie oben definiert sind und $R^{6'}$ ein Chloratom oder eine Methylgruppe bedeutet,
ein Kohlensäurederivat der allgemeinen Formel II

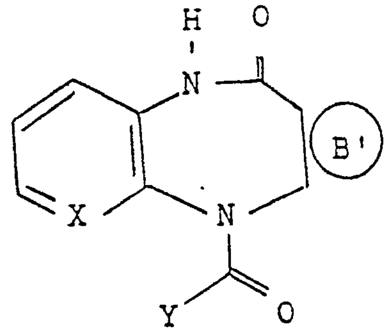

(II),

in der

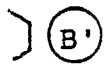

und X wie oben definiert sind und Y ein Halogenatom oder den Rest -OR11 bedeutet, in dem R11 einen gegebenenfalls halogensubstituierten Alkylrest mit 1 bis 5 Kohlenstoffatomen, einen gegebenenfalls durch Halogenatome oder Nitrogruppen substituierten Phenylrest oder einen Aralkylrest mit 7 bis 15 Kohlenstoffatomen darstellt,
mit einer Verbindung der allgemeinen Formel III

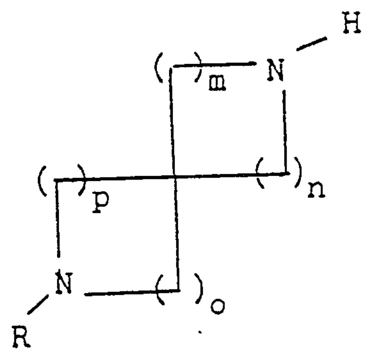

(III),

oder mit ihrer Metallverbindung der allgemeinen Formel IIIa

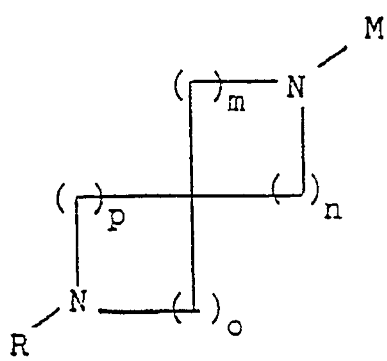

(IIIa),

in welchen R, m, n, o, p wie oben definiert sind und M ein Alkalimetallatom oder 1 Äquivalent eines Erdalkalimetallatoms bedeutet, gegebenenfalls in Anwesenheit eines Lösungsmittels, bei Temperaturen zwischen -10°C und dem Siedepunkt des Reaktionsgemisches, gegebenenfalls in Gegenwart einer Base oder eines Überschusses der Verbindung der allgemeinen Formel III, umgesetzt wird oder

b.) zur Herstellung basisch substituierter kondensierter Diazepinone der vorgenannten Formel Ia ein Tricyclus der allgemeinen Formel IV

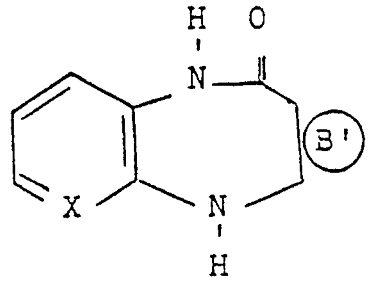

. (IV),

in der
X und

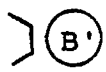

wie oben definiert sind, mit einem Chlorkohlensäurederivat der allgemeinen Formel V

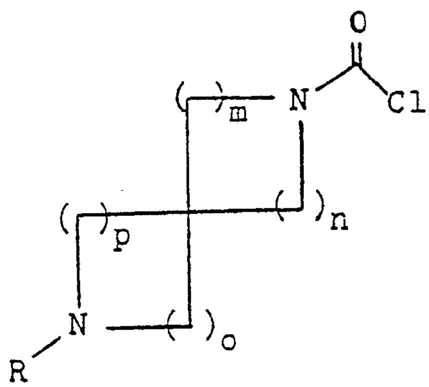

(V),

in der
R, m, n, o und p die oben erwähnten Bedeutungen haben,
in inerten Lösungsmitteln, vorzugsweise in Gegenwart von Basen, bei Temperaturen zwischen +30 und +100 °C umgesetzt wird, oder
c.) zur Herstellung von unter die allgemeine Formel I fallenden Pyrrolo-kondensierten Diazepinonen der allgemeinen Formel Ib

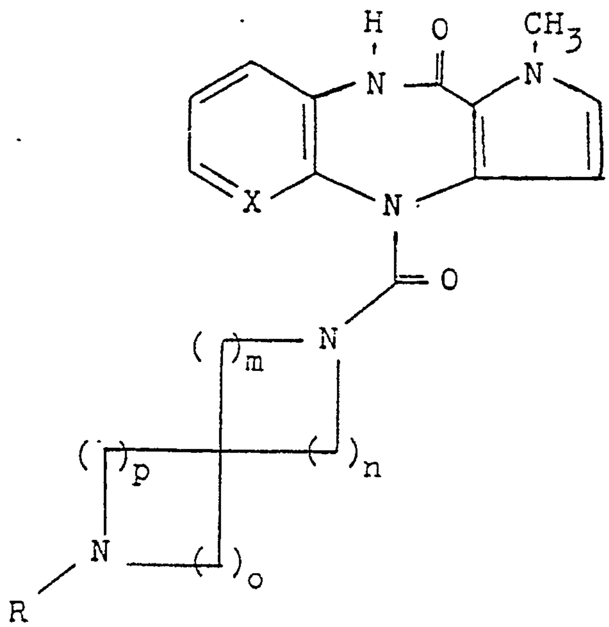

(Ib),

in der

X, R, m, n, o und p die oben erwähnten Bedeutungen haben, Verbindungen der allgemeinen Formel Ia, in der

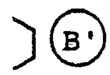

den zweiwertigen Rest (T')

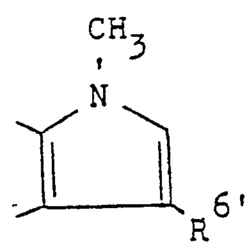

(T'),

bedeutet, worin $R^{6'}$ ein Chloratom darstellt, einer Hydrogenolyse unterworfen werden, und, gegebenenfalls anschließend, so erhaltene Verbindungen in ihre Isomeren aufgetrennt und/oder mit anorganischen oder organischen Säuren in ihre Salze übergeführt werden.

2.) Verfahren gemäß Anspruch 1c, dadurch gekennzeichnet, daß die Hydrogenolyse in Gegenwart von Katalysatoren von Metallen der VIII. Nebengruppe des Periodensystem, bei Wasserstoffdrucken von 1 bis 300 bar und Temperaturen von 0° bis 130° C in Gegenwart von Lösungsmitteln durchgeführt wird.

3.) Verfahren gemäß Anspruch 1c, dadurch gekennzeichnet, daß die Hydrogenolyse

a.) mit Ameisensäure und einem Palladium-auf-Kohle-Katalysator bei Temperaturen zwischen 70 und 110° C in Gegenwart eines Lösungsmittels oder

b.) mit Triethylammoniumformiat in Gegenwart überschüssigen Triethylamins und von Palladium auf Tierkohle oder

c.) mit Palladiumacetat und Triarylphosphinen bei Temperaturen zwischen 40 und 110° C erfolgt.